(19) Europäisches Patentamt
European Patent Office
Office européen des brevets

(11) EP 1 734 121 A2

(12) # EUROPEAN PATENT APPLICATION

(43) Date of publication:
**20.12.2006 Bulletin 2006/51**

(51) Int Cl.:
***C12N 15/15*** *(2006.01)* ***C07K 14/81*** *(2006.01)*
***C12N 1/15*** *(2006.01)* ***A61K 38/57*** *(2006.01)*

(21) Application number: **05025964.7**

(22) Date of filing: **15.12.1995**

(84) Designated Contracting States:
**AT BE CH DE DK ES FR GB GR IE IT LI LU MC NL PT SE**

(30) Priority: **16.12.1994 US 358160**

(62) Document number(s) of the earlier application(s) in accordance with Art. 76 EPC:
**95943819.3 / 0 797 666**

(71) Applicant: **Dyax Corporation**
**Cambridge, MA 02139 (US)**

(72) Inventors:
- **Ley, Arthur Charles**
  **Newton, MA 02165 (US)**
- **Ladner, Robert Charles**
  **Ijamsville, MD 21754 (US)**
- **Guterman, Sonia Kosow**
  **Belmont, MA 02178 (US)**
- **Roberts, Bruce Lindsay**
  **Southborough, MA 01772 (US)**
- **Markland, William**
  **Southborough, MA 01772 (US)**
- **Kent, Rachel Baribault**
  **Boxborough, MA 01719 (US)**

(74) Representative: **Wichmann, Hendrik**
**Isenbruck Bösl Hörschler Wichmann Huhn**
**Prinzregentenstrasse 68**
**81675 München (DE)**

Remarks:
•This application was filed on 29.11.2005 as a divisional application to the application mentioned under INID code 62.
•The sequence listing, which is published as annex to the application documents, was filed after the date of filing. The applicant has declared that it does not include matter which goes beyond the content of the application as filed.

(54) **Genetically engineered human-derived Kunitz domains that inhibit human neutrophil elastase**

(57) This invention relates to novel proteins that inhibit human neutrophil elastase (hNE). A large fraction of the sequence of each of these proteins is identical to a known human protein which has very little or no inhibitory activity with respect to hNE.

EP 1 734 121 A2

Annex to the application documents - subsequently filed sequences listing

SEQUENCE LISTING

<110> Dyax Corp.

<120> GENETICALLY ENGINEERED HUMAN-DERIVED KUNITZ DOMAINS THAT INHIBIT HUMAN NEUTROPHIL ELASTASE

<130> D39075PCEPT1

<140> EP05025964.7
<141> 1995-12-15

<150> PCT/US95/16349
<151> 1995-12-15

<150> US 08/358,160
<151> 1994-12-16

<160> 85

<170> PatentIn version 3.3

<210> 1
<211> 276
<212> DNA
<213> Artificial sequence

<220>

<223> Synthetic sequence IIIsp::bpti::matIII(fragment)

<400> 1
```
gtgaaaaaat tattattcgc aattccttta gttgttcctt tctattctgg cgcccgtccg     60

gatttctgtc tcgagccacc atacactggg ccctgcaaag cgcgcatcat ccgctatttc    120

tacaatgcta aagcaggcct gtgccagacc tttgtatacg gtggttgccg tgctaagcgt    180

aacaacttta aatcggccga agattgcatg cgtacctgcg gtggcgccgc tgaaactgtt    240

gaaagttgtt tagcaaaacc ccatacagaa aattca                              276
```

<210> 2
<211> 92
<212> PRT
<213> Artificial sequence

<220>

<223> Synthetic sequence IIIsp::bpti::matIII(fragment)

<400> 2

```
Met Lys Lys Leu Leu Phe Ala Ile Pro Leu Val Val Pro Phe Tyr Ser
1               5                   10                  15

Gly Ala Arg Pro Asp Phe Cys Leu Glu Pro Pro Tyr Thr Gly Pro Cys
            20                  25                  30

Lys Ala Arg Ile Ile Arg Tyr Phe Tyr Asn Ala Lys Ala Gly Leu Cys
        35                  40                  45

Gln Thr Phe Val Tyr Gly Gly Cys Arg Ala Lys Arg Asn Asn Phe Lys
    50                  55                  60

Ser Ala Glu Asp Cys Met Arg Thr Cys Gly Gly Ala Ala Glu Thr Val
65                  70                  75                  80
```

```
Glu Ser Cys Leu Ala Lys Pro His Thr Glu Asn Ser
                85                  90


<210>  3
<211>  285
<212>  DNA
<213>  Artificial sequence

<220>

<223>  Synthetic sequence IIsp::itiD1::matIII(frag)

<400>  3
gtgaaaaaat tattattcgc aattccttta gttgttcctt tctattctgg cgccaaagaa      60

gactcttgcc agctgggcta ctcggccggt ccctgcatgg gaatgaccag caggtatttc     120

tataatggta catccatggc ctgtgagact ttccagtacg gcggctgcat gggcaacggt     180

aacaacttcg tcacagaaaa ggagtgtctg cagacctgcc gaactgtggg cgccgctgaa     240

actgttgaaa gttgtttagc aaaaccccat acagaaaatt cattt                     285


<210>  4
<211>  95
<212>  PRT
<213>  Artificial sequence

<220>

<223>  Synthetic sequence IIsp::itiD1::matIII(frag)

<400>  4

Met Lys Lys Leu Leu Phe Ala Ile Pro Leu Val Val Pro Phe Tyr Ser
1               5                   10                  15

Gly Ala Lys Glu Asp Ser Cys Gln Leu Gly Tyr Ser Ala Gly Pro Cys
            20                  25                  30

Met Gly Met Thr Ser Arg Tyr Phe Tyr Asn Gly Thr Ser Met Ala Cys
        35                  40                  45

Glu Thr Phe Gln Tyr Gly Gly Cys Met Gly Asn Gly Asn Asn Phe Val
    50                  55                  60

Thr Glu Lys Glu Cys Leu Gln Thr Cys Arg Thr Val Gly Ala Ala Glu
65                  70                  75                  80


Thr Val Glu Ser Cys Leu Ala Lys Pro His Thr Glu Asn Ser Phe
                85                  90                  95


<210>  5
<211>  58
<212>  PRT
<213>  Homo sapiens

<400>  5

Arg Pro Asp Phe Cys Leu Leu Pro Ala Glu Thr Gly Pro Cys Arg Ala
1               5                   10                  15

Met Ile Pro Arg Phe Tyr Tyr Asn Ala Lys Ser Gly Lys Cys Glu Pro
            20                  25                  30
```

```
Phe Ile Tyr Gly Gly Cys Gly Gly Asn Ala Asn Asn Phe Lys Thr Glu
        35                  40                  45

Glu Glu Cys Arg Arg Thr Cys Gly Gly Ala
    50                  55


<210>  6
<211>  58
<212>  PRT
<213>  Homo sapiens

<400>  6

Arg Pro Asp Phe Cys Leu Glu Pro Pro Tyr Thr Gly Pro Cys Lys Ala
1               5                   10                  15

Arg Ile Ile Arg Tyr Phe Tyr Asn Ala Lys Ala Gly Leu Cys Gln Thr
            20                  25                  30

Phe Val Tyr Gly Gly Cys Arg Ala Lys Arg Asn Asn Phe Lys Ser Ala
        35                  40                  45

Glu Asp Cys Met Arg Thr Cys Gly Gly Ala
    50                  55


<210>  7
<211>  58
<212>  PRT
<213>  Homo sapiens

<400>  7

Arg Pro Asp Phe Cys Leu Glu Pro Pro Tyr Thr Gly Pro Cys Ile Ala
1               5                   10                  15

Phe Phe Pro Arg Tyr Phe Tyr Asn Ala Lys Ala Gly Leu Cys Gln Thr
            20                  25                  30

Phe Val Tyr Gly Gly Cys Met Gly Asn Gly Asn Asn Phe Lys Ser Ala
        35                  40                  45


Glu Asp Cys Met Arg Thr Cys Gly Gly Ala
    50                  55


<210>  8
<211>  62
<212>  PRT
<213>  Homo sapiens

<400>  8

Glu Ala Glu Ala Arg Pro Asp Phe Cys Leu Glu Pro Pro Tyr Thr Gly
1               5                   10                  15

Pro Cys Ile Ala Phe Phe Pro Arg Tyr Phe Tyr Asn Ala Lys Ala Gly
            20                  25                  30

Leu Cys Gln Thr Phe Val Tyr Gly Gly Cys Met Gly Asn Gly Asn Asn
        35                  40                  45

Phe Lys Ser Ala Glu Asp Cys Met Arg Thr Cys Gly Gly Ala
    50                  55                  60
```

<210> 9
<211> 58
<212> PRT
<213> Homo sapiens

<400> 9

Arg Pro Asp Phe Cys Leu Glu Pro Pro Tyr Thr Gly Pro Cys Val Ala
1 5 10 15

Met Phe Pro Arg Tyr Phe Tyr Asn Ala Lys Ala Gly Leu Cys Gln Thr
20 25 30

Phe Val Tyr Gly Gly Cys Met Gly Asn Gly Asn Asn Phe Lys Ser Ala
35 40 45

Glu Asp Cys Met Arg Thr Cys Gly Gly Ala .
50 55

<210> 10
<211> 58
<212> PRT
<213> Homo sapiens

<400> 10

Arg Pro Asp Phe Cys Leu Glu Pro Pro Tyr Thr Gly Pro Cys Val Gly
1 5 10 15

Phe Phe Ser Arg Tyr Phe Tyr Asn Ala Lys Ala Gly Leu Cys Gln Thr
20 25 30

Phe Val Tyr Gly Gly Cys Met Gly Asn Gly Asn Asn Phe Lys Ser Ala
35 40 45

Glu Asp Cys Met Arg Thr Cys Gly Gly Ala
50 55

<210> 11
<211> 58
<212> PRT
<213> Homo sapiens

<400> 11

Arg Pro Asp Phe Cys Leu Glu Pro Pro Tyr Thr Gly Pro Cys Val Gly
1 5 10 15

Phe Phe Gln Arg Tyr Phe Tyr Asn Ala Lys Ala Gly Leu Cys Gln Thr
20 25 30

Phe Val Tyr Gly Gly Cys Met Gly Asn Gly Asn Asn Phe Lys Ser Ala
35 40 45

Glu Asp Cys Met Arg Thr Cys Gly Gly Ala
50 55

<210> 12
<211> 58
<212> PRT
<213> Homo sapiens

<400> 12

Arg Pro Asp Phe Cys Leu Glu Pro Pro Tyr Thr Gly Pro Cys Val Ala

1 5 10 15

Ile Phe Pro Arg Tyr Phe Tyr Asn Ala Lys Ala Gly Leu Cys Gln Thr
20 25 30

Phe Val Tyr Gly Gly Cys Met Gly Asn Gly Asn Asn Phe Lys Ser Ala
35 40 45

Glu Asp Cys Met Arg Thr Cys Gly Gly Ala
50 55

<210> 13
<211> 58
<212> PRT
<213> Homo sapiens

<400> 13

Arg Pro Asp Phe Cys Leu Glu Pro Pro Tyr Thr Gly Pro Cys Val Ala
1 5 10 15

Phe Phe Lys Arg Ser Phe Tyr Asn Ala Lys Ala Gly Leu Cys Gln Thr
20 25 30

Phe Val Tyr Gly Gly Cys Met Gly Asn Gly Asn Asn Phe Lys Ser Ala
35 40 45

Glu Asp Cys Met Arg Thr Cys Gly Gly Ala
50 55

<210> 14
<211> 58
<212> PRT
<213> Homo sapiens

<400> 14

Arg Pro Asp Phe Cys Leu Glu Pro Pro Tyr Thr Gly Pro Cys Ile Ala
1 5 10 15

Phe Phe Gln Arg Tyr Phe Tyr Asn Ala Lys Ala Gly Leu Cys Gln Thr
20 25 30

Phe Val Tyr Gly Gly Cys Met Gly Asn Gly Asn Asn Phe Lys Ser Ala
35 40 45

Glu Asp Cys Met Arg Thr Cys Gly Gly Ala
50 55

<210> 15
<211> 58
<212> PRT
<213> Homo sapiens

<400> 15

Arg Pro Asp Phe Cys Leu Glu Pro Pro Tyr Thr Gly Pro Cys Ile Ala
1 5 10 15

Leu Phe Lys Arg Tyr Phe Tyr Asn Ala Lys Ala Gly Leu Cys Gln Thr
20 25 30

Phe Val Tyr Gly Gly Cys Met Gly Asn Gly Asn Asn Phe Lys Ser Ala
35 40 45

Glu Asp Cys Met Arg Thr Cys Gly Gly Ala
50 55

<210> 16
<211> 58
<212> PRT
<213> Homo sapiens

<400> 16

Lys Glu Asp Ser Cys Gln Leu Gly Tyr Ser Ala Gly Pro Cys Met Gly
1 5 10 15

Met Thr Ser Arg Tyr Phe Tyr Asn Gly Thr Ser Met Ala Cys Glu Thr
20 25 30

Phe Gln Tyr Gly Gly Cys Met Gly Asn Gly Asn Asn Phe Val Thr Glu
35 40 45

Lys Asp Cys Leu Gln Thr Cys Arg Thr Val
50 55

<210> 17
<211> 58
<212> PRT
<213> Homo sapiens

<400> 17

Arg Pro Asp Phe Cys Gln Leu Gly Tyr Ser Ala Gly Pro Cys Val Ala
1 5 10 15

Met Phe Pro Arg Tyr Phe Tyr Asn Gly Thr Ser Met Ala Cys Gln Thr
20 25 30

Phe Val Tyr Gly Gly Cys Met Gly Asn Gly Asn Asn Phe Val Thr Glu
35 40 45

Lys Asp Cys Leu Gln Thr Cys Arg Gly Ala
50 55

<210> 18
<211> 58
<212> PRT
<213> Homo sapiens

<400> 18

Arg Pro Asp Phe Cys Gln Leu Gly Tyr Ser Ala Gly Pro Cys Val Ala
1 5 10 15

Met Phe Pro Arg Tyr Phe Tyr Asn Gly Ala Ser Met Ala Cys Gln Thr
20 25 30

Phe Val Tyr Gly Gly Cys Met Gly Asn Gly Asn Asn Phe Val Thr Glu
35 40 45

Lys Asp Cys Leu Gln Thr Cys Arg Gly Ala
50 55

<210> 19
<211> 58
<212> PRT
<213> Homo sapiens

<400> 19

Arg Pro Asp Phe Cys Gln Leu Gly Tyr Ser Ala Gly Pro Cys Val Ala
1 5 10 15

Met Phe Pro Arg Tyr Phe Tyr Asn Gly Thr Ser Met Ala Cys Glu Thr
20 25 30

Phe Val Tyr Gly Gly Cys Met Gly Asn Gly Asn Asn Phe Val Thr Glu
35 40 45

Lys Asp Cys Leu Gln Thr Cys Arg Gly Ala
50 55

<210> 20
<211> 58
<212> PRT
<213> Homo sapiens

<400> 20

Arg Pro Asp Phe Cys Gln Leu Gly Tyr Ser Ala Gly Pro Cys Val Gly
1 5 10 15

Met Phe Ser Arg Tyr Phe Tyr Asn Gly Thr Ser Met Ala Cys Gln Thr
20 25 30

Phe Val Tyr Gly Gly Cys Met Gly Asn Gly Asn Asn Phe Val Thr Glu
35 40 45

Lys Asp Cys Leu Gln Thr Cys Arg Gly Ala
50 55

<210> 21
<211> 58
<212> PRT
<213> Homo sapiens

<400> 21

Lys Glu Asp Ser Cys Gln Leu Gly Tyr Ser Ala Gly Pro Cys Val Ala
1 5 10 15

Met Phe Pro Arg Tyr Phe Tyr Asn Gly Thr Ser Met Ala Cys Glu Thr
20 25 30

Phe Gln Tyr Gly Gly Cys Met Gly Asn Gly Asn Asn Phe Val Thr Glu
35 40 45

Lys Asp Cys Leu Gln Thr Cys Arg Gly Ala
50 55

<210> 22
<211> 58
<212> PRT
<213> Homo sapiens

<400> 22

Lys Glu Asp Phe Cys Gln Leu Gly Tyr Ser Ala Gly Pro Cys Val Ala
1 5 10 15

Met Phe Pro Arg Tyr Phe Tyr Asn Gly Thr Ser Met Ala Cys Glu Thr
20 25 30

```
Phe Gln Tyr Gly Gly Cys Met Gly Asn Gly Asn Asn Phe Val Thr Glu
        35                  40                  45

Lys Asp Cys Leu Gln Thr Cys Arg Gly Ala
    50                  55


<210>  23
<211>  58
<212>  PRT
<213>  Homo sapiens

<400>  23

Lys Pro Asp Ser Cys Gln Leu Gly Tyr Ser Ala Gly Pro Cys Val Ala
1               5                   10                  15

Met Phe Pro Arg Tyr Phe Tyr Asn Gly Thr Ser Met Ala Cys Glu Thr
            20                  25                  30

Phe Gln Tyr Gly Gly Cys Met Gly Asn Gly Asn Asn Phe Val Thr Glu
        35                  40                  45

Lys Asp Cys Leu Gln Thr Cys Arg Gly Ala
    50                  55


<210>  24
<211>  58
<212>  PRT
<213>  Homo sapiens

<400>  24

Arg Pro Asp Phe Cys Gln Leu Gly Tyr Ser Ala Gly Pro Cys Ile Gly
1               5                   10                  15

Met Phe Ser Arg Tyr Phe Tyr Asn Gly Thr Ser Met Ala Cys Glu Thr
            20                  25                  30

Phe Gln Tyr Gly Gly Cys Met Gly Asn Gly Asn Asn Phe Val Thr Glu
        35                  40                  45

Lys Asp Cys Leu Gln Thr Cys Arg Gly Ala
    50                  55


<210>  25
<211>  58
<212>  PRT
<213>  Homo sapiens

<400>  25

Thr Val Ala Ala Cys Asn Leu Pro Ile Val Arg Gly Pro Cys Arg Ala
1               5                   10                  15

Phe Ile Gln Leu Trp Ala Phe Asp Ala Val Lys Gly Lys Cys Val Leu
            20                  25                  30

Phe Pro Tyr Gly Gly Cys Gln Gly Asn Gly Asn Lys Phe Tyr Ser Glu
        35                  40                  45

Lys Glu Cys Arg Glu Tyr Cys Gly Val Pro
    50                  55
```

<210> 26
<211> 56
<212> PRT
<213> Homo sapiens

<400> 26

Ala Ala Cys Asn Leu Pro Ile Val Arg Gly Pro Cys Ile Ala Phe Phe
1               5                   10                  15

Pro Arg Trp Ala Phe Asp Ala Val Lys Gly Lys Cys Val Leu Phe Pro
            20                  25                  30

Tyr Gly Gly Cys Gln Gly Asn Gly Asn Lys Phe Tyr Ser Glu Lys Glu
        35                  40                  45

Cys Arg Glu Tyr Cys Gly Val Pro
    50                  55

<210> 27
<211> 56
<212> PRT
<213> Homo sapiens

<400> 27

Glu Ala Cys Asn Leu Pro Ile Val Arg Gly Pro Cys Ile Ala Phe Phe
1               5                   10                  15

Pro Arg Trp Ala Phe Asp Ala Val Lys Gly Lys Cys Val Leu Phe Pro
            20                  25                  30

Tyr Gly Gly Cys Gln Gly Asn Gly Asn Lys Phe Tyr Ser Glu Lys Glu
        35                  40                  45

Cys Arg Glu Tyr Cys Gly Val Pro
    50                  55

<210> 28
<211> 58
<212> PRT
<213> Homo sapiens

<400> 28

Val Arg Glu Val Cys Ser Glu Gln Ala Glu Thr Gly Pro Cys Arg Ala
1               5                   10                  15

Met Ile Ser Arg Trp Tyr Phe Asp Val Thr Glu Gly Lys Cys Ala Pro
            20                  25                  30

Phe Phe Tyr Gly Gly Cys Gly Gly Asn Arg Asn Asn Phe Asp Thr Glu
        35                  40                  45

Glu Tyr Cys Met Ala Val Cys Gly Ser Ala
    50                  55

<210> 29
<211> 58
<212> PRT
<213> Homo sapiens

<400> 29

Val Arg Glu Val Cys Ser Glu Gln Ala Tyr Thr Gly Pro Cys Ile Ala
1 5 10 15

Phe Phe Pro Arg Tyr Tyr Phe Asp Val Thr Glu Gly Lys Cys Gln Thr
20 25 30

Phe Val Tyr Gly Gly Cys Met Gly Asn Gly Asn Asn Phe Asp Thr Glu
35 40 45

Glu Tyr Cys Met Ala Val Cys Gly Ser Ala
50 55

<210> 30
<211> 58
<212> PRT
<213> Homo sapiens

<400> 30

Val Arg Glu Val Cys Ser Glu Gln Ala Glu Thr Gly Pro Cys Ile Ala
1 5 10 15

Met Phe Ser Arg Trp Tyr Phe Asp Val Thr Glu Gly Lys Cys Ala Pro
20 25 30

Phe Val Tyr Gly Gly Cys Gly Gly Asn Arg Asn Asn Phe Asp Thr Glu
35 40 45

Glu Tyr Cys Met Ala Val Cys Gly Ser Ala
50 55

<210> 31
<211> 58
<212> PRT
<213> Homo sapiens

<400> 31

Val Arg Glu Val Cys Ser Glu Gln Ala Glu Thr Gly Pro Cys Ile Ala
1 5 10 15

Phe Phe Ser Arg Trp Tyr Phe Asp Val Thr Glu Gly Lys Cys Ala Thr
20 25 30

Phe Val Tyr Gly Gly Cys Met Gly Asn Arg Asn Asn Phe Asp Thr Glu
35 40 45

Glu Tyr Cys Met Ala Val Cys Gly Ser Ala
50 55

<210> 32
<211> 58
<212> PRT
<213> Homo sapiens

<400> 32

Asn Ala Glu Ile Cys Leu Leu Pro Leu Asp Tyr Gly Pro Cys Arg Ala
1 5 10 15

Leu Leu Leu Arg Tyr Tyr Tyr Asp Arg Tyr Thr Gln Ser Cys Arg Gln
20 25 30

Phe Leu Tyr Gly Gly Cys Glu Gly Asn Ala Asn Asn Phe Tyr Thr Trp
35 40 45

Glu Ala Cys Asp Asp Ala Cys Trp Arg Ile
50 55

<210> 33
<211> 58
<212> PRT
<213> Homo sapiens

<400> 33

Asn Ala Glu Ile Cys Leu Leu Pro Leu Tyr Thr Gly Pro Cys Ile Ala
1 5 10 15

Phe Phe Pro Arg Tyr Tyr Tyr Asp Arg Tyr Thr Gln Ser Cys Gln Thr
20 25 30

Phe Val Tyr Gly Gly Cys Met Gly Asn Ala Asn Asn Phe Tyr Thr Trp
35 40 45

Glu Ala Cys Asp Asp Ala Cys Trp Arg Ile
50 55

<210> 34
<211> 58
<212> PRT
<213> Homo sapiens

<400> 34

Asn Ala Glu Ile Cys Leu Leu Pro Leu Asp Tyr Gly Pro Cys Ile Ala
1 5 10 15

Leu Phe Leu Arg Tyr Tyr Tyr Asp Arg Tyr Thr Gln Ser Cys Arg Gln
20 25 30

Phe Val Tyr Gly Gly Cys Glu Gly Asn Ala Asn Asn Phe Tyr Thr Trp
35 40 45

Glu Ala Cys Asp Asp Ala Cys Trp Arg Ile
50 55

<210> 35
<211> 58
<212> PRT
<213> Homo sapiens

<400> 35

Asn Ala Glu Ile Cys Leu Leu Pro Leu Asp Thr Gly Pro Cys Ile Ala
1 5 10 15

Phe Phe Leu Arg Tyr Tyr Tyr Asp Arg Tyr Thr Gln Ser Cys Gln Thr
20 25 30

Phe Val Tyr Gly Gly Cys Met Gly Asn Ala Asn Asn Phe Tyr Thr Trp
35 40 45

Glu Ala Cys Asp Asp Ala Cys Trp Arg Ile
50 55

<210> 36
<211> 61
<212> PRT

<213> Homo sapiens

<400> 36

Val Pro Lys Val Cys Arg Leu Gln Val Ser Val Asp Asp Gln Cys Glu
1 5 10 15

Gly Ser Thr Glu Lys Tyr Phe Phe Asn Leu Ser Ser Met Thr Cys Glu
20 25 30

Lys Phe Phe Ser Gly Gly Cys His Arg Asn Arg Ile Glu Asn Arg Phe
35 40 45

Pro Asp Glu Ala Thr Cys Met Gly Phe Cys Ala Pro Lys
50 55 60

<210> 37
<211> 58
<212> PRT
<213> Homo sapiens

<400> 37

Val Pro Lys Val Cys Arg Leu Gln Val Val Arg Gly Pro Cys Ile Ala
1 5 10 15

Phe Phe Pro Arg Trp Phe Phe Asn Leu Ser Ser Met Thr Cys Val Leu
20 25 30

Phe Pro Tyr Gly Gly Cys Gln Gly Asn Gly Asn Arg Phe Pro Asp Glu
35 40 45

Ala Thr Cys Met Gly Phe Cys Ala Pro Lys
50 55

<210> 38
<211> 61
<212> PRT
<213> Homo sapiens

<400> 38

Val Pro Lys Val Cys Arg Leu Gln Val Ser Val Asp Asp Gln Cys Ile
1 5 10 15

Gly Ser Phe Glu Lys Tyr Phe Phe Asn Leu Ala Ser Met Thr Cys Glu
20 25 30

Thr Phe Val Ser Gly Gly Cys His Arg Asn Arg Ile Glu Asn Arg Phe
35 40 45

Pro Asp Glu Ala Thr Cys Met Gly Phe Cys Ala Pro Lys
50 55 60

<210> 39
<211> 58
<212> PRT
<213> Homo sapiens

<400> 39

Val Pro Lys Val Cys Arg Leu Gln Val Val Ala Gly Pro Cys Ile Gly
1 5 10 15

Phe Phe Lys Arg Tyr Phe Phe Ala Leu Ser Ser Met Thr Cys Glu Thr

20 25 30

Phe Val Ser Gly Gly Cys His Arg Asn Arg Asn Arg Phe Pro Asp Glu
35 40 45

Ala Thr Cys Met Gly Phe Cys Ala Pro Lys
50 55

<210> 40
<211> 58
<212> PRT
<213> Homo sapiens

<400> 40

Ile Pro Ser Phe Cys Tyr Ser Pro Lys Asp Glu Gly Leu Cys Ser Ala
1 5 10 15

Asn Val Thr Arg Tyr Tyr Phe Asn Pro Arg Tyr Arg Thr Cys Asp Ala
20 25 30

Phe Thr Tyr Thr Gly Cys Gly Gly Asn Asp Asn Asn Phe Val Ser Arg
35 40 45

Glu Asp Cys Lys Arg Ala Cys Ala Lys Ala
50 55

<210> 41
<211> 58
<212> PRT
<213> Homo sapiens

<400> 41

Ile Pro Ser Phe Cys Tyr Ser Pro Lys Ser Ala Gly Pro Cys Val Ala
1 5 10 15

Met Phe Pro Arg Tyr Tyr Phe Asn Pro Arg Tyr Arg Thr Cys Glu Thr
20 25 30

Phe Val Tyr Gly Gly Cys Met Gly Asn Gly Asn Asn Phe Val Ser Arg
35 40 45

Glu Asp Cys Lys Arg Ala Cys Ala Lys Ala
50 55

<210> 42
<211> 58
<212> PRT
<213> Homo sapiens

<400> 42

Ile Pro Ser Phe Cys Tyr Ser Pro Lys Asp Glu Gly Leu Cys Ile Ala
1 5 10 15

Phe Phe Thr Arg Tyr Tyr Phe Asn Pro Arg Tyr Arg Thr Cys Asp Ala
20 25 30

Phe Thr Tyr Thr Gly Cys Gly Gly Asn Asp Asn Asn Phe Val Ser Arg
35 40 45

Glu Asp Cys Lys Arg Ala Cys Ala Lys Ala
50 55

<210> 43
<211> 58
<212> PRT
<213> Homo sapiens

<400> 43

```
Ile Pro Ser Phe Cys Tyr Ser Pro Lys Asp Thr Gly Pro Cys Ile Ala
1               5                   10                  15

Phe Phe Thr Arg Tyr Tyr Phe Asn Pro Arg Tyr Arg Thr Cys Asp Thr
            20                  25                  30

Phe Val Tyr Gly Gly Cys Gly Gly Asn Asp Asn Asn Phe Val Ser Arg
        35                  40                  45

Glu Asp Cys Lys Arg Ala Cys Ala Lys Ala
    50                  55
```

<210> 44
<211> 58
<212> PRT
<213> Homo sapiens

<400> 44

```
Met His Ser Phe Cys Ala Phe Lys Ala Asp Asp Gly Pro Cys Lys Ala
1               5                   10                  15

Ile Met Lys Arg Phe Phe Phe Asn Ile Phe Thr Arg Gln Cys Glu Glu
            20                  25                  30

Phe Ile Tyr Gly Gly Cys Glu Gly Asn Gln Asn Arg Phe Glu Ser Leu
        35                  40                  45

Glu Glu Cys Lys Lys Met Cys Thr Arg Asp
    50                  55
```

<210> 45
<211> 58
<212> PRT
<213> Homo sapiens

<400> 45

```
Met His Ser Phe Cys Ala Phe Lys Ala Ser Ala Gly Pro Cys Val Ala
1               5                   10                  15

Met Phe Pro Arg Tyr Phe Phe Asn Ile Phe Thr Arg Gln Cys Glu Thr
            20                  25                  30

Phe Val Tyr Gly Gly Cys Met Gly Asn Gly Asn Arg Phe Glu Ser Leu
        35                  40                  45

Glu Glu Cys Lys Lys Met Cys Thr Arg Asp
    50                  55
```

<210> 46
<211> 58
<212> PRT
<213> Homo sapiens

<400> 46

Met His Ser Phe Cys Ala Phe Lys Ala Asp Asp Gly Pro Cys Ile Ala
1 5 10 15

Ile Phe Lys Arg Phe Phe Phe Asn Ile Phe Thr Arg Gln Cys Glu Glu
20 25 30

Phe Ile Tyr Gly Gly Cys Glu Gly Asn Gln Asn Arg Phe Glu Ser Leu
35 40 45

Glu Glu Cys Lys Lys Met Cys Thr Arg Asp
50 55

<210> 47
<211> 58
<212> PRT
<213> Homo sapiens

<400> 47

Met His Ser Phe Cys Ala Phe Lys Ala Tyr Thr Gly Pro Cys Ile Ala
1 5 10 15

Phe Phe Lys Arg Phe Phe Phe Asn Ile Phe Thr Arg Gln Cys Glu Thr
20 25 30

Phe Ile Tyr Gly Gly Cys Glu Gly Asn Gln Asn Arg Phe Glu Ser Leu
35 40 45

Glu Glu Cys Lys Lys Met Cys Thr Arg Asp
50 55

<210> 48
<211> 58
<212> PRT
<213> Homo sapiens

<400> 48

Lys Pro Asp Phe Cys Phe Leu Glu Glu Asp Pro Gly Ile Cys Arg Gly
1 5 10 15

Tyr Ile Thr Arg Tyr Phe Tyr Asn Asn Gln Thr Lys Gln Cys Glu Arg
20 25 30

Phe Lys Tyr Gly Gly Cys Leu Gly Asn Met Asn Asn Phe Glu Thr Leu
35 40 45

Glu Glu Cys Lys Asn Ile Cys Glu Asp Gly
50 55

<210> 49
<211> 58
<212> PRT
<213> Homo sapiens

<400> 49

Lys Pro Asp Phe Cys Phe Leu Glu Glu Ser Ala Gly Pro Cys Val Ala
1 5 10 15

Met Phe Pro Arg Tyr Phe Tyr Asn Asn Gln Thr Lys Gln Cys Glu Thr
20 25 30

Phe Val Tyr Gly Gly Cys Met Gly Asn Gly Asn Asn Phe Glu Thr Leu

35 40 45

Glu Glu Cys Lys Asn Ile Cys Glu Asp Gly
50 55

<210> 50
<211> 58
<212> PRT
<213> Homo sapiens

<400> 50

Lys Pro Asp Phe Cys Phe Leu Glu Glu Asp Pro Gly Ile Cys Val Gly
1 5 10 15

Tyr Phe Thr Arg Tyr Phe Tyr Asn Asn Gln Thr Lys Gln Cys Glu Arg
20 25 30

Phe Lys Tyr Gly Gly Cys Leu Gly Asn Met Asn Asn Phe Glu Thr Leu
35 40 45

Glu Glu Cys Lys Asn Ile Cys Glu Asp Gly
50 55

<210> 51
<211> 58
<212> PRT
<213> Homo sapiens

<400> 51

Lys Pro Asp Phe Cys Phe Leu Glu Glu Asp Pro Gly Ile Cys Val Gly
1 5 10 15

Phe Phe Thr Arg Tyr Phe Tyr Asn Asn Gln Thr Lys Gln Cys Glu Arg
20 25 30

Phe Val Tyr Gly Gly Cys Leu Gly Asn Met Asn Asn Phe Glu Thr Leu
35 40 45

Glu Glu Cys Lys Asn Ile Cys Glu Asp Gly
50 55

<210> 52
<211> 58
<212> PRT
<213> Homo sapiens

<400> 52

Lys Pro Asp Phe Cys Phe Leu Glu Glu Asp Pro Gly Ile Cys Val Gly
1 5 10 15

Phe Phe Thr Arg Tyr Phe Tyr Asn Ala Gln Thr Lys Gln Cys Glu Arg
20 25 30

Phe Val Tyr Gly Gly Cys Leu Gly Asn Met Asn Asn Phe Glu Thr Leu
35 40 45

Glu Glu Cys Lys Asn Ile Cys Glu Asp Gly
50 55

<210> 53
<211> 58

<212> PRT
<213> Homo sapiens

<400> 53

Lys Pro Asp Phe Cys Phe Leu Glu Glu Asp Pro Gly Pro Cys Val Gly
1 5 10 15

Phe Phe Gln Arg Tyr Phe Tyr Asn Ala Gln Thr Lys Gln Cys Glu Arg
20 25 30

Phe Val Tyr Gly Gly Cys Gln Gly Asn Met Asn Asn Phe Glu Thr Leu
35 40 45

Glu Glu Cys Lys Asn Ile Cys Glu Asp Gly
50 55

<210> 54
<211> 58
<212> PRT
<213> Homo sapiens

<400> 54

Lys Pro Asp Phe Cys Phe Leu Glu Glu Asp Pro Gly Pro Cys Val Gly
1 5 10 15

Phe Phe Thr Arg Tyr Phe Tyr Asn Asn Gln Thr Lys Gln Cys Glu Arg
20 25 30

Phe Val Tyr Gly Gly Cys Gln Gly Asn Met Asn Asn Phe Glu Thr Leu
35 40 45

Glu Glu Cys Lys Asn Ile Cys Glu Asp Gly
50 55

<210> 55
<211> 58
<212> PRT
<213> Homo sapiens

<400> 55

Lys Pro Asp Phe Cys Phe Leu Glu Glu Asp Pro Gly Pro Cys Ile Gly
1 5 10 15

Phe Phe Pro Arg Tyr Phe Tyr Asn Asn Gln Thr Lys Gln Cys Glu Arg
20 25 30

Phe Val Tyr Gly Gly Cys Gln Gly Asn Met Asn Asn Phe Glu Thr Leu
35 40 45

Glu Glu Cys Lys Asn Ile Cys Glu Asp Gly
50 55

<210> 56
<211> 58
<212> PRT
<213> Homo sapiens

<400> 56

Gly Pro Ser Trp Cys Leu Thr Pro Ala Asp Arg Gly Leu Cys Arg Ala
1 5 10 15

Asn Glu Asn Arg Phe Tyr Tyr Asn Ser Val Ile Gly Lys Cys Arg Pro
20 25 30

Phe Lys Tyr Ser Gly Cys Gly Gly Asn Glu Asn Asn Phe Thr Ser Lys
35 40 45

Gln Glu Cys Leu Arg Ala Cys Lys Lys Gly
50 55

<210> 57
<211> 58
<212> PRT
<213> Homo sapiens

<400> 57

Gly Pro Ser Trp Cys Leu Thr Pro Ala Val Arg Gly Pro Cys Ile Ala
1 5 10 15

Phe Phe Pro Arg Trp Tyr Tyr Asn Ser Val Ile Gly Lys Cys Val Leu
20 25 30

Phe Pro Tyr Gly Gly Cys Gln Gly Asn Gly Asn Asn Phe Thr Ser Lys
35 40 45

Gln Glu Cys Leu Arg Ala Cys Lys Lys Gly
50 55

<210> 58
<211> 58
<212> PRT
<213> Homo sapiens

<400> 58

Gly Pro Ser Trp Cys Leu Thr Pro Ala Asp Arg Gly Leu Cys Val Ala
1 5 10 15

Asn Phe Asn Arg Phe Tyr Tyr Asn Ser Val Ile Gly Lys Cys Arg Pro
20 25 30

Phe Lys Tyr Ser Gly Cys Gly Gly Asn Glu Asn Asn Phe Thr Ser Lys
35 40 45

Gln Glu Cys Leu Arg Ala Cys Lys Lys Gly
50 55

<210> 59
<211> 58
<212> PRT
<213> Homo sapiens

<400> 59

Gly Pro Ser Trp Cys Leu Thr Pro Ala Asp Arg Gly Leu Cys Val Ala
1 5 10 15

Phe Phe Asn Arg Phe Tyr Tyr Asn Ser Val Ile Gly Lys Cys Arg Pro
20 25 30

Phe Lys Tyr Ser Gly Cys Gly Gly Asn Glu Asn Asn Phe Lys Ser Lys
35 40 45

Gln Glu Cys Leu Arg Ala Cys Lys Lys Gly
50 55

<210> 60
<211> 58
<212> PRT
<213> Homo sapiens

<400> 60

Gly Pro Ser Trp Cys Leu Thr Pro Ala Val Arg Gly Pro Cys Val Ala
1 5 10 15

Phe Phe Asn Arg Phe Tyr Tyr Asn Ser Val Ile Gly Lys Cys Arg Pro
20 25 30

Phe Lys Tyr Gly Gly Cys Gly Gly Asn Glu Asn Asn Phe Lys Ser Lys
35 40 45

Gln Glu Cys Leu Arg Ala Cys Lys Lys Gly
50 55

<210> 61
<211> 58
<212> PRT
<213> Homo sapiens

<400> 61

Gly Pro Ser Trp Cys Leu Thr Pro Ala Asp Arg Gly Pro Cys Ile Ala
1 5 10 15

Phe Phe Pro Arg Trp Tyr Tyr Asn Ser Val Ile Gly Lys Cys Gln Thr
20 25 30

Phe Val Tyr Gly Gly Cys Gly Gly Asn Glu Asn Asn Phe Ala Ser Lys
35 40 45

Gln Glu Cys Leu Arg Ala Cys Lys Lys Gly
50 55

<210> 62
<211> 58
<212> PRT
<213> Homo sapiens

<400> 62

Glu Thr Asp Ile Cys Lys Leu Pro Lys Asp Glu Gly Thr Cys Arg Asp
1 5 10 15

Phe Ile Leu Lys Trp Tyr Tyr Asp Pro Asn Thr Lys Ser Cys Ala Arg
20 25 30

Phe Trp Tyr Gly Gly Cys Gly Gly Asn Glu Asn Lys Phe Gly Ser Gln
35 40 45

Lys Glu Cys Glu Lys Val Cys Ala Pro Val
50 55

<210> 63
<211> 58
<212> PRT
<213> Homo sapiens

<400> 63

Glu Thr Asp Ile Cys Lys Leu Pro Lys Val Arg Gly Pro Cys Ile Ala
1               5                   10                  15

Phe Phe Pro Arg Trp Tyr Tyr Asp Pro Asn Thr Lys Ser Cys Val Leu
            20                  25                  30

Phe Pro Tyr Gly Gly Cys Gln Gly Asn Gly Asn Lys Phe Gly Ser Gln
        35                  40                  45

Lys Glu Cys Glu Lys Val Cys Ala Pro Val
    50                  55

<210> 64
<211> 58
<212> PRT
<213> Homo sapiens

<400> 64

Glu Thr Asp Ile Cys Lys Leu Pro Lys Asp Glu Gly Thr Cys Ile Ala
1               5                   10                  15

Phe Phe Leu Lys Trp Tyr Tyr Asp Pro Asn Thr Lys Ser Cys Ala Arg
            20                  25                  30

Phe Val Tyr Gly Gly Cys Gly Gly Asn Glu Asn Lys Phe Gly Ser Gln
        35                  40                  45

Lys Glu Cys Glu Lys Val Cys Ala Pro Val
    50                  55

<210> 65
<211> 58
<212> PRT
<213> Homo sapiens

<400> 65

Glu Thr Asp Ile Cys Lys Leu Pro Lys Asp Glu Gly Pro Cys Ile Ala
1               5                   10                  15

Phe Phe Leu Arg Trp Tyr Tyr Asp Pro Asn Thr Lys Ser Cys Ala Arg
            20                  25                  30

Phe Val Tyr Gly Gly Cys Gly Gly Asn Glu Asn Lys Phe Gly Ser Gln
        35                  40                  45

Lys Glu Cys Glu Lys Val Cys Ala Pro Val
    50                  55

<210> 66
<211> 58
<212> PRT
<213> Homo sapiens

<400> 66

Leu Pro Asn Val Cys Ala Phe Pro Met Glu Lys Gly Pro Cys Gln Thr
1               5                   10                  15

Tyr Met Thr Arg Trp Phe Phe Asn Phe Glu Thr Gly Glu Cys Glu Leu
            20                  25                  30

Phe Ala Tyr Gly Gly Cys Gly Gly Asn Ser Asn Asn Phe Leu Arg Lys

35 40 45

Glu Lys Cys Glu Lys Phe Cys Lys Phe Thr
50 55

<210> 67
<211> 58
<212> PRT
<213> Homo sapiens

<400> 67

Leu Pro Asn Val Cys Ala Phe Pro Met Val Arg Gly Pro Cys Ile Ala
1 5 10 15

Phe Phe Pro Arg Trp Phe Phe Asn Phe Glu Thr Gly Glu Cys Val Leu
20 25 30

Phe Val Tyr Gly Gly Cys Gln Gly Asn Gly Asn Asn Phe Leu Arg Lys
35 40 45

Glu Lys Cys Glu Lys Phe Cys Lys Phe Thr
50 55

<210> 68
<211> 58
<212> PRT
<213> Homo sapiens

<400> 68

Leu Pro Asn Val Cys Ala Phe Pro Met Glu Lys Gly Pro Cys Ile Ala
1 5 10 15

Tyr Phe Thr Arg Trp Phe Phe Asn Phe Glu Thr Gly Glu Cys Glu Leu
20 25 30

Phe Ala Tyr Gly Gly Cys Gly Gly Asn Ser Asn Asn Phe Leu Arg Lys
35 40 45

Glu Lys Cys Glu Lys Phe Cys Lys Phe Thr
50 55

<210> 69
<211> 58
<212> PRT
<213> Homo sapiens

<400> 69

Leu Pro Asn Val Cys Ala Phe Pro Met Glu Lys Gly Pro Cys Ile Ala
1 5 10 15

Tyr Phe Pro Arg Trp Phe Phe Asn Phe Glu Thr Gly Glu Cys Val Leu
20 25 30

Phe Val Tyr Gly Gly Cys Gly Gly Asn Ser Asn Asn Phe Leu Arg Lys
35 40 45

Glu Lys Cys Glu Lys Phe Cys Lys Phe Thr
50 55

<210> 70
<211> 8157

<212> DNA
<213> Artificial sequence

<220>

<223> Synthetic sequence pHIL-D2

<400> 70
agatcgcggc cgcgatctaa catccaaaga cgaaaggttg aatgaaacct ttttgccatc 60

cgacatccac aggtccattc tcacacataa gtgccaaacg caacaggagg ggatacacta 120

gcagcagacc gttgcaaacg caggacctcc actcctcttc tcctcaacac ccacttttgc 180

catcgaaaaa ccagcccagt tattgggctt gattggagct cgctcattcc aattccttct 240
attaggctac taacaccatg actttattag cctgtctatc ctggcccccc tggcgaggtc 300

atgtttgttt atttccgaat gcaacaagct ccgcattaca cccgaacatc actccagatg 360

agggctttct gagtgtgggg tcaaatagtt tcatgttccc aaatggccca aaactgacag 420

tttaaacgct gtcttggaac ctaatatgac aaaagcgtga tctcatccaa gatgaactaa 480

gtttggttcg ttgaaatgct aacggccagt tggtcaaaaa gaaacttcca aaagtcgcca 540

taccgtttgt cttgtttggt attgattgac gaatgctcaa aaataatctc attaatgctt 600

agcgcagtct ctctatcgct tctgaacccg gtggcacctg tgccgaaacg caaatgggga 660

aacaacccgc tttttggatg attatgcatt gtcctccaca ttgtatgctt ccaagattct 720

ggtgggaata ctgctgatag cctaacgttc atgatcaaaa tttaactgtt ctaaccccta 780

cttgacaggc aatatataaa cagaaggaag ctgccctgtc ttaaaccttt ttttttatca 840

tcattattag cttactttca taattgcgac tggttccaat tgacaagctt ttgattttaa 900

cgacttttaa cgacaacttg agaagatcaa aaaacaacta attattcgaa acgaggaatt 960

cgccttagac atgactgttc ctcagttcaa gttgggcatt acgagaagac cggtcttgct 1020

agattctaat caagaggatg tcagaatgcc atttgcctga gagatgcagg cttcattttt 1080

gatacttttt tatttgtaac ctatatagta taggattttt tttgtcattt tgtttcttct 1140

cgtacgagct tgctcctgat cagcctatct cgcagctgat gaatatcttg tggtaggggt 1200

ttgggaaaat cattcgagtt tgatgttttt cttggtattt cccactcctc ttcagagtac 1260

agaagattaa gtgagaagtt cgtttgtgca agcttatcga taagctttaa tgcggtagtt 1320

tatcacagtt aaattgctaa cgcagtcagg caccgtgtat gaaatctaac aatgcgctca 1380

tcgtcatcct cggcaccgtc accctggatg ctgtaggcat aggcttggtt atgccggtac 1440

tgccgggcct cttgcgggat atcgtccatt ccgacagcat cgccagtcac tatggcgtgc 1500

tgctagcgct atatgcgttg atgcaatttc tatgcgcacc cgttctcgga gcactgtccg 1560

accgctttgg ccgccgccca gtcctgctcg cttcgctact tggagccact atcgactacg 1620

cgatcatggc gaccacaccc gtcctgtgga tctatcgaat ctaaatgtaa gttaaaatct 1680

ctaaataatt aaataagtcc cagtttctcc atacgaacct taacagcatt gcggtgagca 1740

tctagacctt caacagcagc cagatccatc actgcttggc caatatgttt cagtccctca 1800

ggagttacgt cttgtgaagt gatgaacttc tggaaggttg cagtgttaac tccgctgtat 1860

```
tgacgggcat atccgtacgt tggcaaagtg tggttggtac cggaggagta atctccacaa 1920
ctctctggag agtaggcacc aacaaacaca gatccagcgt gttgtacttg atcaacataa 1980
gaagaagcat tctcgatttg caggatcaag tgttcaggag cgtactgatt ggacatttcc 2040
aaagcctgct cgtaggttgc aaccgatagg gttgtagagt gtgcaataca cttgcgtaca 2100
atttcaaccc ttggcaactg cacagcttgg ttgtgaacag catcttcaat tctggcaagc 2160
tccttgtctg tcatatcgac agccaacaga atcacctggg aatcaatacc atgttcagct 2220
tgagcagaag gtctgaggca acgaaatctg gatcagcgta tttatcagca ataactagaa 2280
cttcagaagg cccagcaggc atgtcaatac tacacagggc tgatgtgtca ttttgaacca 2340
tcatcttggc agcagtaacg aactggtttc ctggaccaaa tattttgtca cacttaggaa 2400
cagtttctgt tccgtaagcc atagcagcta ctgcctgggc gcctcctgct agcacgatac 2460
acttagcacc aaccttgtgg gcaacgtaga tgacttctgg ggtaagggta ccatccttct 2520
taggtggaga tgcaaaaaca atttctttgc aaccagcaac tttggcagga acacccagca 2580
tcagggaagt ggaaggcaga attgcggttc caccaggaat atagaggcca actttctcaa 2640
taggtcttgc aaaacgagag cagactacac cagggcaagt ctcaacttgc aacgtctccg 2700
ttagttgagc ttcatggaat ttcctgacgt tatctataga gagatcaatg gctctcttaa 2760
cgttatctgg caattgcata agttcctctg ggaaaggagc ttctaacaca ggtgtcttca 2820
aagcgactcc atcaaacttg gcagttagtt ctaaaagggc tttgtcacca ttttgacgaa 2880
cattgtcgac aattggtttg actaattcca taatctgttc cgttttctgg ataggacgac 2940
gaagggcatc ttcaatttct tgtgaggagg ccttagaaac gtcaattttg cacaattcaa 3000
tacgaccttc agaagggact tctttaggtt tggattcttc tttaggttgt tccttggtgt 3060
atcctggctt ggcatctcct ttccttctag tgacctttag ggacttcata tccaggtttc 3120
tctccacctc gtccaacgtc acaccgtact tggcacatct aactaatgca aaataaaata 3180
agtcagcaca ttcccaggct atatcttcct tggatttagc ttctgcaagt tcatcagctt 3240
cctccctaat tttagcgttc aacaaaactt cgtcgtcaaa taaccgtttg gtataagaac 3300
cttctggagc attgctctta cgatcccaca aggtgcttcc atggctctaa gaccctttga 3360
ttggccaaaa caggaagtgc gttccaagtg acagaaacca acacctgttt gttcaaccac 3420
aaatttcaag cagtctccat cacaatccaa ttcgataccc agcaactttt gagttcgtcc 3480
agatgtagca cctttatacc acaaaccgtg acgacgagat tggtagactc cagtttgtgt 3540
ccttatagcc tccggaatag actttttgga cgagtacacc aggcccaacg agtaattaga 3600
agagtcagcc accaaagtag tgaatagacc atcggggcgg tcagtagtca aagacgccaa 3660
caaaatttca ctgacaggga actttttgac atcttcagaa agttcgtatt cagtagtcaa 3720
ttgccgagca tcaataatgg ggattatacc agaagcaaca gtggaagtca catctaccaa 3780
ctttgcggtc tcagaaaaag cataaacagt tctactaccg ccattagtga aacttttcaa 3840
atcgcccagt ggagaagaaa aaggcacagc gatactagca ttagcgggca aggatgcaac 3900
tttatcaacc agggtcctat agataaccct agcgcctggg atcatccttt ggacaactct 3960
ttctgccaaa tctaggtcca aaatcacttc attgatacca ttatacggat gactcaactt 4020
```

gcacattaac ttgaagctca gtcgattgag tgaacttgat caggttgtgc agctggtcag 4080
cagcataggg aaacacggct tttcctacca aactcaagga attatcaaac tctgcaacac 4140
ttgcgtatgc aggtagcaag ggaaatgtca tacttgaagt cggacagtga gtgtagtctt 4200
gagaaattct gaagccgtat ttttattatc agtgagtcag tcatcaggag atcctctacg 4260
ccggacgcat cgtggccggc atcaccggcg ccacaggtgc ggttgctggc gcctatatcg 4320
ccgacatcac cgatggggaa gatcgggctc gccacttcgg gctcatgagc gcttgtttcg 4380
gcgtgggtat ggtggcaggc cccgtggccg ggggactgtt gggcgccatc tccttgcatg 4440
caccattcct tgcggcggcg gtgctcaacg gcctcaacct actactgggc tgcttcctaa 4500
tgcaggagtc gcataaggga gagcgtcgag tatctatgat tggaagtatg ggaatggtga 4560
tacccgcatt cttcagtgtc ttgaggtctc ctatcagatt atgcccaact aaagcaaccg 4620
gaggaggaga tttcatggta aatttctctg acttttggtc atcagtagac tcgaactgtg 4680
agactatctc ggttatgaca gcagaaatgt ccttcttgga gacagtaaat gaagtcccac 4740
caataaagaa atccttgtta tcaggaacaa acttcttgtt tcgaactttt tcggtgcctt 4800
gaactataaa atgtagagtg gatatgtcgg gtaggaatgg agcgggcaaa tgcttacctt 4860
ctggaccttc aagaggtatg tagggtttgt agatactgat gccaacttca gtgacaacgt 4920
tgctatttcg ttcaaaccat tccgaatcca gagaaatcaa agttgtttgt ctactattga 4980
tccaagccag tgcggtcttg aaactgacaa tagtgtgctc gtgttttgag gtcatctttg 5040
tatgaataaa tctagtcttt gatctaaata atcttgacga gccaaggcga taaataccca 5100
aatctaaaac tcttttaaaa cgttaaaagg acaagtatgt ctgcctgtat taaaccccaa 5160
atcagctcgt agtctgatcc tcatcaactt gaggggcact atcttgtttt agagaaattt 5220
gcggagatgc gatatcgaga aaaaggtacg ctgattttaa acgtgaaatt tatctcaaga 5280
tcgcggccgc gatctcgaat aataactgtt atttttcagt gttcccgatc tgcgtctatt 5340
tcacaatacc aacatgagtc agcttatcga tgataagctg tcaaacatga gaattaattc 5400
gatgataagc tgtcaaacat gagaaatctt gaagacgaaa gggcctcgtg atacgcctat 5460
ttttataggt taatgtcatg ataataatgg tttcttagac gtcaggtggc acttttcggg 5520
gaaatgtgcg cggaacccct atttgtttat ttttctaaat acattcaaat atgtatccgc 5580
tcatgagaca ataaccctga taaatgcttc aataatattg aaaaaggaag agtatgagta 5640
ttcaacattt ccgtgtcgcc cttattccct tttttgcggc attttgcctt cctgtttttg 5700
ctcacccaga aacgctggtg aaagtaaaag atgctgaaga tcagttgggt gcacgagtgg 5760
gttacatcga actggatctc aacagcggta agatccttga gagttttcgc cccgaagaac 5820
gttttccaat gatgagcact tttaaagttc tgctatgtgg cgcggtatta tcccgtgttg 5880
acgccgggca agagcaactc ggtcgccgca tacactattc tcagaatgac ttggttgagt 5940
actcaccagt cacagaaaag catcttacgg atggcatgac agtaagagaa ttatgcagtg 6000
ctgccataac catgagtgat aacactgcgg ccaacttact tctgacaacg atcggaggac 6060
cgaaggagct aaccgctttt ttgcacaaca tgggggatca tgtaactcgc cttgatcgtt 6120

gggaaccgga gctgaatgaa gccataccaa acgacgagcg tgacaccacg atgcctgcag 6180
caatggcaac aacgttgcgc aaactattaa ctggcgaact acttactcta gcttcccggc 6240
aacaattaat agactggatg gaggcggata aagttgcagg accacttctg cgctcggccc 6300
ttccggctgg ctggtttatt gctgataaat ctggagccgg tgagcgtggg tctcgcggta 6360
tcattgcagc actggggcca gatggtaagc cctcccgtat cgtagttatc tacacgacgg 6420
ggagtcaggc aactatggat gaacgaaata gacagatcgc tgagataggt gcctcactga 6480
ttaagcattg gtaactgtca gaccaagttt actcatatat actttagatt gatttaaatt 6540
gtaaacgtta atattttgtt aaaattcgcg ttaaattttt gttaaatcag ctcatttttt 6600
aaccaatagg ccgaaatcgg caaaatccct tataaatcaa aagaatagac cgagataggg 6660
ttgagtgttg ttccagtttg gaacaagagt ccactattaa agaacgtgga ctccaacgtc 6720
aaagggcgaa aaaccgtcta tcagggcgat ggcccactac gtgaaccatc accctaatca 6780
agttttttgg ggtcgaggtg ccgtaaagca ctaaatcgga accctaaagg gagcccccga 6840
tttagagctt gacggggaaa gccggcgaac gtggcgagaa aggaagggaa gaaagcgaaa 6900
ggagcgggcg ctagggcgct ggcaagtgta gcggtcacgc tgcgcgtaac caccacaccc 6960
gccgcgctta atgcgccgct acagggcgcg taaaaggatc taggtgaaga tcctttttga 7020
taatctcatg accaaaatcc cttaacgtga gttttcgttc cactgagcgt cagaccccgt 7080
agaaaagatc aaaggatctt cttgagatcc tttttttctg cgcgtaatct gctgcttgca 7140
aacaaaaaaa ccaccgctac cagcggtggt ttgtttgccg gatcaagagc taccaactct 7200
ttttccgaag gtaactggct tcagcagagc gcagatacca aatactgtcc ttctagtgta 7260
gccgtagtta ggccaccact tcaagaactc tgtagcaccg cctacatacc tcgctctgct 7320
aatcctgtta ccagtggctg ctgccagtgg cgataagtcg tgtcttaccg ggttggactc 7380
aagacgatag ttaccggata aggcgcagcg gtcgggctga acggggggtt cgtgcacaca 7440
gcccagcttg gagcgaacga cctacaccga actgagatac ctacagcgtg agcattgaga 7500
aagcgccacg cttcccgaag ggagaaaggc ggacaggtat ccggtaagcg gcagggtcgg 7560
aacaggagag cgcacgaggg agcttccagg gggaaacgcc tggtatcttt atagtcctgt 7620
cgggtttcgc cacctctgac ttgagcgtcg atttttgtga tgctcgtcag gggggcggag 7680
cctatggaaa aacgccagca acgcggcctt tttacggttc ctggcctttt gctggccttt 7740
tgctcacatg ttctttcctg cgttatcccc tgattctgtg gataaccgta ttaccgcctt 7800
tgagtgagct gataccgctc gccgcagccg aacgaccgag cgcagcgagt cagtgagcga 7860
ggaagcggaa gagcgcctga tgcggtattt tctccttacg catctgtgcg gtatttcaca 7920
ccgcatatgg tgcactctca gtacaatctg ctctgatgcc gcatagttaa gccagtatac 7980
actccgctat cgctacgtga ctgggtcatg gctgcgcccc gacacccgcc aacacccgct 8040
gacgcgccct gacgggcttg tctgctcccg gcatccgctt acagacaagc tgtgaccgtc 8100
tccgggagct gcatgtgtca gaggttttca ccgtcatcac cgaaacgcgc gaggcag 8157

<210> 71
<211> 8584
<212> DNA
<213> Artificial sequence

<220>

<223> Synthetic sequence pHIL-D2 (MFalphaPrePro::EPI-HNE-3)

<400> 71
agatcgcggc cgcgatctaa catccaaaga cgaaaggttg aatgaaacct ttttgccatc 60
cgacatccac aggtccattc tcacacataa gtgccaaacg caacaggagg ggatacacta 120
gcagcagacc gttgcaaacg caggacctcc actcctcttc tcctcaacac ccacttttgc 180
catcgaaaaa ccagcccagt tattgggctt gattggagct cgctcattcc aattccttct 240
attaggctac taacaccatg actttattag cctgtctatc ctggcccccc tggcgaggtc 300
atgtttgttt atttccgaat gcaacaagct ccgcattaca cccgaacatc actccagatg 360
agggctttct gagtgtgggg tcaaatagtt tcatgttccc aaatggccca aaactgacag 420
tttaaacgct gtcttggaac ctaatatgac aaaagcgtga tctcatccaa gatgaactaa 480
gtttggttcg ttgaaatgct aacggccagt tggtcaaaaa gaaacttcca aaagtcgcca 540
taccgtttgt cttgtttggt attgattgac gaatgctcaa aaataatctc attaatgctt 600
agcgcagtct ctctatcgct tctgaacccg gtggcacctg tgccgaaacg caaatgggga 660
aacaacccgc tttttggatg attatgcatt gtcctccaca ttgtatgctt ccaagattct 720
ggtgggaata ctgctgatag cctaacgttc atgatcaaaa tttaactgtt ctaaccccta 780
cttgacaggc aatatataaa cagaaggaag ctgccctgtc ttaaaccttt ttttttatca 840
tcattattag cttactttca taattgcgac tggttccaat tgacaagctt ttgattttaa 900
cgacttttaa cgacaacttg agaagatcaa aaaacaacta attattcgaa acgatgagat 960
ttccatctat cttcactgct gttttgttcg ctgcttcctc tgctttggct gctccagtta 1020
acaccactac tgaagacgag actgctcaaa ttcctgctga ggctgtcatc ggttactctg 1080
acttggaagg tgacttcgac gtcgctgttt tgccattctc taactctact aacaacggtt 1140
tgttgttcat caacactacc atcgcttcta tcgctgctaa ggaggaaggt gtttccttgg 1200
acaagagagc tgcttgtaac ttgccaatcg tcagaggtcc atgcattgct ttcttcccaa 1260
gatgggcttt cgacgctgtt aagggtaagt gcgtcttgtt cccatacggt ggttgtcaag 1320
gtaacggtaa caagttctac tctgagaagg agtgtagaga gtactgtggt gttccatagt 1380
aagaattcgc cttagacatg actgttcctc agttcaagtt gggcattacg agaagaccgg 1440
tcttgctaga ttctaatcaa gaggatgtca gaatgccatt tgcctgagag atgcaggctt 1500
catttttgat acttttttat ttgtaaccta tatagtatag gatttttttt gtcattttgt 1560
ttcttctcgt acgagcttgc tcctgatcag cctatctcgc agctgatgaa tatcttgtgg 1620
taggggtttg ggaaaatcat tcgagtttga tgtttttctt ggtatttccc actcctcttc 1680
agagtacaga agattaagtg agaagttcgt ttgtgcaagc ttatcgataa gctttaatgc 1740
ggtagtttat cacagttaaa ttgctaacgc agtcaggcac cgtgtatgaa atctaacaat 1800

gcgctcatcg tcatcctcgg caccgtcacc ctggatgctg taggcatagg cttggttatg 1860
ccggtactgc cgggcctctt gcgggatatc gtccattccg acagcatcgc cagtcactat 1920
ggcgtgctgc tagcgctata tgcgttgatg caatttctat gcgcacccgt tctcggagca 1980
ctgtccgacc gctttggccg ccgcccagtc ctgctcgctt cgctacttgg agccactatc 2040
gactacgcga tcatggcgac cacacccgtc ctgtggatct atcgaatcta aatgtaagtt 2100
aaaatctcta aataattaaa taagtcccag tttctccata cgaaccttaa cagcattgcg 2160
gtgagcatct agaccttcaa cagcagccag atccatcact gcttggccaa tatgtttcag 2220
tccctcagga gttacgtctt gtgaagtgat gaacttctgg aaggttgcag tgttaactcc 2280
gctgtattga cgggcatatc cgtacgttgg caaagtgtgg ttggtaccgg aggagtaatc 2340
tccacaactc tctggagagt aggcaccaac aaacacagat ccagcgtgtt gtacttgatc 2400
aacataagaa gaagcattct cgatttgcag gatcaagtgt tcaggagcgt actgattgga 2460
catttccaaa gcctgctcgt aggttgcaac cgatagggtt gtagagtgtg caatacactt 2520
gcgtacaatt tcaacccttg gcaactgcac agcttggttg tgaacagcat cttcaattct 2580
ggcaagctcc ttgtctgtca tatcgacagc caacagaatc acctgggaat caataccatg 2640
ttcagcttga gcagaaggtc tgaggcaacg aaatctggat cagcgtattt atcagcaata 2700
actagaactt cagaaggccc agcaggcatg tcaatactac acagggctga tgtgtcattt 2760
tgaaccatca tcttggcagc agtaacgaac tggtttcctg gaccaaatat tttgtcacac 2820
ttaggaacag tttctgttcc gtaagccata gcagctactg cctgggcgcc tcctgctagc 2880
acgatacact tagcaccaac cttgtgggca acgtagatga cttctggggt aagggtacca 2940
tccttcttag gtggagatgc aaaaacaatt tctttgcaac cagcaacttt ggcaggaaca 3000
cccagcatca gggaagtgga aggcagaatt gcggttccac caggaatata gaggccaact 3060
ttctcaatag gtcttgcaaa acgagagcag actacaccag ggcaagtctc aacttgcaac 3120
gtctccgtta gttgagcttc atggaatttc ctgacgttat ctatagagag atcaatggct 3180
ctcttaacgt tatctggcaa ttgcataagt tcctctggga aaggagcttc taacacaggt 3240
gtcttcaaag cgactccatc aaacttggca gttagttcta aaagggcttt gtcaccattt 3300
tgacgaacat tgtcgacaat tggtttgact aattccataa tctgttccgt tttctggata 3360
ggacgacgaa gggcatcttc aatttcttgt gaggaggcct tagaaacgtc aattttgcac 3420
aattcaatac gaccttcaga agggacttct ttaggtttgg attcttcttt aggttgttcc 3480
ttggtgtatc ctggcttggc atctcctttc cttctagtga cctttaggga cttcatatcc 3540
aggtttctct ccacctcgtc caacgtcaca ccgtacttgg cacatctaac taatgcaaaa 3600
taaaataagt cagcacattc ccaggctata tcttccttgg atttagcttc tgcaagttca 3660
tcagcttcct ccctaatttt agcgttcaac aaaacttcgt cgtcaaataa ccgtttggta 3720
taagaacctt ctggagcatt gctcttacga tcccacaagg tgcttccatg gctctaagac 3780
cctttgattg gccaaaacag gaagtgcgtt ccaagtgaca gaaaccaaca cctgtttgtt 3840
caaccacaaa tttcaagcag tctccatcac aatccaattc gatacccagc aacttttgag 3900

ttcgtccaga tgtagcacct ttataccaca aaccgtgacg acgagattgg tagactccag 3960
tttgtgtcct tatagcctcc ggaatagact ttttggacga gtacaccagg cccaacgagt 4020
aattagaaga gtcagccacc aaagtagtga atagaccatc ggggcggtca gtagtcaaag 4080
acgccaacaa aatttcactg acagggaact ttttgacatc ttcagaaagt tcgtattcag 4140
tagtcaattg ccgagcatca ataatgggga ttataccaga agcaacagtg gaagtcacat 4200
ctaccaactt tgcggtctca gaaaaagcat aaacagttct actaccgcca ttagtgaaac 4260
ttttcaaatc gcccagtgga gaagaaaaag gcacagcgat actagcatta gcgggcaagg 4320
atgcaacttt atcaaccagg gtcctataga taaccctagc gcctgggatc atcctttgga 4380
caactctttc tgccaaatct aggtccaaaa tcacttcatt gataccatta tacggatgac 4440
tcaacttgca cattaacttg aagctcagtc gattgagtga acttgatcag gttgtgcagc 4500
tggtcagcag catagggaaa cacggctttt cctaccaaac tcaaggaatt atcaaactct 4560
gcaacacttg cgtatgcagg tagcaaggga aatgtcatac ttgaagtcgg acagtgagtg 4620
tagtcttgag aaattctgaa gccgtatttt tattatcagt gagtcagtca tcaggagatc 4680
ctctacgccg gacgcatcgt ggccggcatc accggcgcca caggtgcggt tgctggcgcc 4740
tatatcgccg acatcaccga tggggaagat cgggctcgcc acttcgggct catgagcgct 4800
tgtttcggcg tgggtatggt ggcaggcccc gtggccgggg gactgttggg cgccatctcc 4860
ttgcatgcac cattccttgc ggcggcggtg ctcaacggcc tcaacctact actgggctgc 4920
ttcctaatgc aggagtcgca taagggagag cgtcgagtat ctatgattgg aagtatggga 4980
atggtgatac ccgcattctt cagtgtcttg aggtctccta tcagattatg cccaactaaa 5040
gcaaccggag gaggagattt catggtaaat ttctctgact tttggtcatc agtagactcg 5100
aactgtgaga ctatctcggt tatgacagca gaaatgtcct tcttggagac agtaaatgaa 5160
gtcccaccaa taaagaaatc cttgttatca ggaacaaact tcttgtttcg aactttttcg 5220
gtgccttgaa ctataaaatg tagagtggat atgtcgggta ggaatggagc gggcaaatgc 5280
ttaccttctg gaccttcaag aggtatgtag ggtttgtaga tactgatgcc aacttcagtg 5340
acaacgttgc tatttcgttc aaaccattcc gaatccagag aaatcaaagt tgtttgtcta 5400
ctattgatcc aagccagtgc ggtcttgaaa ctgacaatag tgtgctcgtg ttttgaggtc 5460
atctttgtat gaataaatct agtctttgat ctaaataatc ttgacgagcc aaggcgataa 5520
atacccaaat ctaaaactct tttaaaacgt taaaaggaca agtatgtctg cctgtattaa 5580
accccaaatc agctcgtagt ctgatcctca tcaacttgag gggcactatc ttgttttaga 5640
gaaatttgcg gagatgcgat atcgagaaaa aggtacgctg attttaaacg tgaaatttat 5700
ctcaagatcg cggccgcgat ctcgaataat aactgttatt tttcagtgtt cccgatctgc 5760
gtctatttca caataccaac atgagtcagc ttatcgatga taagctgtca aacatgagaa 5820
ttaattcgat gataagctgt caaacatgag aaatcttgaa gacgaaaggg cctcgtgata 5880
cgcctatttt tataggttaa tgtcatgata ataatggttt cttagacgtc aggtggcact 5940
tttcggggaa atgtgcgcgg aacccctatt tgtttatttt tctaaataca ttcaaatatg 6000

```
tatccgctca tgagacaata accctgataa atgcttcaat aatattgaaa aaggaagagt   6060
atgagtattc aacatttccg tgtcgccctt attccctttt ttgcggcatt ttgccttcct   6120
gtttttgctc acccagaaac gctggtgaaa gtaaaagatg ctgaagatca gttgggtgca   6180
cgagtgggtt acatcgaact ggatctcaac agcggtaaga tccttgagag ttttcgcccc   6240
gaagaacgtt ttccaatgat gagcactttt aaagttctgc tatgtggcgc ggtattatcc   6300
cgtgttgacg ccgggcaaga gcaactcggt cgccgcatac actattctca gaatgacttg   6360
gttgagtact caccagtcac agaaaagcat cttacggatg gcatgacagt aagagaatta   6420
tgcagtgctg ccataaccat gagtgataac actgcggcca acttacttct gacaacgatc   6480
ggaggaccga aggagctaac cgcttttttg cacaacatgg gggatcatgt aactcgcctt   6540
gatcgttggg aaccggagct gaatgaagcc ataccaaacg acgagcgtga caccacgatg   6600
cctgcagcaa tggcaacaac gttgcgcaaa ctattaactg gcgaactact tactctagct   6660
tcccggcaac aattaataga ctggatggag gcggataaag ttgcaggacc acttctgcgc   6720
tcggcccttc cggctggctg gtttattgct gataaatctg gagccggtga gcgtgggtct   6780
cgcggtatca ttgcagcact ggggccagat ggtaagccct cccgtatcgt agttatctac   6840
acgacgggga gtcaggcaac tatggatgaa cgaaatagac agatcgctga gataggtgcc   6900
tcactgatta agcattggta actgtcagac caagtttact catatatact ttagattgat   6960
ttaaattgta aacgttaata ttttgttaaa attcgcgtta aatttttgtt aaatcagctc   7020
atttttaac caataggccg aaatcggcaa aatcccttat aaatcaaaag aatagaccga   7080
gatagggttg agtgttgttc cagtttggaa caagagtcca ctattaaaga acgtggactc   7140
caacgtcaaa gggcgaaaaa ccgtctatca gggcgatggc ccactacgtg aaccatcacc   7200
ctaatcaagt tttttggggt cgaggtgccg taaagcacta aatcggaacc ctaaagggag   7260
cccccgattt agagcttgac ggggaaagcc ggcgaacgtg gcgagaaagg aagggaagaa   7320
agcgaaagga gcgggcgcta gggcgctggc aagtgtagcg gtcacgctgc gcgtaaccac   7380
cacacccgcc gcgcttaatg cgccgctaca gggcgcgtaa aaggatctag gtgaagatcc   7440
tttttgataa tctcatgacc aaaatccctt aacgtgagtt ttcgttccac tgagcgtcag   7500
accccgtaga aaagatcaaa ggatcttctt gagatccttt ttttctgcgc gtaatctgct   7560
gcttgcaaac aaaaaaacca ccgctaccag cggtggtttg tttgccggat caagagctac   7620
caactctttt tccgaaggta actggcttca gcagagcgca gataccaaat actgtccttc   7680
tagtgtagcc gtagttaggc caccacttca agaactctgt agcaccgcct acatacctcg   7740
ctctgctaat cctgttacca gtggctgctg ccagtggcga taagtcgtgt cttaccgggt   7800
tggactcaag acgatagtta ccggataagg cgcagcggtc gggctgaacg gggggttcgt   7860
gcacacagcc cagcttggag cgaacgacct acaccgaact gagataccta cagcgtgagc   7920
attgagaaag cgccacgctt cccgaaggga gaaaggcgga caggtatccg gtaagcggca   7980
gggtcggaac aggagagcgc acgagggagc ttccaggggg aaacgcctgg tatctttata   8040
gtcctgtcgg gtttcgccac ctctgacttg agcgtcgatt tttgtgatgc tcgtcagggg   8100
```

ggcggagcct atggaaaaac gccagcaacg cggccttttt acggttcctg gccttttgct 8160

ggccttttgc tcacatgttc tttcctgcgt tatcccctga ttctgtggat aaccgtatta 8220

ccgcctttga gtgagctgat accgctcgcc gcagccgaac gaccgagcgc agcgagtcag 8280

tgagcgagga agcggaagag cgcctgatgc ggtattttct ccttacgcat ctgtgcggta 8340

tttcacaccg catatggtgc actctcagta caatctgctc tgatgccgca tagttaagcc 8400

agtatacact ccgctatcgc tacgtgactg ggtcatggct gcgccccgac acccgccaac 8460
acccgctgac gcgccctgac gggcttgtct gctcccggca tccgcttaca gacaagctgt 8520

gaccgtctcc gggagctgca tgtgtcagag gttttcaccg tcatcaccga aacgcgcgag 8580

gcag 8584

<210> 72
<211> 141
<212> PRT
<213> Artificial sequence

<220>

<223> Synthetic sequence pHIL-D2 (MFalphaPrePro::EPI-HNE-3)

<400> 72

Met Arg Phe Pro Ser Ile Phe Thr Ala Val Leu Phe Ala Ala Ser Ser
1 5 10 15

Ala Leu Ala Ala Pro Val Asn Thr Thr Thr Glu Asp Glu Thr Ala Gln
20 25 30

Ile Pro Ala Glu Ala Val Ile Gly Tyr Ser Asp Leu Glu Gly Asp Phe
35 40 45

Asp Val Ala Val Leu Pro Phe Ser Asn Ser Thr Asn Asn Gly Leu Leu
50 55 60

Phe Ile Asn Thr Thr Ile Ala Ser Ile Ala Ala Lys Glu Glu Gly Val
65 70 75 80

Ser Leu Asp Lys Arg Ala Ala Cys Asn Leu Pro Ile Val Arg Gly Pro
85 90 95

Cys Ile Ala Phe Phe Pro Arg Trp Ala Phe Asp Ala Val Lys Gly Lys
100 105 110

Cys Val Leu Phe Pro Tyr Gly Gly Cys Gln Gly Asn Gly Asn Lys Phe
115 120 125

Tyr Ser Glu Lys Glu Cys Arg Glu Tyr Cys Gly Val Pro
130 135 140

<210> 73
<211> 444
<212> DNA
<213> Artificial sequence

<220>

<223> Synthetic sequence EPI-HNE-4 cassette

<400> 73
ttcgaaacga tgagattccc atctatcttc actgctgttt tgttcgctgc ttcctctgct 60

ttggctgctc cagttaacac cactactgaa gacgagactg ctcaaattcc tgctgaggct 120

gtcatcggtt actctgactt ggaaggtgac ttcgacgtcg ctgttttgcc attctctaac 180
tctactaaca acggtttgtt gttcatcaac actaccatcg cttctatcgc tgctaaggag 240

gaaggtgttt ccttggacaa gagagaggct tgtaacttgc caatcgtcag aggtccatgc 300

attgctttct tcccaagatg ggctttcgac gctgttaagg gtaagtgcgt cttgttccca 360

tacggtggtt gtcaaggtaa cggtaacaag ttctactctg agaaggagtg tagagagtac 420

tgtggtgttc catagtaaga attc 444

<210> 74
<211> 141
<212> PRT
<213> Homo sapiens

<400> 74

Met Arg Phe Pro Ser Ile Phe Thr Ala Val Leu Phe Ala Ala Ser Ser
1 5 10 15

Ala Leu Ala Ala Pro Val Asn Thr Thr Thr Glu Asp Glu Thr Ala Gln
20 25 30

Ile Pro Ala Glu Ala Val Ile Gly Tyr Ser Asp Leu Glu Gly Asp Phe
35 40 45

Asp Val Ala Val Leu Pro Phe Ser Asn Ser Thr Asn Asn Gly Leu Leu
50 55 60

Phe Ile Asn Thr Thr Ile Ala Ser Ile Ala Ala Lys Glu Glu Gly Val
65 70 75 80

Ser Leu Asp Lys Arg Glu Ala Cys Asn Leu Pro Ile Val Arg Gly Pro
85 90 95

Cys Ile Ala Phe Phe Pro Arg Trp Ala Phe Asp Ala Val Lys Gly Lys
100 105 110

Cys Val Leu Phe Pro Tyr Gly Gly Cys Gln Gly Asn Gly Asn Lys Phe
115 120 125

Tyr Ser Glu Lys Glu Cys Arg Glu Tyr Cys Gly Val Pro
130 135 140

<210> 75
<211> 8590
<212> DNA
<213> Artificial sequence

<220>

<223> Synthetic sequence pD2pick (MFalphaPrePro::EPI-HNE-3)

<400> 75
agatcgcggc cgcgatctaa catccaaaga cgaaaggttg aatgaaacct ttttgccatc 60

cgacatccac aggtccattc tcacacataa gtgccaaacg caacaggagg ggatacacta 120

gcagcagacc gttgcaaacg caggacctcc actcctcttc tcctcaacac ccacttttgc 180
catcgaaaaa ccagcccagt tattgggctt gattggagct cgctcattcc aattccttct 240

attaggctac taacaccatg actttattag cctgtctatc ctggcccccc tggcgaggtc 300

```
atgtttgttt atttccgaat gcaacaagct ccgcattaca cccgaacatc actccagatg   360
agggctttct gagtgtgggg tcaaatagtt tcatgttccc aaatggccca aaactgacag   420
tttaaacgct gtcttggaac ctaatatgac aaaagcgtga tctcatccaa gatgaactaa   480
gtttggttcg ttgaaatgct aacggccagt tggtcaaaaa gaaacttcca aaagtcgcca   540
taccgtttgt cttgtttggt attgattgac gaatgctcaa aaataatctc attaatgctt   600
agcgcagtct ctctatcgct tctgaacccg gtggcacctg tgccgaaacg caaatgggga   660
aacaacccgc tttttggatg attatgcatt gtcctccaca ttgtatgctt ccaagattct   720
ggtgggaata ctgctgatag cctaacgttc atgatcaaaa tttaactgtt ctaacccta   780
cttgacaggc aatatataaa cagaaggaag ctgccctgtc ttaaaccttt tttttatca   840
tcattattag cttactttca taattgcgac tggttccaat tgacaagctt ttgattttaa   900
cgacttttaa cgacaacttg agaagatcaa aaaacaacta attattcgaa acgatgagat   960
tcccatctat cttcactgct gttttgttcg ctgcttcctc tgctttggct gctccagtta   1020
acaccactac tgaagacgag actgctcaaa ttcctgctga ggctgtcatc ggttactctg   1080
acttggaagg tgacttcgac gtcgctgttt tgccattctc taactctact aacaacggtt   1140
tgttgttcat caacactacc atcgcttcta tcgctgctaa ggaggaaggt gtttccttgg   1200
acaagagagc tgcttgtaac ttgccaatcg tcagaggtcc atgcattgct ttcttcccaa   1260
gatgggcttt cgacgctgtt aagggtaagt gcgtcttgtt cccatacggt ggttgtcaag   1320
gtaacggtaa caagttctac tctgagaagg agtgtagaga gtactgtggt gttccatagt   1380
aagaattcgc cttagacatg actgttcctc agttcaagtt gggcattacg agaagaccgg   1440
tcttgctaga ttctaatcaa gaggatgtca gaatgccatt tgcctgagag atgcaggctt   1500
catttttgat acttttttat ttgtaaccta tatagtatag gatttttttt gtcattttgt   1560
ttcttctcgt acgagcttgc tcctgatcag cctatctcgc agctgatgaa tatcttgtgg   1620
taggggtttg ggaaaatcat tcgagtttga tgtttttctt ggtatttccc actcctcttc   1680
agagtacaga agattaagtg agaagttcgt ttgtgcaagc ttatcgataa gctttaatgc   1740
ggtagtttat cacagttaaa ttgctaacgc agtcaggcac cgtgtatgaa atctaacaat   1800
gcgctcatcg tcatcctcgg caccgtcacc ctggatgctg taggcatagg cttggttatg   1860
ccggtactgc cgggcctctt gcgggatatc gtccattccg acagcatcgc cagtcactat   1920
ggcgtgctgc tagcgctata tgcgttgatg caatttctat gcgcacccgt tctcggagca   1980
ctgtccgacc gctttggccg ccgcccagtc ctgctcgctt cgctacttgg agccactatc   2040
gactacgcga tcatggcgac cacacccgtc ctgtggatct atcgaatcta aatgtaagtt   2100
aaaatctcta aataattaaa taagtcccag tttctccata cgaaccttaa cagcattgcg   2160
gtgagcatct agaccttcaa cagcagccag atccatcact gcttggccaa tatgtttcag   2220
tccctcagga gttacgtctt gtgaagtgat gaacttctgg aaggttgcag tgttaactcc   2280
gctgtattga cgggcatatc cgtacgttgg caaagtgtgg ttggtaccgg aggagtaatc   2340
tccacaactc tctggagagt aggcaccaac aaacacagat ccagcgtgtt gtacttgatc   2400
```

aacataagaa gaagcattct cgatttgcag gatcaagtgt tcaggagcgt actgattgga 2460
catttccaaa gcctgctcgt aggttgcaac cgatagggtt gtagagtgtg caatacactt 2520
gcgtacaatt tcaacccttg gcaactgcac agcttggttg tgaacagcat cttcaattct 2580
ggcaagctcc ttgtctgtca tatcgacagc caacagaatc acctgggaat caataccatg 2640
ttcagcttga gcagaaggtc tgaggcaacg aaatctggat cagcgtattt atcagcaata 2700
actagaactt cagaaggccc agcaggcatg tcaatactac acagggctga tgtgtcattt 2760
tgaaccatca tcttggcagc agtaacgaac tggtttcctg gaccaaatat tttgtcacac 2820
ttaggaacag tttctgttcc gtaagccata gcagctactg cctgggcgcc tcctgctagc 2880
acgatacact tagcaccaac cttgtgggca acgtagatga cttctggggt aagggtacca 2940
tccttcttag gtggagatgc aaaaacaatt tctttgcaac cagcaacttt ggcaggaaca 3000
cccagcatca gggaagtgga aggcagaatt gcggttccac caggaatata gaggccaact 3060
ttctcaatag gtcttgcaaa acgagagcag actacaccag ggcaagtctc aacttgcaac 3120
gtctccgtta gttgagcttc atggaatttc ctgacgttat ctatagagag atcaatggct 3180
ctcttaacgt tatctggcaa ttgcataagt tcctctggga aaggagcttc taacacaggt 3240
gtcttcaaag cgactccatc aaacttggca gttagttcta aaagggcttt gtcaccattt 3300
tgacgaacat tgtcgacaat tggtttgact aattccataa tctgttccgt tttctggata 3360
ggacgacgaa gggcatcttc aatttcttgt gaggaggcct tagaaacgtc aattttgcac 3420
aattcaatac gaccttcaga agggacttct ttaggtttgg attcttcttt aggttgttcc 3480
ttggtgtatc ctggcttggc atctcctttc cttctagtga cctttaggga cttcatatcc 3540
aggtttctct ccacctcgtc caacgtcaca ccgtacttgg cacatctaac taatgcaaaa 3600
taaaataagt cagcacattc ccaggctata tcttccttgg atttagcttc tgcaagttca 3660
tcagcttcct ccctaatttt agcgttcaac aaaacttcgt cgtcaaataa ccgtttggta 3720
taagaacctt ctggagcatt gctcttacga tcccacaagg tgcttccatg gctctaagac 3780
cctttgattg gccaaaacag gaagtgcgtt ccaagtgaca gaaaccaaca cctgtttgtt 3840
caaccacaaa tttcaagcag tctccatcac aatccaattc gatacccagc aacttttgag 3900
ttcgtccaga tgtagcacct ttataccaca aaccgtgacg acgagattgg tagactccag 3960
tttgtgtcct tatagcctcc ggaatagact ttttggacga gtacaccagg cccaacgagt 4020
aattagaaga gtcagccacc aaagtagtga atagaccatc ggggcggtca gtagtcaaag 4080
acgccaacaa aatttcactg acagggaact ttttgacatc ttcagaaagt tcgtattcag 4140
tagtcaattg ccgagcatca ataatgggga ttataccaga agcaacagtg gaagtcacat 4200
ctaccaactt tgcggtctca gaaaaagcat aaacagttct actaccgcca ttagtgaaac 4260
ttttcaaatc gcccagtgga gaagaaaaag gcacagcgat actagcatta gcgggcaagg 4320
atgcaacttt atcaaccagg gtcctataga taaccctagc gcctgggatc atcctttgga 4380
caactctttc tgccaaatct aggtccaaaa tcacttcatt gataccatta tacggatgac 4440
tcaacttgca cattaacttg aagctcagtc gattgagtga acttgatcag gttgtgcagc 4500

tggtcagcag catagggaaa cacggctttt cctaccaaac tcaaggaatt atcaaactct 4560
gcaacacttg cgtatgcagg tagcaaggga aatgtcatac ttgaagtcgg acagtgagtg 4620
tagtcttgag aaattctgaa gccgtatttt tattatcagt gagtcagtca tcaggagatc 4680
ctctacgccg gacgcatcgt ggccggcatc accggcgcca caggtgcggt tgctggcgcc 4740
tatatcgccg acatcaccga tggggaagat cgggctcgcc acttcgggct catgagcgct 4800
tgtttcggcg tgggtatggt ggcaggcccc gtggccgggg gactgttggg cgccatctcc 4860
ttgcatgcac cattccttgc ggcggcggtg ctcaacggcc tcaacctact actgggctgc 4920
ttcctaatgc aggagtcgca taagggagag cgtcgagtat ctatgattgg aagtatggga 4980
atggtgatac ccgcattctt cagtgtcttg aggtctccta tcagattatg cccaactaaa 5040
gcaaccggag gaggagattt catggtaaat ttctctgact tttggtcatc agtagactcg 5100
aactgtgaga ctatctcggt tatgacagca gaaatgtcct tcttggagac agtaaatgaa 5160
gtcccaccaa taaagaaatc cttgttatca ggaacaaact tcttgtttcg cgaacttttt 5220
cggtgccttg aactataaaa tgtagagtgg atatgtcggg taggaatgga gcgggcaaat 5280
gcttaccttc tggaccttca agaggtatgt agggtttgta gatactgatg ccaacttcag 5340
tgacaacgtt gctatttcgt tcaaaccatt ccgaatccag agaaatcaaa gttgtttgtc 5400
tactattgat ccaagccagt gcggtcttga aactgacaat agtgtgctcg tgttttgagg 5460
tcatctttgt atgaataaat ctagtctttg atctaaataa tcttgacgag ccaaggcgat 5520
aaatacccaa atctaaaact cttttaaaac gttaaaagga caagtatgtc tgcctgtatt 5580
aaaccccaaa tcagctcgta gtctgatcct catcaacttg aggggcacta tcttgtttta 5640
gagaaatttg cggagatgcg atatcgagaa aaaggtacgc tgattttaaa cgtgaaattt 5700
atctcaagat cgcggccgcg atctcgaata ataactgtta tttttcagtg ttcccgatct 5760
gcgtctattt cacaatacca acatgagtca gcttatcgat gataagctgt caaacatgag 5820
aattaattcg atgataagct gtcaaacatg agaaatcttg aagacgaaag ggcctcgtga 5880
tacgcctatt tttataggtt aatgtcatga taataatggt ttcttagacg tacgtcaggt 5940
ggcacttttc ggggaaatgt gcgcggaacc cctatttgtt tatttttcta aatacattca 6000
aatatgtatc cgctcatgag acaataaccc tgataaatgc ttcaataata ttgaaaaagg 6060
aagagtatga gtattcaaca tttccgtgtc gcccttattc ccttttttgc ggcattttgc 6120
cttcctgttt ttgctcaccc agaaacgctg gtgaaagtaa aagatgctga agatcagttg 6180
ggtgcacgag tgggttacat cgaactggat ctcaacagcg gtaagatcct tgagagtttt 6240
cgccccgaag aacgttttcc aatgatgagc acttttaaag ttctgctatg tggcgcggta 6300
ttatcccgtg ttgacgccgg gcaagagcaa ctcggtcgcc gcatacacta ttctcagaat 6360
gacttggttg agtactcacc agtcacagaa aagcatctta cggatggcat gacagtaaga 6420
gaattatgca gtgctgccat aaccatgagt gataacactg cggccaactt acttctgaca 6480
acgatcggag gaccgaagga gctaaccgct tttttgcaca acatggggga tcatgtaact 6540
cgccttgatc gttgggaacc ggagctgaat gaagccatac caaacgacga gcgtgacacc 6600

acgatgcctg cagcaatggc aacaacgttg cgcaaactat taactggcga actacttact 6660
ctagcttccc ggcaacaatt aatagactgg atggaggcgg ataaagttgc aggaccactt 6720
ctgcgctcgg cccttccggc tggctggttt attgctgata aatctggagc cggtgagcgt 6780
gggtctcgcg gtatcattgc agcactgggg ccagatggta agccctcccg tatcgtagtt 6840
atctacacga cggggagtca ggcaactatg gatgaacgaa atagacagat cgctgagata 6900
ggtgcctcac tgattaagca ttggtaactg tcagaccaag tttactcata tatactttag 6960
attgatttaa attgtaaacg ttaatatttt gttaaaattc gcgttaaatt tttgttaaat 7020
cagctcattt tttaaccaat aggccgaaat cggcaaaatc ccttataaat caaaagaata 7080
gaccgagata gggttgagtg ttgttccagt ttggaacaag agtccactat taaagaacgt 7140
ggactccaac gtcaaagggc gaaaaaccgt ctatcagggc gatggcccac tacgtgaacc 7200
atcaccctaa tcaagttttt tggggtcgag gtgccgtaaa gcactaaatc ggaaccctaa 7260
agggagcccc cgatttagag cttgacgggg aaagccggcg aacgtggcga gaaaggaagg 7320
gaagaaagcg aaaggagcgg gcgctagggc gctggcaagt gtagcggtca cgctgcgcgt 7380
aaccaccaca cccgccgcgc ttaatgcgcc gctacagggc gcgtaaaagg atctaggtga 7440
agatcctttt tgataatctc atgaccaaaa tcccttaacg tgagttttcg ttccactgag 7500
cgtcagaccc cgtagaaaag atcaaaggat cttcttgaga tccttttttt ctgcgcgtaa 7560
tctgctgctt gcaaacaaaa aaaccaccgc taccagcggt ggtttgtttg ccggatcaag 7620
agctaccaac tctttttccg aaggtaactg gcttcagcag agcgcagata ccaaatactg 7680
tccttctagt gtagccgtag ttaggccacc acttcaagaa ctctgtagca ccgcctacat 7740
acctcgctct gctaatcctg ttaccagtgg ctgctgccag tggcgataag tcgtgtctta 7800
ccgggttgga ctcaagacga tagttaccgg ataaggcgca gcggtcgggc tgaacggggg 7860
gttcgtgcac acagcccagc ttggagcgaa cgacctacac cgaactgaga tacctacagc 7920
gtgagcattg agaaagcgcc acgcttcccg aagggagaaa ggcggacagg tatccggtaa 7980
gcggcagggt cggaacagga gagcgcacga gggagcttcc agggggaaac gcctggtatc 8040
tttatagtcc tgtcgggttt cgccacctct gacttgagcg tcgatttttg tgatgctcgt 8100
caggggggcg gagcctatgg aaaaacgcca gcaacgcggc ctttttacgg ttcctggcct 8160
tttgctggcc ttttgctcac atgttctttc ctgcgttatc ccctgattct gtggataacc 8220
gtattaccgc ctttgagtga gctgataccg ctcgccgcag ccgaacgacc gagcgcagcg 8280
agtcagtgag cgaggaagcg gaagagcgcc tgatgcggta ttttctcctt acgcatctgt 8340
gcggtatttc acaccgcata tggtgcactc tcagtacaat ctgctctgat gccgcatagt 8400
taagccagta tacactccgc tatcgctacg tgactgggtc atggctgcgc cccgacaccc 8460
gccaacaccc gctgacgcgc cctgacgggc ttgtctgctc ccggcatccg cttacagaca 8520
agctgtgacc gtctccggga gctgcatgtg tcagaggttt tcaccgtcat caccgaaacg 8580
cgcgaggcag 8590

<210> 76

<211> 141
<212> PRT
<213> Artificial sequence

<220>

<223> Synthetic sequence pD2pick (MFalphaPrePro::EPI-HNE-3)

<400> 76

```
Met Arg Phe Pro Ser Ile Phe Thr Ala Val Leu Phe Ala Ala Ser Ser
1               5                   10                  15

Ala Leu Ala Ala Pro Val Asn Thr Thr Thr Glu Asp Glu Thr Ala Gln
            20                  25                  30

Ile Pro Ala Glu Ala Val Ile Gly Tyr Ser Asp Leu Glu Gly Asp Phe
        35                  40                  45

Asp Val Ala Val Leu Pro Phe Ser Asn Ser Thr Asn Asn Gly Leu Leu
    50                  55                  60

Phe Ile Asn Thr Thr Ile Ala Ser Ile Ala Ala Lys Glu Glu Gly Val
65                  70                  75                  80

Ser Leu Asp Lys Arg Ala Ala Cys Asn Leu Pro Ile Val Arg Gly Pro
                85                  90                  95

Cys Ile Ala Phe Phe Pro Arg Trp Ala Phe Asp Ala Val Lys Gly Lys
            100                 105                 110

Cys Val Leu Phe Pro Tyr Gly Gly Cys Gln Gly Asn Gly Asn Lys Phe
        115                 120                 125

Tyr Ser Glu Lys Glu Cys Arg Glu Tyr Cys Gly Val Pro
    130                 135                 140
```

<210> 77
<211> 147
<212> PRT
<213> Homo sapiens

<400> 77

```
Ala Val Leu Pro Gln Glu Glu Glu Gly Ser Gly Gly Gly Gln Leu Val
1               5                   10                  15

Thr Glu Val Thr Lys Lys Glu Asp Ser Cys Gln Leu Gly Tyr Ser Ala
            20                  25                  30

Gly Pro Cys Met Gly Met Thr Ser Arg Tyr Phe Tyr Asn Gly Thr Ser
        35                  40                  45

Met Ala Cys Glu Thr Phe Gln Tyr Gly Gly Cys Met Gly Asn Gly Asn
    50                  55                  60

Asn Phe Val Thr Glu Lys Glu Cys Leu Gln Thr Cys Arg Thr Val Ala
65                  70                  75                  80

Ala Cys Asn Leu Pro Ile Val Arg Gly Pro Cys Arg Ala Phe Ile Gln
                85                  90                  95

Leu Trp Ala Phe Asp Ala Val Lys Gly Lys Cys Val Leu Phe Pro Tyr
            100                 105                 110

Gly Gly Cys Gln Gly Asn Gly Asn Lys Phe Tyr Ser Glu Lys Glu Cys
        115                 120                 125
```

Arg Glu Tyr Cys Gly Val Pro Gly Asp Gly Asp Glu Glu Leu Leu Arg
    130                 135                 140

Phe Ser Asn
145

<210> 78
<211> 249
<212> DNA
<213> Artificial sequence

<220>

<223> Synthetic sequence M13IIIsig::HuLACI-D2::matIII

<400> 78
atgaagaagc ttctcttcgc cattcctctg gtggtacctt tctattccgg cgccaagcct 60

gacttctgct tcctcgagga ggatcccggg atttgccgcg gttatattac gcgttatttc 120

tataataacc agactaagca atgtgagcgg ttcaagtatg gtggttgcct aggtaatatg 180

aacaacttcg agactctaga agagtgtaag aacatatgtg aggatggtgg tgctgagact 240

gttgagtct 249

<210> 79
<211> 83
<212> PRT
<213> Artificial sequence

<220>

<223> Synthetic sequence M13IIIsig::HuLACI-D2::matIII

<400> 79

Met Lys Lys Leu Leu Phe Ala Ile Pro Leu Val Val Pro Phe Tyr Ser
1               5                   10                  15

Gly Ala Lys Pro Asp Phe Cys Phe Leu Glu Glu Asp Pro Gly Ile Cys
            20                  25                  30

Arg Gly Tyr Ile Thr Arg Tyr Phe Tyr Asn Asn Gln Thr Lys Gln Cys
        35                  40                  45

Glu Arg Phe Lys Tyr Gly Gly Cys Leu Gly Asn Met Asn Asn Phe Glu
    50                  55                  60

Thr Leu Glu Glu Cys Lys Asn Ile Cys Glu Asp Gly Gly Ala Glu Thr
65                  70                  75                  80

Val Glu Ser

<210> 80
<211> 189
<212> DNA
<213> Artificial sequence

<220>

<223> Synthetic sequence LACI-D1

<400> 80

gcggccgaga tgcattcctt ctgcgctttc aaagctgatg acggtccgtg taaagctatc 60

atgaaacgtt tcttcttcaa cattttcacg cgtcagtgcg aggaattcat ttacggtggt 120

tgtgaaggta accagaaccg gttcgaatct ctagaggaat gtaagaagat gtgcactcgt 180

gacggcgcc 189

<210> 81
<211> 63
<212> PRT
<213> Artificial sequence

<220>

<223> Synthetic sequence LACI-D1 protein

<400> 81

```
Ala Ala Glu Met His Ser Phe Cys Ala Phe Lys Ala Asp Asp Gly Pro
1               5                   10                  15

Cys Lys Ala Ile Met Lys Arg Phe Phe Phe Asn Ile Phe Thr Arg Gln
            20                  25                  30

Cys Glu Glu Phe Ile Tyr Gly Gly Cys Glu Gly Asn Gln Asn Arg Phe
        35                  40                  45

Glu Ser Leu Glu Glu Cys Lys Lys Met Cys Thr Arg Asp Gly Ala
    50                  55                  60
```

<210> 82
<211> 189
<212> DNA
<213> Artificial sequence

<220>

<223> Synthetic sequence LACI-D1 hNE library

<220>
<221> misc_feature
<222> (37)..(37)
<223> n is a, c, g, or t

<220>
<221> misc_feature
<222> (40)..(40)
<223> r is a purine

<220>
<221> misc_feature
<222> (41)..(41)
<223> v is a, g, or c

<220>
<221> misc_feature
<222> (42)..(42)
<223> s is g or c

<220>
<221> misc_feature
<222> (47)..(47)
<223> n is a, c, g, or t

<220>
<221> misc_feature

<222> (52)..(52)
<223> r is a purine

<220>
<221> misc_feature
<222> (56)..(56)
<223> s is g or c

<220>
<221> misc_feature
<222> (58)..(58)
<223> n is a, c, g, or t

<220>
<221> misc_feature
<222> (64)..(64)
<223> m is a or c

<220>
<221> misc_feature
<222> (65)..(65)
<223> n is a, c, g, or t

<220>
<221> misc_feature
<222> (66)..(66)
<223> s is g or c

<220>
<221> misc_feature
<222> (71)..(71)
<223> d is a, g, or t

<220>
<221> misc_feature
<222> (72)..(72)
<223> s is g or c

<220>
<221> misc_feature
<222> (100)..(100)
<223> s is g or c

<220>
<221> misc_feature
<222> (101)..(101)
<223> w is a or t

<220>
<221> misc_feature
<222> (103)..(103)
<223> v is a, g, or c

<220>
<221> misc_feature
<222> (104)..(104)
<223> h is a, c, or t

<220>
<221> misc_feature
<222> (109)..(109)
<223> v is a, g, or c
<220>
<221> misc_feature
<222> (110)..(110)
<223> h is a, c, or t

<220>
<221> misc_feature

<222> (124)..(124)
<223> v is a, g, or c

<220>
<221> misc_feature
<222> (125)..(125)
<223> h is a, c, or t

<220>
<221> misc_feature
<222> (128)..(128)
<223> s is g or c

<220>
<221> misc_feature
<222> (133)..(133)
<223> s is g or c

<220>
<221> misc_feature
<222> (134)..(134)
<223> r is a purine

<400> 82
gcggccgaga tgcattcctt ctgcgctttc aaagctnrtr vsggtcnttg trttgstntc 60

ttcmnscgtt dsttcttcaa cattttcacg cgtcagtgcs wgvhattcvh atacggtggt 120

tgtvhggsta acsrgaaccg gttcgaatct ctagaggaat gtaagaagat gtgcactcgt 180

gacggcgcc 189

<210> 83
<211> 63
<212> PRT
<213> Artificial sequence

<220>

<223> Synthetic sequence LACI-D1 hNE library

<220>
<221> MISC_FEATURE
<222> (13)..(13)
<223> Xaa is Cys, Arg, Ser, Gly, Tyr, His, Asp, or Asn

<220>
<221> MISC_FEATURE
<222> (14)..(14)
<223> Xaa is Thr, Asn, Lys, Arg, Ser, Ala, Glu, Gly, or Asp

<220>
<221> MISC_FEATURE
<222> (16)..(16)
<223> Xaa is His, Arg, Pro, or Leu

<220>
<221> MISC_FEATURE
<222> (18)..(18)
<223> Xaa is Val or Ile

<220>
<221> MISC_FEATURE
<222> (19)..(19)
<223> Xaa is Ala or Gly

<220>
<221> MISC_FEATURE

<222> (20)..(20)
<223> Xaa is Phe, Leu, Ile, or Val

<220>
<221> MISC_FEATURE
<222> (22)..(22)
<223> Xaa is Ser, Thr, Asn, Ile, Met, Gln, His, Leu, Pro, Lys, or Arg

<220>
<221> MISC_FEATURE
<222> (24)..(24)
<223> Xaa is Cys, Tyr, Trp, Phe, or Leu

<220>
<221> MISC_FEATURE
<222> (34)..(34)
<223> Xaa is Leu, Gln, Glu, or Val

<220>
<221> MISC_FEATURE
<222> (35)..(35)
<223> Xaa is Gln, Leu, Pro, Thr, Lys, Val, Ile, Glu, or Ala

<220>
<221> MISC_FEATURE
<222> (37)..(37)
<223> Xaa is Gln, Leu, Pro, Thr, Lys, Val, Glu, Ile, or Ala

<220>
<221> MISC_FEATURE
<222> (42)..(42)
<223> Xaa is Gln, Leu, Pro, Thr, Lys, Val, Met, Glu, or Ala

<220>
<221> MISC_FEATURE
<222> (43)..(43)
<223> Xaa is Gly or Ala

<220>
<221> MISC_FEATURE
<222> (45)..(45)
<223> Xaa is Glu, Gly, Gln, or Arg

<400> 83

```
Ala Ala Glu Met His Ser Phe Cys Ala Phe Lys Ala Xaa Xaa Gly Xaa
1               5                   10                  15

Cys Xaa Xaa Xaa Phe Xaa Arg Xaa Phe Phe Asn Ile Phe Thr Arg Gln
            20                  25                  30

Cys Xaa Xaa Phe Xaa Tyr Gly Gly Cys Xaa Xaa Asn Xaa Asn Arg Phe
        35                  40                  45

Glu Ser Leu Glu Glu Cys Lys Lys Met Cys Thr Arg Asp Gly Ala
    50                  55                  60
```

<210> 84
<211> 201
<212> DNA
<213> Artificial sequence

<220>

<223> Synthetic sequence LACI-D2 hNE library

<220>

<221> misc_feature
<222> (34)..(34)
<223> n is a, c, g, or t

<220>
<221> misc_feature
<222> (35)..(35)
<223> r is a purine

<220>
<221> misc_feature
<222> (37)..(38)
<223> v is a, g, or c

<220>
<221> misc_feature
<222> (39)..(39)
<223> s is g or c

<220>
<221> misc_feature
<222> (43)..(43)
<223> m is a or c

<220>
<221> misc_feature
<222> (44)..(44)
<223> n is a, c, g, or t

<220>
<221> misc_feature
<222> (49)..(49)
<223> r is a purine

<220>
<221> misc_feature
<222> (53)..(53)
<223> s is g or c
<220>
<221> misc_feature
<222> (55)..(55)
<223> n is a, c, g, or t

<220>
<221> misc_feature
<222> (56)..(56)
<223> w is a or t

<220>
<221> misc_feature
<222> (61)..(61)
<223> m is a or c

<220>
<221> misc_feature
<222> (62)..(62)
<223> n is a, c, g, or t

<220>
<221> misc_feature
<222> (63)..(63)
<223> s is g or c

<220>
<221> misc_feature
<222> (68)..(68)
<223> d is a, g, or t

<220>

<221> misc_feature
<222> (69)..(69)
<223> s is g or c

<220>
<221> misc_feature
<222> (97)..(97)
<223> s is g or c

<220>
<221> misc_feature
<222> (98)..(98)
<223> w is a or t

<220>
<221> misc_feature
<222> (100)..(100)
<223> v is a, g, or c

<220>
<221> misc_feature
<222> (101)..(101)
<223> n is a, c, g, or t

<220>
<221> misc_feature
<222> (106)..(106)
<223> v is a, g, or c

<220>
<221> misc_feature
<222> (107)..(107)
<223> h is a, c, or t

<220>
<221> misc_feature
<222> (121)..(121)
<223> v is a, g, or c

<220>
<221> misc_feature
<222> (122)..(122)
<223> h is a, c, or t

<220>
<221> misc_feature
<222> (125)..(125)
<223> s is g or c

<220>
<221> misc_feature
<222> (130)..(130)
<223> v is a, g, or c

<220>
<221> misc_feature
<222> (131)..(131)
<223> b is g, c, or t

<400> 84
ggcgccaagc ctgacttctg cttcctcgag gagnrtvvsg ggmnttgcrt tgstnwtttt 60

mnscgttdst tctataataa ccaggctaag caatgtswgv nattcvhata tggtggttgc 120

vhggstaatv bgaacaactt cgagactcta gaagagtgta agaacatatg tgaggatggt 180

ggtgctgaga ctgttgagtc t 201

<210> 85
<211> 67
<212> PRT
<213> Artificial sequence

<220>

<223> Synthetic sequence LACI-D2 hNE library

<220>
<221> MISC_FEATURE
<222> (12)..(12)
<223> Xaa is Cys, Arg, Ser, Gly, Tyr, His, Asp, or Asn

<220>
<221> MISC_FEATURE
<222> (13)..(13)
<223> Xaa is Pro, His, Thr, Asn, Lys, Arg, Ser, Ala, Glu, Gly, Asp, or Gln

<220>
<221> MISC_FEATURE
<222> (15)..(15)
<223> Xaa is His, Arg, Pro, Leu, Asn, Ser, Ile, or Thr

<220>
<221> MISC_FEATURE
<222> (17)..(17)
<223> Xaa is Val or Ile

<220>
<221> MISC_FEATURE
<222> (18)..(18)
<223> Xaa is Gly or Ala

<220>
<221> MISC_FEATURE
<222> (19)..(19)
<223> Xaa is Phe, Leu, Ile, Val, Tyr, His, Asn, or Asp

<220>
<221> MISC_FEATURE
<222> (21)..(21)
<223> Xaa is Ile, Asn, Gln, Met, Leu, His, Lys, Pro, Thr, or Arg

<220>
<221> MISC_FEATURE
<222> (23)..(23)
<223> Xaa is Cys, Phe, Leu, Tyr, or Trp

<220>
<221> MISC_FEATURE
<222> (33)..(33)
<223> Xaa is Leu, Gln, Glu, or Val

<220>
<221> MISC_FEATURE
<222> (34)..(34)
<223> Xaa is Gln, Gly, Leu, Pro, Thr, Lys, Val, Ile, Glu, or Ala

<220>
<221> MISC_FEATURE
<222> (36)..(36)
<223> Xaa is Gln, Leu, Pro, Thr, Val, Glu, Ile, or Ala

<220>
<221> MISC_FEATURE
<222> (41)..(41)
<223> Xaa is Gln, Pro, Thr, Lys, Val, Met, Glu, or Ala

```
<220>
<221>  MISC_FEATURE
<222>  (42)..(42)
<223>  Xaa is Gly or Ala

<220>
<221>  MISC_FEATURE
<222>  (44)..(44)
<223>  Xaa is Arg, Gly, Lys, Glu, Leu, Gln, Met, or Val

<400>  85

Gly Ala Lys Pro Asp Phe Cys Phe Leu Glu Glu Xaa Xaa Gly Xaa Cys
1               5                   10                  15

Xaa Xaa Xaa Phe Xaa Arg Xaa Phe Tyr Asn Asn Gln Ala Lys Gln Cys
            20                  25                  30

Xaa Xaa Phe Xaa Tyr Gly Gly Cys Xaa Xaa Asn Xaa Asn Asn Phe Glu
        35                  40                  45

Thr Leu Glu Glu Cys Lys Asn Ile Cys Glu Asp Gly Gly Ala Glu Thr
    50                  55                  60

Val Glu Ser
65
```

## Description

Cross-Reference to Related Applications

This application is a continuation-in-part of application 08/358,160 filed 16 December 1994 which is a continuation-in-part of application 08/133,031 filed 28 February 1992, which is related to the continuation-in-part application filed March 1, 1991 of Ladner, Guterman, Roberts, Markland, Ley, and Kent, Serial No. 07/664,989, now abandoned, which is a continuation-in-part of Ladner, Guterman, Roberts, and Markland, Ser. No. 07/487,063, filed March 2, 1990, now abandoned, which is a continuation-in-part of Ladner and Guterman, Ser. No. 07/240,160, filed Sept. 2, 1988, now abandoned. All of the foregoing applications are hereby incorporated by reference.

The following related and commonly-owned applications are also incorporated by reference:

Robert Charles Ladner, Sonia Kosow Guterman, Rachel Baribault Kent, and Arthur Charles Ley are named as joint inventors on U.S.S.N. 07/293,980, filed January 8, 1989, and entitled GENERATION AND SELECTION OF NOVEL DNA-BINDING PROTEINS AND POLYPEPTIDES. This application has been assigned to Protein Engineering Corporation.

Robert Charles Ladner, Sonia Kosow Guterman, and Bruce Lindsay Roberts are named as a joint inventors on a U.S.S.N. 07/470,651 filed 26 January 1990 (now abandoned), entitled "PRODUCTION OF NOVEL SEQUENCE-SPECIFIC DNA-ALTERING ENZYMES", likewise assigned to Protein Engineering Corp.

Ladner, Guterman, Kent, Ley, and Markland, Ser. No. 07/558,011 is also assigned to Protein Engineering Corporation.

Ladner filed an application on May 17, 1991, Ser. No. 07/715,834 that is hereby incorporated by reference.

## BACKGROUND OF THE INVENTION

Field of the Invention

This invention relates to novel proteins that inhibit human neutrophil elastase (hNE). A large fraction of the sequence of each of these proteins is identical to a known human protein which has very little or no inhibitory activity with respect to hNE.

### Information Disclosure Statement

1. hNE , its natural inhibitors, and pathologies

Human Neutrophil Elastase (hNE, also known as Human Leukocyte Elastase (hLE); EC 3.4.21.11) is a 29 Kd protease with a wide spectrum of activity against extracellular matrix components (CAMP82, CAMP88, MCWH89). The enzyme is one of the major neutral proteases of the azurophil granules of polymorphonuclear leucocytes and is involved in the elimination of pathogens and in connective tissue restructuring (TRAV88). In cases of hereditary reduction of the circulating α-1-protease inhibitor (API, formerly known as α1 antitrypsin), the principal systemic physiological inhibitor of hNE (HEID86), or the inactivation of API by oxidation ("smoker's emphysema"), extensive destruction of lung tissue may result from uncontrolled elastolytic activity of hNE (CANT89). Several human respiratory disorders, including cystic fibrosis and emphysema, are characterized by an increased neutrophil burden on the epithelial surface of the lungs (SNID91, MCEL91, GOLD86) and hNE release by neutrophils is implicated in the progress of these disorders (MCEL91, WEIS89). A preliminary study of aerosol administration of API to cystic fibrosis patients indicates that such treatment can be effective both in prevention of respiratory tissue damage and in augmentation of host antimicrobial defenses (MCEL91).

API presents some practical problems to large-scale routine use as a pulmonary anti-elastolytic agent. These include the relatively large size of the molecule (394 residues, 51 k Dalton), the lack of intramolecular stabilizing disulfide bridges, and specific post translational modifications of the protein by glycosylation at three sites. Perhaps of even greater importance is the sensitivity of API to oxidation, such as those released by activated neutrophils. Hence a small stable nontoxic highly efficacious inhibitor of hNE would be of great therapeutic value.

2. ITI domain 1 and ITI domain 2 as an initial protein binding domains (IPBD)

Many mammalian species have a protein in their plasma that can be identified, by sequence homology and similarity of physical and chemical properties, as inter-α-trypsin inhibitor (ITI), a large ($M_r$ *ca* 240,000) circulating protease inhibitor (for recent reviews see ODOM90, SALI90, GEBH90, GEBH86). The sequence of human ITI is shown in Table 400. The intact inhibitor is a glycoprotein and is currently believed to consist of three glycosylated subunits that interact through a strong glycosaminoglycan linkage (ODOM90, SALI90, ENGH89, SELL87). The anti-trypsin activity of ITI is located on the smallest subunit (ITI light chain, unglycosylated $M_r$ *ca* 15,000) which is identical in amino acid sequence to an acid stable inhibitor found in urine (UTI) and serum (STI) (GEBH86, GEBH90). The amino-acid sequence of the ITI light chain is shown in Table 400. The mature light chain consists of a 21 residue N-terminal sequence, glycosylated at $Ser_{10}$, followed by two tandem Kunitz-type domains the first of which is glycosylated at $Asn_{45}$ (ODOM90). In the human protein, the second Kunitz-type domain has been shown to inhibit trypsin, chymotrypsin, and plasmin (ALBR83a, ALBR83b, SELL87, SWAI88). The first domain lacks these activities but has been reported to inhibit leukocyte elastase ($\approx$ 1 $\mu$M>$K_i$> $\approx$ 1 nM) (ALBR83a,b, ODOM90). cDNA encoding the ITI light chain also codes for $\alpha$-1-microglobulin (TRAB86, KAUM86, DIAR90); the proteins are separated post-translationally by proteolysis.

The two Kunitz domains of the ITI light chain (ITI-D1 and ITI-D2) possesses a number of characteristics that make them useful as Initial Potential Binding Domains (IPBDs). ITI-D1 comprises at least residues 26 to 76 of the UTI sequence shown in Fig. 1 of GEBH86. The Kunitz domain could be thought of as comprising residues from as early as residue 22 to as far as residue 79. Residues 22 through 79 constitute a 58-amino-acid domain having the same length as bovine pancreatic trypsin inhibitor (BPTI) and having the cysteines aligned. ITI-D2 comprises at least residues 82 through 132; residues as early as 78 and as later as 135 could be included to give domains closer to the classical 58-amino-acid length. As the space between the last cysteine of ITI-D1 (residue 76 of ITI light chain) and the first cysteine of ITI-D2 (residue 82 of ITI light chain) is only 5 residues, one can not assign 58 amino acids to each domain without some overlap. Unless otherwise stated, herein, we have taken the second domain to begin at residue 78 of the ITI light chain. Each of the domains are highly homologous to both BPTI and the EpiNE series of proteins described in US patent 5,223,409. Although x-ray structures of the isolated domains ITI-D1 and ITI-D2 are not available, crystallographic studies of the related Kunitz-type domain isolated from the Alzheimer's amyloid $\beta$-protein (AA$\beta$P) precursor show that this polypeptide assumes a 3D structure almost identical to that of BPTI (HYNE90).

The three-dimensional structure of $\alpha$-dendrotoxin from green mamba venom has been determined (SKAR92) and the structure is highly similar to that of BPTI. The author states, "Although the main-chain fold of $\alpha$-DTX is similar to that of homologous bovine pancreatic trypsin inhibitor (BPTI), there are significant differences involving segments of the polypeptide chain close to the 'antiprotease site' of BPTI. Comparison of the structure of $\alpha$-DTX with the existing models of BPTI and its complexes with trypsin and kallikrein reveals structural differences that explain the inability of $\alpha$-DTX to inhibit trypsin and chymotrypsin."

The structure of the black mamba K venom has been determined by NMR spectroscopy and has a 3D structure that is highly similar to that of BPTI despite 32 amino-acid sequence differences between residues 5 and 55 (the first and last cysteines)(BERN93). "The solution structure of Toxin K is very similar to the solution structure of the basic pancreatic trypsin inhibitor (BPTI) and the X-ray crystal structure of the $\alpha$-dendrotoxin from Dendroaspis angusticeps ($\alpha$-DTX), with r.m.s.d. values of 1.31 Å and 0.92 Å, respectively, for the backbone atoms of residues 2 to 56. Some local structural differences between Toxin K and BPTI are directly related to the fact that intermolecular interactions with two of the four internal molecules of hydration water in BPTI are replaced by intramolecular hydrogen bonds in Toxin K." Thus, it is likely that the solution 3D structure of either of the isolated ITI-D1 domain or of the isolated ITI-D2 domain will be highly similar to the structures of BPTI, AA$\beta$P, and black mamba K venom. In this case, the advantages described previously for use of BPTI as an IPBD apply to ITI-D1 and to ITI-D2. ITI-D1 and ITI-D2 provide additional advantages as an IPBD for the development of specific anti-elastase inhibitory activity. First, the ITI-D1 domain has been reported to inhibit both leukocyte elastase (ALBR83a,b, ODOM90) and Cathepsin-G (SWAI88, ODOM90); activities which BPTI lacks. Second, ITI-D1 lacks affinity for the related serine proteases trypsin, chymotrypsin, and plasmin (ALBR83a, b, SWAI88), an advantage for the development of specificity in inhibition. ITI-D2 has the advantage of not being glycosylated. Additionally, ITI-D1 and ITI-D2 are human-derived polypeptides so that derivatives are anticipated to show minimal antigenicity in clinical applications.

3. Secretion of heterologous proteins from *Pichia pastoris*

Others have produced a number of proteins in the yeast *Pichia pastoris*. For example, Vedvick *et al*. (VEDV91) and Wagner *et al.* (WAGN92) produced aprotinin from the alcohol oxidase promoter with induction by methanol as a secreted protein in the culture medium (CM) at $\approx$ 1 mg/mL. Gregg *et al.* (GREG93) have reviewed production of a number of proteins in *P. pastoris*. Table 1 of GREG93 shows proteins that have been produced in *P. pastoris* and the yields.

4. Recombinant production of Kunitz Domains:

Aprotinin has been made via recombinant-DNA technology (AUER87, AUER88, AUER89, AUER90, BRIN90, BRIN91, ALTM91).

5. Construction methods:

Unless otherwise stated, genetic constructions and other manipulations are carries out by standard methods, such as found in standard references (*e.g.* AUSU87 and SAMB89).

No admission is made that any cited reference is prior art or pertinent prior art, and the dates given are those appearing on the reference and may not be identical to the actual publication date. The descriptions of the teachings of any cited reference are based on our present reading thereof, and we reserve the right to, revise the description if an error comes to our attention, and to challenge whether the description accurately reflects the actual work reported. We reserve the right to challenge the interpretation of cited works, particularly in light of new or contradictory evidence.

## SUMMARY OF THE INVENTION

The present invention describes a series of small potent proteinaceous inhibitors of human neutrophil elastase (hNE). One group of inhibitors is derived from a Kunitz-type inhibitory domain found in a protein of human origin, namely, the light chain of human Inter-α-trypsin inhibitor (ITI) which contains domains designated ITI-D1 and ITI-D2. The present invention discloses variants of ITI-D2 that have very high affmty for hNE. The present invention comprises modifications to the ITI-D2 sequence that facilitate its production in the yeast *Pichia pastoris* and that are highly potent inhibitors of hNE. The invention also relates to methods of transferring segments of sequence from one Kunitz domain to another and to methods of production.

The invention is presented as a series of examples that describe design, production, and testing of actual inhibitors and additional examples describing how other inhibitors could be discovered. The invention relates to proteins that inhibit human neutrophil elastase (hNE) with high affinity.

NOMENCLATURE and ABBREVIATIONS

| Term | Meaning | | |
|---|---|---|---|
| *x*::*y* | Fusion of gene *x* to gene *y* in frame. | | |
| X::Y | Fusion protein expressed from x::y fusion gene. | | |
| μM | Micromolar, $10^{-6}$ molar. : | | |
| nM | Namomolar, $10^{-9}$ molar. | | |
| pM | Picomolar, $10^{-12}$ molar. | | |
| Single-letter amino-acid codes: | | | |
| A: Ala | C: Cys | D: Asp | E: Glu |
| F: Phe | G: Gly | H: His | I: Ile |
| K: Lys | L: Leu | M: Met | N: Asn |
| P: Pro | Q: Gln | R: Arg | S: Ser |
| T: Thr | V: Val | W: Tip | Y: Tyr |

## DETAILED DESCRIPTION OF THE PREFERRED EMBODIMENTS OF THE INVENTION

A protein sequence can be called an "aprotinin-like Kunitz domain" if it contains a sequence that when aligned to minimize mismatches, can be aligned, with four or fewer mismatches, to the pattern:

Cys-$(Xaa)_6$-Gly-Xaa-Cys-$(Xaa)_8$-[Tyr|Phe]-$(Xaa)_6$-Cys-$(Xaa)_2$-Phe-Xaa-[Tyr|Trp|Phe]-Xaa-Gly-Cys-$(Xaa)_4$-[Asn|Gly]-Xaa-[Phe|Tyr]-$(Xaa)_5$-Cys-$(Xaa)_3$-Cys, where bracketed amino acids separated by a | symbol are alternative amino acids for a single position.
For example, [Tyr|Phe] indicates that at that position, the amino acid may be either Tyr or Phe. The symbol Xaa denotes that at that position, any amino acid may be used. For the above test, an insertion or deletion counts as one mismatch.

In aprotonin, the cysteines are numbered 5, 14, 30, 38, 51, and 55 and are joined by disulfides 5-to-55, 14-to-38, and 30-to-51. Residue 15 is called the P1 residue (SCHE67); residues toward the amino terminus are called P2 (residue 14), P3(residue 13), *etc.* Residue 16 is called P1', 17 is P2', 18 is P3', *etc.*
There are many homologues of aprotonin, which differ from it at one or more positions but retain the fundamamental structure defined above. For a given list of homologues, it is possible to tabulate the frequency of occurrence of each amino acid at each ambiguous position. (The sequence having the most prevalent amino acid at each ambiguous position is listed as "Consensus Kunitz Domain" in Table 100).

A "human aprotonin-like Kunitz domain" is an aprotonin-like Kunitz domain which is found in nature in a human protein. Human aprotonin-like Kunitz domains include, but are not limited to, ITI-D1, ITI-D2, App-I, TFPI2-D1, TFPI2-D2, TFPI2-D3, LACI-D1, LACI-D2, LACI-D3, A3 collagen, and the HKI B9 domain. In this list, D1, D2, etc., denote the first, second, etc. domain of the indicated multidomain protein.

"Weak", "Moderate", "Strong" and "Very Strong" binding to and inhibition of hNE are defined in accordance with Table 55. Preferably, the proteins of the present invention have a Ki of less than 1000 pM (i.e., are "strong" inhibitors), more preferably less than 50 pM, most preferably less than 10 pM (i.e., are "very strong" inhibitors).

For purposes of the present invention, an aprotonin-like Kunitz domain may be divided into ten segments, based on the consensus sequence and the location of the catalytic site. Using the amino acid numbering scheme of aprotonin, these segments are as follows (see Table 100):

1: 1-4 (residues before first Cys)
2: 5-9 (first Cys and subsequent residues before P6)
3: 10-13 (P6 to P3)
4: 14 (second Cys; P2)
5: 15-21 (P1, and P1' to P6')
6: 22-30 (after P6 and up to and incl. third Cys.)
7: 31-36 (after third Cys and up to consensus Gly-Cys)
8: 37-38 (consensus Gly-Cys)
9: 39-42 (residues after Gly-Cys and before consensus [Asn|Gly]
10: 43-55 (up to last Cys)(also includes residues after last Cys, if any)

It will be appreciated that in those aprotonin-like Kunitz domains that differ from aprotonin by one or more amino acid insertions or deletions, or which have a different number of amino acids before the first cysteine or after the last cysteine, the actual amino acid position may differ from that given above. It is applicant's intent that these domains be numbered so as to correspond to the aligned aprotonin sequence, e.g., the first cysteine of the domain is numbered amino acid 5, for the purpose of segment identification. Note that segment 1, while a part of aprotonin, is not a part of the formal definition of an aprotonin-like Kunitz domain, and therefore it is not required that the proteins of the present invention include a sequence corresponding to segment 1. Similarly, part of segment 10 (after the last Cys) is not a required part of the domain.

A "humanized inhibitor" is one in which at least one of segments 3, 5, 7 and 9 differs by at least one nonconservative modification from the most similar (based on amino acid identities) human aprotonin-like Kunitz domain, at least one of segments 2, 6, and 10 (considered up to the last Cys) is identical, or differs only by conservative modifications, from said most similar human aprotonin-like Kunitz domain, and which is not identical to any naturally occurring nonhuman aprotonin-like Kunitz domain. (Note that segment 1 is ignored in making this determination since it is outside the sequence used to define a domain, and segments 4 and 8 are ignored because they are required by the definition of an aprotonin-like Kunitz domain.)

The proteins of the present invention are preferably humanized strong or very strong hNE inhibitors. It should be noted that the human aprbtonin-lilte Kunitz domains thus far identified are merely weak hNE inhibitors.

For the purpose of the appended claims, an aprotonin-like Kunitz domain is "substantially homologous" to a reference domain if, over the critical region (aprotonin residues 5-55) set forth above, it is at least at least 50 % identical in amino acid sequence to the corresponding sequence of or within the reference domain, and all divergences take the form of conservative and/or semi-conservative modifications.

Proteins of the present invention include those comprising a Kunitz domain that is substantially homologous to the reference proteins EPI-HNE-3, EPI-HNE-4, DPI.1.1, DPI.1.2, DPI.1.3, DPI.2.1, DPI.2.2, DPI.2.3, DPI.3.1, DPI.3.2, DPI.3.3, DPI.4.1, DPI.4.2, DPI.4.3, DPI.5.1, DPI.5.2, DPI.5.3, DPI.6.1, DPI.6.2, DPI.6.3, DPI.6.4, DPI.6.5, DPI.6.6, DPI.6.7, DPI.7.1, DPI.7.2, DPI.7.3, DPI.7.4, DPI.7.5, DPI.8.1, DPI.8.2, DPI.8.3, DPI.9.1, DPI.9.2, or DPI.9.3, as defined in Table 100. Homologues of EPI-HNE-3 and EPI-HNE-4 are especially preferred.

Preferably, the hNE-binding domains of the proteins of the present invention are at least 80 % identical, more preferably, at least 90% identical, in amino acid sequence to the corresponding reference sequence. Most preferably, the number of mismatches is zero, one, two, three, four or five. Desirably, the hNE-binding domains diverge from the reference domain solely by one or more conservative modifications. "Conservative modifications" are defined as:

a) conservative substitutions of amino acids as hereafter defined, and
b) single or multiple insertions or deletions of amino acids at the termini, at interdomain boundaries, in loops or in other segments of relatively high mobility (as indicated, for example, by high temperature factors or lack of resolution in X-ray diffraction, neutron diffraction, or NMR). Preferably, except at the termini, no more than about five amino acids are inserted or deleted at a particular locus, and the modifications are outside regions known to contain binding sites important to activity.

"Conservative substitutions" are herein defined as exchanges within on of the following five groups:

I. Small aliphatic, nonpolar or slightly polar residues: [Ala, Ser, Thr, (Pro, Gly)],
II. Acidic amino acids and their amides: [Asp, Glu, Asn, Gln],
III. Polar, positively charged residues: [His, Lys, Arg],
IV. Aliphatic nonpolar residues: [Met, Leu, Ile, Val, (Cys)], and
V. Large, aromatic residues: [Phe, Tyr, Trp]

Residues Pro, Gly, and Cys are parenthesized because they have special conformational roles. Cys often participates in disulfide bonds; when not so doing, it is highly hydrophobic. Gly imparts flexibility to the chain; it is often described as a "helix breaker" although many $\alpha$ helices contain Gly. Pro imparts rigidity to the chain and is also described as a "helix breaker". Although Pro is most often found in turns, Pro is also found in helices and sheets. These residues may be essential at certain positions and substitutable elsewhere.

Semi-Conservative Modifications" are defined herein as transpositions of adjacent amino acids (or their conservative replacements), and semi-conservative substitutions. "Semi-conservative substitutions" are defined to be exchanges between two of groups (I)-(V) above which are limited either to the supergroup consisting of (1), (II), and (III) or to the supergroup consisting of (IV) and (V). For the purpose of this definition, however, glycine and alanine are considered to be members of both supergroups. '

"Non-conservative modifications" are modifications which are neither conservative nor semi-conservative.

Preferred proteins of the present invention are further characterized by one of more of the preferred, highly preferred, or most preferred mutations set forth in Table 711.

Preferably, the proteins of the present invention have hNE-inhibitory domains which are not only substantially homologous to a reference domain, but also qualify as humanized inhibitors.

Claim 1 of PCT/US92/01501 refers to proteins denoted EpiNEalpha, EpiNE1, EpiNE2, EpiNE3, EpiNE4, EpiNE5, EpiNE6, EpiNE7, and EpiNE8. Claim 3 refers to proteins denoted ITI-E7, BITI-E7, BITI-E&-1222, AMINO1, AMINO2, MUTP1, BITI-E7-141, MUTT26A, MUTQE, and MUT1619. (With the exception of EpiNEalpha, the sequences of all of these domains appears in Table 100.) Claims 4-6 related to inhibitors which are homologous to, but not identical with, the aforementioned inhibitors. These homologous inhibitors could differ from the lead inhibitors by one or more class A substitutions (claim 4), one or more class A or B substitutions (claim 5), or one or more class A, B or C substitutions (claim 6). Class A, B and C substitutions were defined in Table 65 of PCT/US92/01501. For convenience, Table 65 has been duplicated in this specification.

The meaning of classes A, B and C were as follows: A, no major effect expected if molecular charge stays in range -1 to +1; B, major effects not expected, but more likely than with A; and C, residue in binding interface, any change must be tested. Each residue position was assigned an A, B, C or X rating; X meant no substitution allowed. At the non-X positions, allowed substitutions were noted.

The present invention exludes domains corresponding exactly to the lead domains of claims 1 and 3 of PCT/US92/01501. Preferably, the domains of the present invention also differ from these lead domains by one or more mutations which are not class A substitutions, more preferably, not class A or B substitutions, and still more preferably, not class A, B or C substitutions, as defined in Table 65. Desirably, the domains of the present invention are each more similar to one of the aforementioned reference proteins than to any of the lead proteins set forth in PCT/US92/01501.

The examples contain numerous examples of amino-acid sequences accompanied by DNA sequences that encode them. It is to be understood that the invention is not limited to the particular DNA sequence shown.

**Example 1:** Expression and display of BPTI, ITI-D1, and other Kunitz Domains.

Table 30 shows a display gene that encodes: 1) the M13 III signal peptide, 2) BPTI, and 3) the first few amino-acids of mature M13 III protein. Phage have been made in which this gene is the only *iii*-like gene so that all copies of III expressed are expected to be modified at the amino terminus of the mature protein. Substitutions in the BPTI domain can be made in the cassettes delimited by the *Acc*III, *Xho*I, *Pfl*MI, *Apa*I, *Bss*HII, *Stu*I, *Xca*I, *Esp*I, *Sph*I, or *Nar*I (same recognition as *Kas*I) sites. Table 100 gives amino-acid sequences of a number of Kunitz domains, some of which inhibit hNE. Each of the hNE-inhibiting sequences shown in Table 100 can be expressed as an intact hNE-binding protein or can be incorporated into a larger protein as a domain. Proteins that comprise a substantial part of one of the hNE-inhibiting sequences found in Table 100 are expected to exhibit hNE-inhibitory activity. This is particularly true if the sequence beginning with the first cysteine and continuing through the last cysteine is retained.

ITI domain 1 is a Kunitz domain as discussed below. The ability of display phage to be retained on matrices that display hNE is related to the affinity of the particular Kunitz domain (or other protein) displayed on the phage.

Expression of the *ITI domain 1::iii* fusion gene and display of the fusion protein on the surface of phage were demonstrated by Western analysis and phage titer neutralization experiments. The infectivity of ITI-D1-display phage was blocked by up to 99 % by antibodies that bind ITI while wild-type phage were unaffected.

Table 35 gives the sequence of a fusion gene comprising: a) the signal sequence of M13 III, b) ITI-D1, and c) the initial part of mature III of M13. The displayed ITI-D1 domain can be altered by standard methods including: i) oligonucleotide-directed mutagenesis of single-stranded phage DNA, and ii) cassette mutagenesis of RF DNA using the restriction sites (*Bgl*I, *Eag*I, *Nco*I, *Sty*I, *Pst*I, and *Kas*I (two sites)) designed into the gene.

**Example 2**: Fractionation of MA-ITI-D1 phage bound to agarose-immobilized protease beads.

To test if phage displaying the ITI-D1::III fusion protein interact strongly with the proteases human neutrophil elastase (hNE), aliquots of display phage were incubated with agarose-immobilized hNE beads ("hNE beads"). The beads were washed and bound phage eluted by pH fractionation as described in US 5,223,409. The pHs used in the step gradient were 7.0, 6.0, 5.5, 5.0, 4.5, 4.0, 3.5, 3.0, 2.5, and 2.0. Following elution and neutralization, the various input, wash, and pH elution fractions were titered. Phage displaying ITI-D1 were compared to phage that display EpiNE-7.

The results of several fractionations are shown in Table 212 (EpiNE-7 or MA-ITI-D1 phage bound to hNE beads). The pH elution profiles obtained using the control display phage (EpiNE-7) were similar previous profiles (US 5,223,409). About 0.3% of the EpiNE-7 display phage applied to the hNE beads eluted during the fractionation procedure and the elution profile had a maximum for elution at about pH 4.0.

The MA-ITI-D1 phage show no evidence of great affinity for hNE beads. The pH elution profiles for MA-ITI-D1 phage bound to hNE beads show essentially monotonic decreases in phage recovered with decreasing pH. Further, the total fractions of the phage applied to the beads that were recovered during the fractionation procedures were quite low: 0.002%.

Published values of $K_i$ for inhibition neutrophil elastase by the intact, large ($M_r$=240,000) ITI protein range between 60 and 150 nM (SWAI88, ODOM90). Our own measurements of pH fraction of display phage bound to hNE beads show that phage displaying proteins with low affinity (> 1 $\mu$M) for hNE are not bound by the beads while phage displaying proteins with greater affinity (nM) bind to the beads and are eluted at about pH 5. If the first Kunitz-type domain of the ITI light chain is entirely responsible for the inhibitory activity of ITI against hNE, and if this domain is correctly displayed on the MA-ITI-D1 phage, then it appears that the minimum affinity of an inhibitor for hNE that allows binding and fractionation of display phage on hNE beads is between 50 and 100 nM.

**Example 3:** Alteration of the P1 region of ITI-D1.

We assume that ITI-D1 and EpiNE-7 have the same 3D configuration in solution as BPTI. Although EpiNE-7 and ITI-D1 are identical at positions 13, 17, 20, 32, and 39, they differ greatly in their affinities for hNE. To improve the affinity of ITI-D1 for hNE, the EpiNE-7 sequence $\underline{Val_{15}}$-$\underline{Ala_{16}}$-$Met_{17}$-$\underline{Phe_{18}}$-$\underline{Pro_{19}}$-$Arg_{20}$ (**bold**, underscored amino acids are alterations) was incorporated into the ITI-D1 sequence by cassette mutagenesis between the *Eag*I and *Sty*I/*Nco*I sites shown in Table 35. Phage isolates containing the ITI-D1::III fusion gene with the EpiNE-7 changes around the P1 position are called MA-ITI-D1E7.

**Example 4:** Fractionation of MA-ITI-D1E7 phage.

To test if ITI-D1E7-display phage bind hNE beads, pH elution profiles were measured. Aliquots of EpiNE-7, MA-ITI-D1, and MA-ITI-D1E7 display phage were incubated with hNE beads for three hours at room temperature (RT). The beads were washed and phage were eluted as described in US 5,223,409, except that only three pH elutions were performed. These data are in Table 215. The pH elution profile of EpiNE-7 display phage is as described. MA-ITI-D1E7 phage show a broad elution maximum around pH 5. The total fraction of MA-ITI-D1E7 phage obtained on pH elution from hNE beads was about 40-fold less than that obtained using EpiNE-7 display phage.

The pH elution behavior of MA-ITI-D1E7 phage bound to hNE beads is qualitatively similar to that seen using BPTI[K15L]-III-MA phage. BPTI with the K15L mutation has an affinity for hNE of ≈ 3 nM. (Alterations and mutations are indicated by giving the original (wild-type) amino-acid type, then the position, and then the new amino-acid type; thus K15L means change $Lys_{15}$ to Leu.) Assuming all else remains the same, the pH elution profile for MA-ITI-D1E7 suggests that the affinity of the free ITI-D1E7 domain for hNE might be in the nM range. If this is the case, the substitution of the EpiNE-7 sequence in place of the ITI-D1 sequence around the P1 region has produced a 20- to 50-fold increase in affinity for hNE (assuming $K_i$ = 60 to 150 nM for the unaltered ITI-D1).

If EpiNE-7 and ITI-D1E7 have the same solution structure, these proteins present the identical amino acid sequences to hNE over the interaction surface. Despite this similarity, EpiNE-7 exhibits a roughly 1000-fold greater affinity for hNE than does ITI-D1E7. This observation highlights the importance of non-contacting secondary residues in modulating interaction strengths.

Native ITI light chain is glycosylated at two positions, $Ser_{10}$ and $Asn_{45}$ (GEBH86). Removal of the glycosaminoglycan chains has been shown to decrease the affinity of the inhibitor for hNE about 5-fold (SELL87). Another potentially important difference between EpiNE-7 and ITI-D1E7 is that of net charge. The changes in BPTI that produce EpiNE-7 reduce the total charge on the molecule from + 6 to +1. Sequence differences between EpiNE-7 and ITI-D1E7 further reduce the charge on the latter to -1. Furthermore, the change in net charge between these two molecules arises from sequence differences occurring in the central portions of the molecules. Position 26 is Lys in EpiNE-7 and is Thr in ITI-D1E7, while at position 31 these residues are Gln and Glu, respectively. These changes in sequence not only alter the net charge on the molecules but also position a negatively charged residue close to the interaction surface in ITI-D1E7. It may be that the occurrence of a negative charge at position 31 (which is not found in any other of the hNE inhibitors described here) destabilized the inhibitor-protease interaction.

**Example 5:** Preparation of BITT-E7 Phase

Possible reasons for MA-ITI-D1E7 phage having lower affinity for hNE than do MA-EpiNE7 phage include: a) incorrect cleavage of the IIIsignal::ITI-D1E7::matureIII fusion protein, b) inappropriate negative charge on the ITI-D1E7 domain, c) conformational or dynamic changes in the Kunitz backbone caused by substitutions such as $Phe_4$ to $Ser_4$, and d) non-optimal amino acids in the ITI-D1E7:hNE interface, such as $Q_{34}$ or $A_{11}$.

To investigate the first three possibilities, we substituted the first four amino acids of EpiNE7 for the first four amino acids of ITI-D1E7. This substitution should provide a peptide that can be cleaved by signal peptidase-I in the same manner as is the IIIsignal::EpiNE7::matureIII fusion. Furthermore, $Phe_4$ of BPTI is part of the hydrophobic core of the protein; replacement with serine may alter the stability or dynamic character of ITI-D1E7 unfavorably. ITI-D1E7 has a negatively charged Glu at position 2 while EpiNE7 has Pro. We introduced the three changes at the amino terminus of the ITI-D1E7 protein (K1R, E2P, and S4F) by oligonucleotide-directed mutagenesis to produce BITI-E7; phage that display BITI-E7 are called MA-BITI-E7.

We compared the properties of the ITI-III fusion proteins displayed by phage MA-ITI-D1 and MA-BITI using Western analysis as described previously and found no significant differences in apparent size or relative abundance of the fusion proteins produced by either display phage strain. Thus, there are no large differences in the processed forms of either fusion protein displayed on the phage. By extension, there are also no large differences in the processed forms of the gene III fusion proteins displayed by MA-ITI-D1E7 and MA-EpiNE7. Large changes in protein conformation due to altered processing are therefore not likely to be responsible for the great differences in binding to hNE-beads shown by MA-ITI-D1E7 and MA-EpiNE7 display phage.

We characterized the binding properties to hNE-beads of MA-BITI and MA-BITI-E7 display phage using the extended pH fractionation procedure described in US 5,223,409. The results are in Table 216. The pH elution profiles for MA-BITI and MA-BITI-E7 show significant differences from the profiles exhibited by MA-ITI-D1 and MA-ITI-D1E7. In both cases, the alterations at the putative amino terminus of the displayed fusion protein produce a several-fold increase in the fraction of the input display phage eluted from the hNE-beads.

The binding capacity of hNE-beads for display phage varies among preparations of beads and with age for each individual preparation of beads. Thus, it is difficult to directly compare absolute yields of phage from elutions performed at different times. For example, the fraction of MA-EpiNE7 display phage recovered from hNE-beads varies two-fold among the experiments shown in Tables 212, 215, and 216. However, the shapes of the pH elution profiles are similar. It is possible to correct somewhat for variations in binding capacity of hNE-beads by normalizing display phage yields to the total yield of MA-EpiNE7 phage recovered from the beads in a concurrent elution. When the data shown in Tables 212, 215, and 216 are so normalized, the recoveries of display phage, relative to recovered MA-EpiNE7, are shown in Table 10.

Table 10: Recovery of Display phage

| Display Phage strain | Normalized fraction of input |
|---|---|
| MA-ITI-D1 | 0.0067 |
| MA-BITI | 0.018 |
| MA-ITI-D1E7 | 0.027 |
| MA-BITI-E7 | 0.13 |

Thus, the changes in the amino terminal sequence of the displayed protein produce a three- to five-fold increase in the fraction of display phage eluted from hNE-beads.

In addition to increased binding, the changes introduced into MA-BITI-E7 produce phage that elute from hNE-beads at a lower pH than do the parental MA-ITI-DIE7 phage. While the parental display phage elute with a broad pH maximum centered around pH 5.0, the pH elution profile for MA-BITI-E7 display phage has a pH maximum at around pH 4.75 to pH 4.5.

The pH elution maximum of the MA-BITI-E7 display phage is between the maxima exhibited by the BPTI(K15L) and BPTI(K15V, R17L) display phage (pH 4.75 and pH 4.5 to pH 4.0, respectively) described in US 5,223,409. From the pH maximum exhibited by the display phage we predict that the BITI-E7 protein free in solution may have an affinity for hNE in the 100 pM range. This would represent an approximately ten-fold increase in affinity for hNE over that estimated above for ITI-D1E7.

As was described above, Western analysis of phage proteins show that there are no large changes in gene III fusion proteins upon alteration of the amino terminal sequence. Thus, it is unlikely that the changes in affinity of display phage for hNE-beads can be attributed to large-scale alterations in protein folding resulting from altered ("correct") processing of the fusion protein in the amino terminal mutants. The improvements in binding may in part be due to: 1) the decrease in the net negative charge (-1 to 0) on the protein arising from the Glu to Pro change at position 2, or 2) increased protein stability resulting from the Ser to Phe substitution at residue 4 in the hydrophobic core of the protein, or 3) the combined effects of both substitutions.

**Example 6:** <u>Production and properties of MA-BITI-E7-1222 and MA-BITI-E7-141</u>

Within the presumed Kunitz:hNE interface, BITI-E7 and EpiNE7 differ at only two positions: 11 and 34. In EpiNE7 these residues are Thr and Val, respectively. In BITI-E7 they are Ala and Gln. In addition BITI-E7 has Glu at 31 while EpiNE7 has Gln. This negative charge may influence binding although the residue is not directly in the interface. We used oligonucleotide-directed mutagenesis to investigate the effects of substitutions at positions 11, 31 and 34 on the protease:inhibitor interaction.

ITI-D1 derivative BITT-E7-1222 is BITI-E7 with the alteration A11T. ITI-D1 derivative BITI-E7-141 is BITI-E7 with the alterations E31Q and Q34V; phage that dhe presence of tisplay these proteins are MA-BITI-E7-1222 and MA-BITI-E7-141. We determined the binding properties to hNE-beads of MA-BITI-E7-1222 and MA-BITI-E7-141 display phage using the extended pH fractionation protocol described previously. The results are in Tables 217 (for MA-BITI-E7 and MA-BITI-E7-1222) and 218 (for MA-EpiNE7 and MA-BITI-E7-141). The pH elution profiles for the MA-BITI-E7 and MA-BITI-E7-1222 phage are almost identical. Both phage strains exhibit pH elution profiles with identical maxima (between pH 5.0 and pH 4.5) as well as the same total fraction of input phage eluted from the hNE-beads (0.03%). Thus, the T11A substitution in the displayed ITI-D1 derivative has no appreciable effect on the binding to hNE-beads.

In contrast, the changes at positions 31 and 34 strongly affect the hNE-binding properties of the display phage. The elution profile pH maximum of MA-BITI-E7-141 phage is shifted to lower pH relative to the parental MA-BITI-E7 phage. Further, the position of the maximum (between pH 4.5 and pH 4.0) is identical to that exhibited by MA-EpiNE7 phage in this experiment. Finally, the MA-BITI-E7-141 phage show a ten-fold increase, relative to the parental MA-BITI-E7, in the total fraction of input phage eluted from hNE-beads (0.3% *vs* 0.03%). The total fraction of MA-BITI-E7-141 phage eluted from the hNE-beads is nearly twice that of MA-EpiNE7 phage.

The above results show that binding by MA-BITI-E7-141 display phage to hNE-beads is comparable to that of MA-EpiNE7 phage. If the two proteins (EpiNE7 and BITI-E7-141) in solution have similar affinities for hNE, then the affinity of the BITI-E7-141 protein for hNE is on the order of 1 pM. Such an affinity is approximately 100-fold greater than that estimated above for the parental protein (BITI-E7) and is $10^5$ to $10^6$ times as great as the affinity for hNE reported for the intact ITI protein.

**Example 7:** <u>Mutagenesis of BITI-E7-141</u>

BITI-E7-141 differs from ITI-D1 at nine positions (1, 2, 4, 15, 16, 18, 19, 31, and 34). To obtain the protein having the fewest changes from ITI-D1 while retaining high specific affinity for hNE, we have investigated the effects of reversing the changes at positions 1, 2, 4, 16, 19, 31, and 34. The derivatives of BITI-E7-141 that were tested are MUT1619, MUTP1, and MUTT26A. The derivatives of BITI that were tested are AMINO1 and AMINO2. The derivative of BITI-E7 that was tested is MUTQE. All of these sequences are shown in Table 100. MUT1619 restores the ITI-D1 residues $Ala_{16}$ and $Ser_{19}$. The sequence designated "MUTP1" asserts the amino acids $I_{15}$, $G_{16}$, $S_{19}$ in the context of BITI-E7-141. It is likely that $M_{17}$ and $F_{18}$ are optimal for high affinity hNE binding. $G_{16}$ and $S_{19}$ occurred frequently in the high affinity hNE-binding BPTI-variants obtained from fractionation of a library of BPTI-variants against hNE (ROBE92). Three changes at the putative amino terminus of the displayed ITI-D1 domain were introduced to produce the MA-BITI series of phage. AMINO1 carries the sequence $K_1$- $E_2$ while AMINO2 carries $K_1$-$S_4$. Other amino acids in the amino-terminal region of these sequences are as in ITI-D1. MUTQE is derived from BITI-E7-141 by the alteration Q31E (reasseting the ITI-D1 w.t. residue). Finally, the mutagenic oligonucleotide MUTT26A is intended to remove a potential site of N-linked glycosylation, $N_{24}$-$G_{25}$-$T_{26}$. In the intact ITI molecule isolated from human serum, the light chain polypeptide is glycosylated at this site ($N_{45}$, ODOM90). It is likely that $N_{24}$ will be glycosylated if the BITI-E7-141 protein is produced *via* eukaryotic expression. Such glycosylation may render the protein immunogenic when used for long-term treatment. The MUTT26A contains the alteration T26A and removes the potential glycosylation site with minimal changes in the overall chemical properties of the residue at that position. In addition, an Ala residue is frequently found in other BPTI homologues at position 26 (see Table 34 of US 5,223,409). Mutagenesis was performed on ssDNA of MA-BITI-E7-141 phage.

Example 8: hNE-binding properties of mutagenized MA-BITI-E7-141 display phage

Table 219 shows pH elution data for various display phage eluted from hNE-beads. Total pfu applied to the beads are in column two. The fractions of this input pfu recovered in each pH fraction of the abbreviated pH elution protocol (pH 7.0, pH 3.5, and pH 2.0) are in the next three columns. For data obtained using the extended pH elution protocol, the pH 3.5 listing represents the sum of the fractions of input recovered in the pH 6.0, pH 5.5, pH 5.0, pH 4.5, pH 4.0, and pH 3.5 elution samples. The pH 2.0 listing is the sum of the fractions of input obtained from the pH 3.0, pH 2.5, and pH 2.0 elution samples. The total fraction of input pfu obtained throughout the pH elution protocol is in the sixth column. The final column of the table lists the total fraction of input pfu recovered, normalized to the value obtained for MA-BITI-E7-141 phage.

Two factors must be considered when making comparisons among the data shown in Table 219. The first is that due to the kinetic nature of phage release from hNE-beads and the longer time involved in the extended pH elution protocol, the fraction of input pfu recovered in the pH 3.5 fraction will be enriched at the expense of the pH 2.0 fraction in the extended protocol relative to those values obtained in the abbreviated protocol. The magnitude of this effect can be seen by comparing the results obtained when MA-BITI-E7-141 display phage were eluted from hNE-beads using the two protocols. The second factor is that, for the range of input pfu listed in Table 219, the input pfu influences recovery. The greater the input pfu, the greater the total fraction of the input recovered in the elution. This effect is apparent when input pfu differ by: more than a factor of about 3 to 4. The effect can lead to an overestimate of affinity of display phage for.hNE-beads when data from phage applied at higher titers is compared with that from phage applied at lower titers.

With these caveats in mind, we can interpret the data in Table 219. The effects of the mutations introduced into MA-BITI-E7-141 display phage ("parental") on binding of display phage to hNE-beads can be grouped into three categories: those changes that have little or no measurable effects, those that have moderate (2- to 3-fold) effects, and those that have large (>5-fold) effects.

The MUTT26A and MUTQE changes appear to have little effect on the binding of display phage to hNE-beads. In terms of total pfu recovered, the display phage containing these alterations bind as well as the parental to hNE-beads. Indeed, the pH elution profiles obtained for the parental and the MUTT26A. display phage from the extended pH elution protocol are indistinguishable. The binding of the MUTTQE display phage appears to be slightly reduced relative to the parental and, in light of the applied pfu, it is likely that this binding is somewhat overestimated.

The sequence alterations introduced via the MUTP1 and MUT1619 oligonucleotides appear to reduce display phage binding to hNE-beads about 2- to 3-fold. In light of the input titers and the distributions of pfu recovered among the various elution fractions, it is likely that 1) both of these display phage have lower affinities for hNE-beads than do MA-EpiNE7 display phage, and 2) the MUT1619 display phage have a greater affinity for hNE-beads than do the MUTP1 display phage.

The sequence alterations at the amino terminus of BITI-E7-14 appear to reduce binding by the display phage to hNE-beads at least ten fold. The AMINO2 changes are likely to reduce display phage binding to a substantially greater extent than do the AMINO1 changes.

On the basis of the above interpretations of the data in Table 219, we can conclude that:

1.) The substitution of ALA for THR at position 26 in ITI-D1 and its derivatives has no effect on the interaction of the inhibitor with hNE. Thus, the possibility of glycosylation at $Asn_{24}$ of an inhibitor protein produced in eukaryotic cell culture can be avoided with no reduction in affinity for hNE.

2.) The increase in affinity of display phage for hNE-beads from the changes E31Q and Q34V results primarily from the Val substitution at 34.

3.) All three changes at the amino terminal region of ITI-D1 (positions 1,2, and 4) influence display phage binding to hNE-beads to varying extents. The S4F alteration seems to have about the same effect as does E2P. The change at position 1 appears to have only a small effect.

4.) The changes in the region around the P1 residue in BITI-E7-141 (position 15) influence display phage binding to hNE. The changes A16G and P19S appear to reduce the affinity of the inhibitor somewhat (perhaps 3-fold). The substitution of I15V further reduces binding.

BITI-E7-141 differs from ITI-D1 at nine positions. From the discussion above, it appears likely that a high affinity hNE-inhibitor based on ITI-D1 could be constructed that would differ from the ITI-D1 sequence at only five or six positions. These differences would be: Pro at position 2, Phe at position 4, Val at position 15, Phe at position 18, Val at position 34, and Ala at position 26. If glycosylation of $Asn_{24}$ is not a concern Thr could be retained at 26.

Summary: estimated affinities of isolated ITI-D1 derivatives for hNE

On the basis of display phage binding to and elution from hNE beads, it is possible to estimate affinities for hNE that various derivatives of ITI-D1 may display free in solution. These estimates are summarized in Table 55.

**hNE Inhibitors Derived from ITI Domain 2**

In addition to hNE inhibitors derived from ITI-D1, the present invention comprises hNE inhibitors derived from ITI-D2. These inhibitors have been produced in *Pichia pastoris* in good yield. EPI-HNE-4 inhibits human neutrophil elastase with a $K_D \approx 5$ pM.

**PURIFICATION AND PROPERTIES OF EPI-HNE PROTEINS**

**I. EPI-HNE Proteins.**

Example 9: Amino-acid sequences of EPI-HNE-3 and EPI-HNE-4

Table 100 gives amino acid sequences of four human-neutrophil-elastase (hNE) inhibitor proteins: EPI-HNE-1 (identical to EpiNE1), EPI-HNE-2, EPI-HNE-3, and EPI-HNE-4. These proteins have been derived from the parental Kunitz-type domains shown. Each of the proteins is shown aligned to the parental domain using the six cysteine residues (shaded) characteristic of the Kunitz-type domain. Residues within the inhibitor proteins that differ from those in the parental protein are in upper case. Entire proteins having the sequences EPI-HNE-1, EPI-HNE-2, EPI-HNE-3, and EPI-HNE-4 (Table 100) have been produced. Larger proteins that comprise one of the hNE-inhibiting sequences are expected to have potent hNE-inhibitory activity; EPI-HNE-1, EPI-HNE-2, EPI-HNE-3, and EPI-HNE-4 are particularly preferred. It is expected that proteins that comprise a significant part of one of the hNE-inhibiting sequences found in Table 100 (particularly if the sequence starting at or before the first cysteine and continuing through or beyond the last cysteine is retained) will exhibit potent hNE-inhibitory activity.

The hNE-inhibitors EPI-HNE-1 and EPI-HNE-2 are derived from the bovine protein BPTI (aprotinin). Within the Kunitz-type domain, these two inhibitors differ from BPTI at the same eight positions: K15I, R17F, I18F, I19P, R39M, A40G, K41N, and R42G. In addition, EPI-HNE-2 differs from both BPTI and EPI-HNE-1 in the presence of four additional residues (EAEA) present at the amino terminus. These residues were added to facilitate secretion of the protein in *Pichia pastoris*.

EPI-HNE-3 is derived from the second Kunitz domain of the light chain of the human inter-α-trypsin inhibitor protein (ITI-D2). The amino acid sequence of EPI-HNE-3 differs from that of ITI-D2(3-58) at only four positions: R15I, I18F, Q19P and L20R. EPI-HNE-4 differs from EPI-HNE-3 by the substitution A3E (the amino-terminal residue) which both facilitates secretion of the protein in *P. pastoris* and improves the $K_D$ for hNE. Table 602 gives some physical properties of the hNE inhibitor proteins. All four proteins are small, high-affinity ($K_i$=2 to 6 pM), fast-acting ($k_{on}$=4 to 11 $\times 10^6$ $\underline{M}^{-1}s^{-1}$) inhibitors of hNE.

**II. Production of the hNE-inhibitors EPI-HNE-2, EPI-HNE-3, and EPI-HNE-4.**

**Example 10:** *Pichia pastoris* production system.

Transformed strains of *Pichia pastoris* were used to express the various EPI-HNE proteins derived from BPTI and ITI-D2. Protein expression cassettes are cloned into the plasmid pHIL-D2 using the *Bst*BI and *Eco*RI sites (Table 111). The DNA sequence of pHIL-D2 is given in Table 250. The cloned gene is under transcriptional control of *P. pastoris* upstream (labeled "aoxl 5'") *aox1* gene promoter and regulatory sequences (dark shaded region) and downstream polyadenylation and transcription termination sequences (second cross-hatched region, labeled "aoxl 3'"). *P. pastoris* GS115 is a mutant strain containing a non-functional histidinol dehydrogenase (*his4*) gene. The *his4* gene contained on plasmid pHIL-D2 and its derivatives can be used to complement the histidine deficiency in the host strain. Linearization of plasmid pHIL-D2 at the indicated *Sac*I site directs plasmid incorporation into the host genome at the *aox1* locus by homologous recombination during transformation. Strains of *P. pastoris* containing integrated copies of the expression plasmid will express protein genes under control of the *aox1* promoter when the promoter is activated by growth in the presence of methanol as the sole carbon source.

We have used this high density *Pichia pastoris* production system to produce proteins by secretion into the cell CM. Expression plasmids were constructed by ligating synthetic DNA sequences encoding the *S. cerevisiae* mating factor a prepro peptide fused directly to the amino terminus of the desired hNE inhibitor into the plasmid pHIL-D2 using the *Bst*BI and the *Eco*RI sites shown. Table 251 gives the DNA sequence of a *Bst*BI-to-*Eco*RI insert that converts pHIL-D2 into pHIL-D2(MFα-PrePro::EPI-HNE-3). In this construction, the fusion protein is placed under control of the upstream inducible *P. pastoris aox1* gene promoter and the downstream *aox1* gene transcription termination and polyadenylation sequences. Expression plasmids were linearized by *Sac*I digestion and the linear DNA was incorporated by homologous recombination into the genome of the *P. pastoris* strain GS115 by spheroplast transformation. Regenerated spheroplasts were selected for growth in the absence of added histidine, replated, and individual isolates were screened for methanol utilization phenotype (*mut*$^+$), secretion levels, and gene dose (estimated via Southern hybridization experiments). High level secretion stains were selected for production of hNE inhibitors: PEY-33 for production of EPI-HNE-2 and PEY-43 for production of EPI-HNE-3. In both of these strains, we estimate that four copies of the expression plasmid are integrated as a tandem array into the *aox1* gene locus.

To facilitate alteration of the Kunitz-domain encoding segment of pHIL-D2 derived plasmids, we removed two restriction sites given in Table 111: the *Bst*BI at 4780 and the *Aat*II site at 5498. Thus, the Kunitz-domain encoding segment is bounded by unique *Aat*II and *Eco*RI sites. The new plasmids are called pD2pick("insert") where "insert" defines the domain encoded under control of the *aox1* promoter. Table 253 gives the DNA sequence of pD2pick(MFα::EPI-HNE-3). Table 254 gives a list of restriction sites in pD2pick(MFα::EPI-HNE-3).

EPI-HNE-4 is encoded by pD2pick(MFαPrePro::EPI-HNE-4) which differs from pHIL-D2 in that: 1) the *Aat*II/*Eco*RI segment of the sequence given in Table 251 is replaced by the segment shown in Table 252 and 2) the changes in the restriction sites discussed above have been made. Strain PEY-53 is *P. pastoris* GS115 transformed with pD2pick (MFα::EPI-HNE-4).

**Example 11:** Protein Production

To produce the proteins, *P. pastoris* strains were grown in mixed-feed fermentations similar to the procedure described by Digan *et al*. (DIGA89). Although others have reported production of Kunitz domains in *P. pastoris*, it is well known that many secretion systems involve proteases. Thus, it is not automatic that an altered Kunitz domain having a high potency in inhibiting hNE could be secreted from *P. pastoris* because the new inhibitor might inhibit some key enzyme in the secretion pathway. Nevertheless, we have found that *P. pastoris* can secrete hNE inhibitors in high yield.

Briefly, cultures were first grown in batch mode with glycerol as the carbon source. Following exhaustion of glycerol, the culture was grown for about four hours in glycerol-limited feed mode to further increase cell mass and to derepress the *aox1* promoter. In the final production phase, the culture was grown in methanol-limited feed mode. During this phase, the *aox1* promoter is fully active and protein is secreted into the CM.

Table 607 and Table 608 give the kinetics of cell growth (estimated as $A_{600}$) and protein secretion (mg/l) for cultures of PEY-33 and PEY-43 during the methanol-limited feed portions of the relevant fermentations. Concentrations of the inhibitor proteins in the fermentation cultures were determined from *in vitro* assays of hNE inhibition by diluted aliquots of cell-free culture media obtained at the times indicated. Despite similarities in gene dose, fermentation conditions, cell densities, and secretion kinetics, the final concentrations of inhibitor proteins secreted by the two strains differ by nearly an order of magnitude. The final concentration of EPI-HNE-2 in the PEY-33 fermentation CM was 720 mg/l. The final concentration of EPI-HNE-3 in the PEY-43 fermentation CM was 85 mg/l. The differences in final secreted protein concentrations may result from idiosyncratic differences in the efficiencies with which the yeast synthesis and processing systems interact with the different protein sequences.

Strain PEY-33 secreted EPI-HNE-2 protein into the CM as a single molecular species which amino acid composition and N-terminal sequencing reveled to be the correctly-processed Kunitz domain with the sequence shown in Table 601. The major molecular species produced by PEY-43 cultures was the properly-processed EPI-HNE-3' protein. However, this strain also secreted a small amount (about 15 % to 20 % of the total EPI-HNE-3) of incorrectly-processed material. This material proved to be a mixture of proteins with amino terminal extensions (primarily nine or seven residues in length) arising from incorrect cleavage of the MF α PrePro leader peptide from the mature Kunitz domain. The correctly processed protein was purified substantially free of these contaminants as described below.

**III. Purification of the hNE-inhibitors EPI-HNE-2 and EPI-HNE-3.**

The proteins can be readily purified from fermenter CM by standard biochemical techniques. The specific purification procedure varies with the specific properties of each protein as described below.

**Example 12:** Purification of EPI-HNE-2.

Table 603 gives particulars of the purification of EPI-HNE-2, lot 1. The PEY-33 fermenter culture was harvested by centrifugation at 8000 x g for 15 min and the cell pellet was discarded. The 3.3 liter supernatant fraction was microfiltere d used a Minitan Ultrafiltration System (Millipore Corporation, Bedford, MA) equipped with four 0.2μ filter packets.

The filtrate obtained from the microfiltration step was used in two subsequent ultrafiltration steps. First, two 30K ultrafiltrations were performed on the 0.2μ microfiltrate using the Minitan apparatus equipped with eight 30,000 NMWL polysulfone filter plates (#PLTK0MP04, Millipore Corporation, Bedford, MA). The retentate solution from the first 30K ultrafiltration was diluted with 10 mM NaCitrate, pH=3.5, and subjected to a second 30K ultrafiltration. The two 30K ultrafiltrates were combined to give a final volume of 5 liters containing about 1.4 gram of EPI-HNE-2 protein (estimated from hNE-inhibition measurements).

The 30K ultrafiltrate was concentrated with change of buffer in the second ultrafiltration step using the Minitan apparatus equipped with eight 5,000 NMWL filter plates (#PLCC0MP04, Millipore Corporation, Bedford, MA). At two times during the 5K ultrafiltration, the retentate solution was diluted from about 300 ml to 1.5 liters'with 10 mM NaCitrate, pH=3.5. The final 5K ultrafiltration retentate (Ca. 200 ml) was diluted to a final volume of 1000 ml with 10 mM NaCitrate, pH-3.5.

EPI-HNE-2 protein was obtained from the 5K ultrafiltration retentate solution by ammonium sulfate precipitation at RT. 100 ml of 0.25 M ammonium acetate, pH=3.2, (1/10 volume) was added to the 5K ultrafiltration retentate, followed by one final volume (1.1 liter) of 3 M ammonium sulfate. Following a 30 minute incubation at RT, precipitated material was pelleted by centrifugation at 10,000 x g for 45 minutes. The supernatant solution was removed, the pellet was dissolved in water in a final volume of 400 ml, and the ammonium sulfate precipitation was repeated using the ratios described above. The pellet from the second ammonium sulfate precipitation was dissolved in 50 mM sodium acetate, pH=3.5 at a final volume of 300 ml.

Residual ammonium sulfate was removed from the EPI-HNE-2 preparation by ion exchange chromatography. The solution from the ammonium sulfate precipitation step was applied to a strong cation-exchange column (50 ml bed volume Macroprep 50S) (Bio-Rad Laboratories, Inc, Hercules, CA) previously equilibrated with 10 mM NaCitrate, pH=3.5. After loading, the column was washed with 300 ml of 10 mM NaCitrate, pH=3.5. EPI-HNE-2 was then batch-eluted from the column with 300 ml of 50 mM $NH_4OAc$, pH=6.2. Fractions containing EPI-HNE-2 activity were pooled and the resulting solution was lyophilized. The dried protein powder was dissolved in 50 ml $dH_2O$ and the solution was passed through a 0.2μ filter (#4192, Gelman Sciences, Ann Arbor, MI). The concentration of the active inhibitor in the final stock solution was determined to be 2 mM (13.5 mg/ml). This stock solution (EPI-HNE-2, Lot 1) has been stored as 1 ml aliquots at 4°C and -70°C for more than 11 months with no loss in activity.

Table 603 summarizes the yields and relative purity of EPI-HNE-2 at various steps in the purification procedure. The overall yield of the purification procedure was about 30 %. The major source of loss was retention of material in the retentate fractions of the 0.2μ microfiltration and 30k ultrafiltration steps.

**Example 13:** Purification of EPI-HNE-3.

Purification of EPI-HNE-3, lot 1, is set out in Table 604. The PEY-43 fermenter culture was harvested by centrifugation at 8,000 x g for 15 min and the cell pellet was discarded. The supernatant solution (3100 ml) was microfiltered through 0.2μ Minitan packets (4 packets). After the concentration, a diafiltration of the retentate was performed so that the final filtrate volume from the 0.2μ filtration was 3300 ml.

A 30K ultrafiltration was performed on the filtrate from the 0.2μ microfiltration step. When the retentate volume had been reduced to 250 ml, a diafiltration of the retentate was performed at a constant retentate volume (250 ml) for 30 min at a rate of 10 ml/min. The resulting final volume of filtrate was 3260 ml.

EPI-HNE-3 protein and other medium components were found to precipitate from solution when the fermenter CM was concentrated. For this reason, the 5k ultrafiltration step was not performed.

Properly processed EPI-HNE-3 was purified substantially free of mis-processed forms and other fermenter culture components by ion exchange chromatography. A 30 ml bed volume strong cation ion exchange column (Macroprep 50S) was equilibrated with 10 mM NaCitrate pH=3.5. The 30K ultrafiltration filtrate was applied to the column and binding of EPI-HNE-3 to the column was confirmed by demonstrating the complete loss of inhibitor activity in the column flow through. The column was then washed with 300 ml of 10 mM NaCitrate, pH=3.5.

To remove EPI-HNE-3 from the column, we sequentially eluted it with 300 ml volumes of the following solutions:

100 mM ammonium acetate, pH=3.5
50 mM ammonium acetate, pH=4.8
50 mM ammonium acetate, pH=6.0
50 mM ammonium acetate, pH=6.0, 0.1 M NaCl

50 mM ammonium acetate, pH=6.0, 0.2 M NaCl
50 mM ammonium acetate, pH=6.0, 0.3 M NaCl
50 mM ammonium acetate, pH=6.0, 0.4 M NaCl
50 mN Tris/Cl pH=8.0, 1.0 NaCl

The majority of the EPI-HNE-3 eluted in two 50 mM ammonium acetate, pH=6.0 fractions. The 0.1 M NaCl fraction contained about 19 % of the input EPI-HNE-3 activity (28 mg of 159 mg input) and essentially all of the mis-processed forms of EPI-HNE-3. The 0.2M NaCl fraction contained about 72% (114 mg) of the input EPI-HNE-3 and was almost completely free of the higher molecular weight mis-processed forms and other UV-absorbing contaminants. The fractions from the 50 mM ammonium acetate, pH=6.0, 0.2 M NaCl elution having the highest concentrations of EPI-HNE-3 were combined (95 mg).

An ammonium sulfate precipitation was performed on the 0.2 M NaCl, pH=6.0 ion exchange column eluate. 800 ml of 3 M ammonium sulfate was added to 160 ml of eluate solution (final ammonium sulfate concentration = 2.5 M) and the mixture was incubated at RT for 18 hours. The precipitated material was then pelleted by centrifugation at 10,000 x g for 45 minutes. The supernatant fluid was discarded and the pelleted material was dissolved in 100 ml of water.

Residual ammonium sulfate was removed from the EPI-HNE-3 preparation by batch ion exchange chromatography. The pH of the protein solution was adjusted to 3.0 with diluted (1/10) HOAc and the solution was then applied to a 10 ml bed volume Macroprep 50S column that had been equilibrated with 10 mM NaCitrate, pH=3.5. Following sample loading, the column was washed with 100 ml of 10 mM NaCitrate, pH=3.5 followed by 100 ml of $dH_2O$. EPI-HNE-3 was eluted from the column with 100 ml of 50 mM $NH_4CO_3$, pH=9.0. pH9 fractions having the highest concentrations of EPI-HNE-3 were combined (60 mg) and stored at 4°C for 7 days before lyophilization.

The lyophilized protein powder was dissolved in 26 ml $dH_2O$ and the solution was passed through a 0.2$\mu$ filter (#4912, Gelman Sciences, Ann Arbor, MI). The concentration of active inhibitor in the final stock solution was found to be 250 $\mu$M (1.5 mg/ml). This stock solution (EPI-HNE-3, Lot 1) has been stored as 1 ml aliquots at -70°C for more than six months with no loss of activity. EPI-HNE-3 stored in water solution (without any buffering) deteriorated when kept at 4°C. After five months, about 70 % of the material was active with a $K_i$ of about 12 pM.

Table 604 gives the yield and relative purity of EPI-HNE-3 at various steps in the purification procedure. A major purification step occurred at the first ion exchange chromatography procedure. The ammonium sulfate precipitation step provided only a small degree of further purification. Some loss of inhibitor activity occurred on incubation at pH=9 (See pH stability data). The production and purification of EPI-HNE-1 and EPI-HNE-4 were analogous to that of EPI-HNE-2 and EPI-HNE-3.

**Example 14:** Tricine-PAGE Analysis of EPI-HNE-2 and EPI-HNE-3.

The high resolution tricine gel system of Schagger and von Jagow (SCHA87) was used to analyze the purified proteins produced (*vide supra*). For good resolution of the low molecular weight EPI-HNE proteins we used a 16.5% resolving layer with 10 % separating and 4% stacking layers. Following silver staining, we inspected a gel having:

Lane 1: EPI-HNE-2 25 ng,
Lane 2: EPI-HNE-2 50 ng,
Lane 3: EPI-HNE-2 100 ng,
Lane 4: EPI-HNE-2 200 ng,
Lane 5: EPI-HNE-3 25 ng,
Lane 6: EPI-HNE-3 50 ng,
Lane 7: EPI-HNE-3 100 ng,
Lane 8: EPI-HNE-3 200 ng, and
Lane 9: Molecular-weight standards: RPN 755, (Amersham Corporation, Arlington Heights, IL).

Stained proteins visible on the gel and their molecular weights in Daltons are: ovalbumin (46,000), carbonic anhydrase (30,000), trypsin inhibitor (21;500), lysozyme (14,300), and aprotinin (6,500). The amount of protein loaded was determined from measurements of hNE-inhibition. We found the following features. EPI-HNE-2, Lot 1 shows a single staining band of the anticipated size (*ca.* 6,700 D) at all loadings. Similarly, EPI-HNE-3, Lot 1 protein shows a single staining band of the anticipated size (*ca.* 6,200 D). At the highest loading, traces of the higher molecular weight (*ca*. 7,100 D) incorrectly processed form can be detected. On the basis of silver-stained high-resolution PAGE analysis, the purity of both protein preparations is assessed to be significantly greater than 95%.

**IV. Properties of EPI-HNE-2 and EPI-HNE-3.**

A. Inhibition of hNE.

**Example 15:** Measured $K_D$s of EPI-HNE proteins for hNE

Inhibition constants for the proteins reacting with hNE ($K_i$) were determined using RT measurements of hydrolysis of a fluorogenic substrate (N-methoxysuccinyl-Ala-Ala-Pro-Val-7-amino-4-methylcoumarin, Sigma M-9771) by hNE. For these measurements, aliquots of the appropriately diluted inhibitor stocks were added to 2 ml solutions of 0.847 nM hNE in reaction buffer (50 mM Tris-Cl, pH = 8.0, 150 mM NaCl, 1 mM $CaCl_2$, 0.25 % Triton-X-100) in plastic fluorescence cuvettes. The reactions were incubated at RT for 30 minutes. At the end of the incubation period, the fluorogenic substrate was added at a concentration of 25 $\mu$M and the time course for increase in fluorescence at 470 nm (excitation at 380 nm) due to enzymatic substrate cleavage was recorded using a spectrofluorimeter (Perkin-Elmer 650-15) and strip chart recorder. We did not correct for competition between substrate and inhibitor because ($S_0/K_m$) is 0.07 (where $S_0$ is the substrate concentration and $K_m$ is the binding constant of the substrate for hNE). $K_i$ is related to $K_{apparent}$ by $K_i = K_{apparent}$ x (1/ (1 + ($S_0/K_m$))). The correction is small compared to the possible errors in $K_{apparent}$. Rates of enzymatic substrate cleavage were determined from the linear slopes of the recorded increases in fluorescence. The percent residual activity of hNE in the presence of the inhibitor was calculated as the percentage of the rate of fluorescence increase observed in the presence of the inhibitor to that observed when no added inhibitor was present.

We recorded data used to determine $K_i$ for EPI-HNE-2 and EPI-HNE-3 reacting with hNE. Data obtained as described above are recorded as percent residual activity plotted as a function of added inhibitor. Values for $K_i$ and for active inhibitor concentration in the stock are obtained from a least-squares fit program. From the data, $K_i$ values for EPI-HNE-2 and for EPI-HNE-3 reacting with hNE at RT were calculated to be 4.8 pM and 6.2 pM, respectively. Determinations of $K_i$ for EPI-HNE-2 and EPI-HNE-3 reacting with hNE are given in Table 610 and Table 611.

The kinetic on-rates for the inhibitors reacting with hNE ($k_{on}$) were determined from measurements of progressive inhibition of substrate hydrolytic activity by hNE following addition of inhibitor. For these experiments, a known concentration of inhibitor was added to a solution of hNE (0.847 nM) and substrate (25 $\mu$M) in 2 ml of reaction buffer in a plastic fluorescence cuvette. The change in fluorescence was recorded continuously following addition of the inhibitor. In these experiments, sample fluorescence did not increase linearly with time. Instead, the rate of fluorescence steadily decreased reflecting increasing inhibition of hNE by the added inhibitor. The enzymatic rate at selected times following addition of the inhibitor was determined from the slope of the tangent to the fluorescence time course at that time.

The kinetic constant $k_{on}$ for EPI-HNE-2 reacting with hNE was determined as follows. EPI-HNE-2 at 1.3 nM was added to buffer containing 0.867 nM hNE (I:E = 1.5:1) at time 0. Measured percent residual activity was recorded as a function of time after addition of inhibitor. A least-squares fit program was used to obtain the value of $k_{on}$ = 4.0 x $10^6$ $M^{-1}s^{-1}$.

The kinetic off rate, $k_{off}$, is calculated from the measured values of $K_i$ and $k_{on}$ as:

$$k_{off} = K_D \times k_{on} \quad .$$

The values from such measurements are included in Table 602. The EPI-HNE proteins are small, high affinity, fast acting inhibitors of hNE.

B. Specificity.

**Example 16:** Specificity of EPI-HNE proteins

We attempted to determine inhibition constants for EPI-HNE proteins reacting with several serine proteases. The results are summarized in Table 605. In all cases except chymotrypsin, we were unable to observe any inhibition even when 10 to 100 $\mu$M inhibitor was added to enzyme at concentrations in the nM range. In Table 605, our calculated values for $K_i$ (for the enzymes other than chymotrypsin) are based on the conservative assumption of less than 10% inhibition at the highest concentrations of inhibitor tested. For chymotrypsin, the $K_i$ is about 10 $\mu$M and is probably not specific.

C. In Vitro Stability.

**Example 17:** Resistance to Oxidative Inactivation.

Table 620 shows measurements of the susceptibility of EPI-HNE proteins to oxidative inactivation as compared with that of two other natural protein hNE inhibitors: $\alpha$ 1 Protease Inhibitor (API) and Secretory Leucocyte Protease Inhibitor (SLPI). API (10 $\mu$M), SLPI (8.5 $\mu$M), EPI-HNE-1 (5 $\mu$M), EPI-HNE-2 (10 $\mu$M), EPI-HNE-3 (10 $\mu$M), and EPI-HNE-4 (10 $\mu$M) were exposed to the potent oxidizing agent, Chloramine-T, at the indicated oxidant:inhibitor ratios in 50 mM phosphate buffer, pH=7.0 for 20 minutes at RT. At the end of the incubation period, the oxidation reactions were quenched by adding methionine to a final concentration of 4 mM. After a further 10 minute incubation, the quenched reactions were diluted and assayed for residual inhibitor activity in our standard hNE-inhibition assay.

Both API and SLPI are inactivated by low molar ratios of oxidant to inhibitor. The Chloramine-T:protein molar ratios required for 50 % inhibition of API and SLPI are about 1:1 and 2:1, respectively. These ratios correspond well with the reported preserice of two and four readily oxidized methionine residues in API and SLPI, respectively. In contrast, all four EPI-HNE proteins retain essentially complete hNE-inhibition activity following exposure to Chloramine-T at all molar ratios tested (up to 50:1, in the cases of EPI-HNE-3 and EPI-HNE-4). Neither EPI-HNE-3 nor EPI-HNE-4 contain any methionine residues. In contrast, EPI-HNE-1 and EPI-HNE-2 each contains two methionine residues (see Table 100). The resistance of these proteins to oxidative inactivation indicates that the methionine residues are either inaccessible to the oxidant or are located in a region of the protein that does not interact with hNE.

**Example 18**: pH Stability.

Table 612 shows the results of measurements of the pH stability of EPI-HNE proteins. The stability of the proteins to exposure to pH conditions in the range of pH 1 to pH 10 was assessed by maintaining the inhibitors in buffers of defined pH at 37°C for 18 hours and determining the residual hNE inhibitory activity in the standard hNE-inhibition assay. Proteins were incubated at a concentration of 1 $\mu$M. The buffers shown in Table 14 were formulated as described (STOL90) and used in the pH ranges indicated:

**Table 14:** Buffers used in stability studies

| Buffer | Lowest pH | Highest pH |
|---|---|---|
| Glycine-HCl | 1 | 2.99 |
| Citrate-Phosphate | 3 | 7 |
| Phosphate | 7 | 8 |
| Glycine-NaOH | 8.5 | 10 |

Both BPTI-derived inhibitors, EPI-HNE-1 and EPI-HNE-2, are stable at all pH values tested. EPI-HNE-3 and EPI-HNE-4, the inhibitors derived from the human protein Kunitz-type domain, were stable when incubated at low pH, but showed some loss of activity at high pH. When incubated at 37°C for 18 hours at pH= 7.5, the EPI-HNE-3 preparation lost 10 to 15 % of its hNE-inhibition activity. EPI-HNE-4 retains almost full activity to pH 8.5. Activity of the ITI-D2-derived inhibitor declined sharply at higher pH levels so that at pH 10 only 30% of the original activity remained. The sensitivity of EPI-HNE-3 to incubation at high pH probably explains the loss of activity of the protein in the final purification step noted previously.

**Example 19:** Temperature Stability.

The stability of EPI-HNE proteins to temperatures in the range 0°C to 95°C was assessed by incubating the inhibitors for thirty minutes at various temperatures and determining residual inhibitory activity for hNE. In these experiments, protein concentrations were 1 $\mu$M in phosphate buffer at pH=7. As is shown in Table 630, the four inhibitors are quite temperature stable.

EPI-HNE-1 and EPI-HNE-2 maintain full activity at all temperatures below about 90°C. EPI-HNE-3 and EPI-HNE-4 maintain full inhibitory activity when incubated at temperatures below 65°C. The activity of the protein declines somewhat at higher temperatures. However, all three proteins retain more than ≈ 50% activity even when incubated at 95 °C for 30 minutes.

**Example 20:** ROUTES to OTHER hNE-INHIBITORY SEQUENCES:

The present invention demonstrates that very high-affinity hNE inhibitors can be devised from Kunitz domains of human origin with very few amino-acid substitutions. It is believed that almost any Kunitz domain can be made into a potent and specific hNE inhibitor with eight or fewer substitutions. In particular, any one of the known human Kunitz domains could be remodeled to provide a highly stable, highly potent, and highly selective hNE inhibitor. There are at least three routes to hNE inhibitory Kunitz domains: 1) replacement of segments known to be involved in specifying hNE binding, 2) replacement of single residues thought to be important for hNE binding, and 3) use of libraries of Kunitz domains to select hNE inhibitors.

**Example 21**: Substitution of Segments in Kunitz Domains

Table 100 shows the amino-acid sequences of 11 human Kunitz domains. These sequences have been broken into ten segments: 1:N terminus-residue 4; 2:residue 5; 3:6-9(or 9a); 4:10-13; 5:14; 6:15-21; 7:22-30, 8:31-36; 8:37-38; 9:39-42; and 10:43-C terminus (or 42a-C terminus).

Segments 1, 3, 5, 7, and 9 contain residues that strongly influence the binding properties of Kunitz domains and are double underscored in the Consensus Kunitz Domain of Table 100. Other than segment 1, all the segments are the same length except for TFPI-2 Domain 2 which carries an extra residue in segment 2 and two extra residues in segment 10.

Segment 1 is at the amino terminus and influences the binding by affecting the stability and dynamics of the protein. Segments 3, 5, 7, and 9 contain residues that contact serine proteases when a Kunitz domain binds in the active site. High-affinity hNE inhibition requires a molecule that is highly complementary to hNE. Segments 3, 5, 7, and 9 supply the amino acids that contact the protease. The sequences in segments 1, 3, 5, 7, and 9 must work together in the context supplied by each other and the other segments. Nevertheless, we have demonstrated that very many different sequences are capable of high-affinity hNE inhibition.

It may be desirable to have an hNE inhibitor that is highly similar to a human protein to reduce the chance of immunogenicity. Candidate high-affinity hNE inhibitor protein sequences may be obtained by taking an aprotonin-type Kunitz domain that strongly or very strongly inhibits hNE, and replacing one, two, three, four or all of segments 2, 4, 6, 8, and 10 with the corresponding segment from a human Kunitz domain, such as those listed in Table 100, or other domain known to have relatively low immunogenicity in humans. (Each of segments 2, 4, 6, 8, and 10 may be taken from the same human domain, or they may be taken from different human domains.) Alternatively, a reduced immunogenicity, high hNE inhibiting domain may be obtained by taking one of the human aprotonin-type Kunitz domains and replacing one, two, three or all of segments 3, 5, 7 and 9 (and preferably also segment 1) with the corresponding segment from one or more aprotonin-like Kunitz domains that strongly or very strongly inhibit hNE. In making these humanized hNE inhibitors, one may, of course, use, rather than a segment identical to that of one of the aforementioned source proteins, a segment which differs from the native source segment by one or more conservative modifications. Such differences should, of course, be taken with due consideration for their possible effect on inhibitory activity and/or immunogenicity. In some cases, it may be advantageous that the segment be a hybrid of corresponding segments from two or more human domains (in the case of segments 2, 4, 6, 8 and 10) or from two or more strong or very strong hNE inhibitor domains (in the case of segments 3, 5, 7, and 9). Segment 1 may correspond to the segment 1 of a strong or very strong hNE inhibitor, or the segment 1 of a human aprotonin-like Kunitz domain, or be a chimera of segment 1's from both.

The proteins DPI.1.1, DPL2.1, DPI.3.1, DPI.4.1, DPI.5.1, DPI.6.3, DPI.7.1, DPI.8.1, and DPI.9.1 were designed in this way. DPI.1.1 is derived from App-I by replacing segments 3, 5, 7, and 9 with the corresponding segments from EPI-HNE-1. DPL2.1 is derived from TFPI2-D1 by replacing segments 3, 5, 7, and 9 with the corresponding residues from EPI-HNE-1. DPI.3.1 is derived from TFPI2-D2 by replacing residues 9a-21 with residues 10-21 of EPI-HNE-4 and replacing residues 31-42b with residues 31-42 of EPI-HNE-4. DPI.4.1 is derived from TFPI2-D3 by replacing segments 3, 5, 7, and 9 with the corresponding residues from MUTQE. DPI.5.1 is derived from LACI-D1 by replacing segments 3, 5, 7, and 9 with the corresponding residues from MUTQE. DPI.6.1 is derived from LACI-D2 by replacing segments 3, 5, 7, and 9 with the corresponding residues from MUTQE. DPI.7.1 is derived from LACI-D3 by replacing segments 3, 5, 7, 9 with the corresponding residues from EPI-HNE-4. DPI.8.1 is derived from the A3 collogen Kunitz domain by substitution of segments 3, 5, 7, and 9 from EPI-HNE-4. DPI.9.1 is derived from the HKI B9 domain by replacing segments 3, 5, 7, and 9 with the corresponding resiudes from EPI-HNE-4.

While the above-described chimera constitute preferred embodiments of the present invention, the invention is not limited to these chimera.

**Example 22:** Point substitutions in Kunitz Domains

In this example, certain substitution mutations are discussed. It must be emphasized that this example describes preferred embodiments of the invention, and is not intended to limit the invention.

All of the protein sequences mentioned in this example are to be found in Table 100. Designed protease inhibitors are designated "DPI" and are derived from human Kunitz domains (also listed in Table 100). Each of the sequences designated DPI.i.2 (for i = 1 to 9) is derived from the domain two above it in the table by making minimal point mutations. Each of the sequences designated DPI.i.3 (for i = 1 to 9) is derived from the sequence three above it by more extensive mutations intended to increase affinity. For some parental domains, additional examples are given. The sequences designated DPI.i.1 are discussed in Example 21.

The most important positions are 18 and 15. Any Kunitz domain is likely to become a good hNE inhibitor if Val or Ile is at 15 (with Ile being preferred) and Phe is at 18. (However, these features are not necessarily required for such activity.)

If a Kunitz domain has Phe at 18 and either Ile or Val at 15 and is not a good hNE inhibitor, there may be one or more residues in the interface preventing proper binding.

The Kunitz domains having very high affinity for hNE herein disclosed (as listed in Table 100) have no charged groups at residues 10, 12 through 19, 21, and 32 through 42. At position 11, only neutral and positively charged groups have been observed in very high affinity hNE inhibitors. At position 31, only neutral and negatively charged groups have been observed in high-affinity hNE inhibitors. If a parental Kunitz domain has a charged group at any of those positions where only neutral groups have been observed, then each of the charged groups is preferably changed to an uncharged group picked from the possibilities in Table 790 as the next step in improving binding to hNE. Similarly, negatively charged groups at 11 and 19 and positively charged groups at 31 are preferably replaced by groups picked from Table 790.

At position 10, Tyr, Ser, and Val are seen in high-affinity hNE inhibitors. Asn or Ala may be allowed since this position may not contact hNE. At position 11, Thr, Ala, and Arg have been seen in high-affinity hNE inhibitors. Gln and Pro are very common at 11 in Kunitz domains and may be acceptable. Position 12 is almost always Gly. If 12 is not Gly, try changing it to Gly.

All of the high-affinity hNE inhibitors produced so far have $Pro_{13}$, but it has not been shown that this is required. Many (62.5%) Kunitz domains have $Pro_{13}$. If 13 is not Pro, then changing to Pro may improve the hNE affinity. Val, Ala, Leu, or Ile may also be acceptable here.

Position 14 is Cys. It is possible to make domains highly similar to Kunitz domains in which the 14-38 disulfide is omitted. Such domains are likely to be less stable than true Kunitz domains having the three standard disulfides.

Position 15 is preferably Ile or Val. Ile is more preferred.

Most Kunitz domains (82%) have either Gly or Ala at 16 and this may be quite important. If residue 16 is not Gly or Ala, change 16 to either Gly or Ala; Ala is preferred. Position 17 in very potent hNE inhibitors has either Phe or Met; those having Ile or Leu at 17 are less potent. Phe is preferred. Met should be used only if resistance to oxidation is not important. Position 18 is Phe.

It has been shown that high-affinity hNE inhibitors may have either Pro or Ser at position 19. Gln or Lys at position 19 may be allowed. At position 21, Tyr and Trp have been seen in very high affinity hNE inhibitors; Phe may also work.

At position 31, Gln, Glu, and Val have been observed in high affinity hNE inhibitors. Since this is on the edge of the binding interface, other types are likely to work well. One should avoid basic types (Arg and Lys). At position 32, Thr and Leu have been observed in high-affinity hNE inhibitors. This residue may not make direct contact and other uncharged types may work well. Pro is very common here. Ser has been seen and is similar to Thr. Ala has been seen in natural Kunitz domains and is unlikely to make any conflict. Position 33 is always Phe in Kunitz domains.

It appears that many amino acid types may be placed at position 34 while retaining high affinity for hNE; large hydrophobic residues (Phe, Trp, Tyr) are unfavorable. Val and Pro are most preferred at 34. Positions 35-38 contain the sequence Tyr-Gly-Gly-Cys. There is a little diversity at position 36 in natural Kunitz domains. In the BPTI-Trypsin complex, changing $Gly_{36}$ to Ser greatly reduces the binding to trypsin. Nevertheless, S36 or T36 may not interfere with binding to hNE and could even improve it. If residue 36 is not Gly, one should consider changing it to Gly.

Position 39 seems to tolerate a variety of types. Met and Gln are known to work in very high-affinity inhibitors. Either Ala or Gly are acceptable at position 40; Gly is preferred. At position 41, Asn is by far the most common type in natural Kunitz domains and may act to stabilize the domains. At position 42, Gly is preferred, but Ala is allowed.

Finally, positions that are highly conserved in Kunitz domains may be converted to the conserved type if needed. For example, the mutations X36G, X37G, X41N, and X12G may be desirable in those cases that do not already have these amino acids at these positions.

The above mutations are summarized in Table 711. Table 711 contains, for example, mutations of the form X15I which means change the residue at position 15. (whatever it is) to Ile or leave it alone if it is already Ile. A Kunitz domain that contains the mutation X18F and either X15I or X15V (X15I preferred) will have strong affinity for hNE. As from one up to about 8 of the mutations found in Table 711 are asserted, the affinity of the protein for hNE will increase so that the $K_i$ approaches the range 1-5 pM.

The sequence DPI.1.2 was constructed from the sequence of App-I by the changes R15I, I18F, and F34V and should be a potent hNE inhibitor. DPL 1.3 is likely to be a more potent inhibitor, having the changes R15I, M17F (to avoid sensitivity to oxidation), I18F, P32T, F34V, and G39M.

DPI.2.2 was derived from the sequence of TFPI2-D1 by the changes R15I, L18F, and L34V and should be a potent hNE inhibitor. DPI.2.3 may be more potent due to the changes Y11T, R15I, L17F, L18F, R31Q, Q32T, L34V, and E39M.

DPI.3.2 is derived from TFPI2-D2 by the changes E15I, T18F, S26A(to prevent glycosylation), K32T, and F34V and should be a potent hNE inhibitor. DPI.3.3 may be more potent by having the changes Δ9a, D11A, D12G, Q13P, E15I, S17F, T18F, E19K, K20R, N24A (to prevent glycosylation), K32T, F34V, and Δ42a-42b.

DPI.4.2 is derived from TFPI2-D3 by the changes S15I, N17F, and V18F and should be a potent inhibitor of hNE. DPI.4.3 may be more potent by having the changes E11T, L13P, S15I, N17F, V18F, A32T, T34V, and T36G.

DPI.5.2 is derived from LACI-D1 by the changes K15I and M18F and is likely to be a potent inhibitor of hNE. DPI.5.3 may be more potent due to the changes D10Y, D11T, K15I, I17F, MI8F, and E32T. Other changes that may improve DPI.5.3 include F21W, I34V, E39M, and Q42G.

The sequence of DPI.6.2 was constructed from the sequence of human LACI-D2 by the mutations R15V and I18F. The rest of the sequence of LACI-D2 appears to be compatible with hNE binding. DPI.6.3 carries two further mutations that make it more like the hNE inhibitors here disclosed: Y17F and K34V. Other alterations that are likely to improve the hNE binding of LACI-D2 include I13P, R32T, and D10S. DPI.6.4 is derived from DPI.6.3 by the additional alteration N25A that will prevent glycosylation when the protein is produced in a eukaryotic cell. Other substitutions that would prevent glycosylation include: N25K, T27A, T27E, N25S, and N25S. DPI.6.5 moves further toward the ITI-D1, ITI-D2, and BPTI derivatives that are known to have affinity for hNE in the 1-5 pM range through the mutations I13P, R15V, Y17F, I18F, T19Q, N25A, K34V, and L39Q. In DPI.6.6, the T19Q and N25A mutations have been reverted. Thus the protein would be glycosylated in yeast or other eukaryotic cells at $N_{25}$. DPI.6.7 carries the alterations I13P, R15I, Y17F, I18F, T19P, K34V, and L39Q.

DPI.7.2 is derived from human LACI domain 3 by the mutations R15V and E18F. DPI.7.3 carries the mutations R15V, N17F, E18F, and T46K. The T46K mutation should prevent glycosylation at $N_{44}$. DPI.7.4 carries more mutations so that it is much more similar to the known high-affinity hNE inhibitors. The mutations are D10V, L13P, R15V, N17F, E18F, K34V, S36G, and T46K. DPI.7.5 carries a different set of alterations: L13P, R15I, N17F, E18F, N19P, F21W, R31Q, P32T, K34V, S36G, and T46K; DPI.7.5 should not be glycosylated in eukaryotic cells.

DPI. 8.2 is derived from the sequence of the A3 collagen Kunitz domain by the changes R15I, D16A, I18F, and W34V and is expected to be a potent hNE inhibitor. DPI.8.3 is derived from the A3 collagen Kunitz domain by the changes T13P, R15I, D16A, I18F, K20R, and W34V.

DPI.9.2 is derived from the HKI B9 Kunitz domain by the changes Q15I, T16A, and M18F and is expected to be a potent hNE inhibitor. DPI.9.3 may be more potent due to the changes Q15I, T16A, M18F, T19P, E31V, and A34V.

**Example 23:** <u>Libraries of Kunitz Domains</u>

Other Kunitz domains that can potently inhibit hNE may be derived from human Kunitz domains either by substituting hNE-inhibiting sequences into human domains or by using the methods of US 5,223,409 and related patents. Table 720 shows a gene that will cause display of human LACI-D2 on M13 gIIIp; essentially the same gene could be used to achieve display on M13 gVIIIp or other anchor proteins (such as bacterial outer-surface proteins (OSPs)). Table 725 shows a gene to cause display of human LACI D1.

Table 730 and Table 735 give variegations of LACI-D1 and LACI-D2 respectively. Each of these is divided into variegation of residues 10-21 in one segment and residues 31-42 in another. In each case, the appropriate vgDNA is introduced into a vector that displays the parental protein and the library of display phage are fractionated for binding to immobilized hNE.

Tables

**Table 30:** *IIIsp::bpti::mautreIII(initial fragment)* fusion gene. The DNA sequence has SEQ ID NO. 001; Amino-acid sequence has SEQ ID NO. 002. The DNA is linear and is shown on the lines that do not begin with "!". The DNA encoding mature III is identical to the DNA found in M13mp18. The amino-acid sequence is processed *in vivo* and disulfide bonds form.

```
!  SEQ ID NO. 002       m   k   k   l   l   f   a   I   p   l
!                       1   2   3   4   5   6   7   8   9  10
   SEQ ID NO. 001 5'-gtg aaa aaa tta tta ttc gca att cct tta
!                    |<---- gene III signal peptide --------
!
!                                           ┌ cleavage site
!                                           ↓
!        v   v   p   f   y   s   G   A
!       11  12  13  14  15  16  17  18
        gtt gtt cct ttc tat tct GGc Gcc
!       ---------------------------->|
!
!                         | R | P | D | F | C | L | E |
!                         | 19| 20| 21| 22| 23| 24| 25|
                          |CGT|CCG|GAT|TTC|TGT|CTC|GAG| -
! M13/BPTI Jnct   ↑     |AccIII|            |  XhoI  |  (& AvaI)!
!
! | P | P | Y | T | G | P | C | K | A | R |
! | 26| 27| 28| 29| 30| 31| 32| 33| 34| 35|
  |CCA|CCA|TAC|ACT|GGG|CCC|TGC|AAA|GCG|CGC| -
!     |   PflMI      |   |   |     | BssHII |
!                  | ApaI  |   |
!                  | DraII     |  = PssI
!
! | I | I | R | Y | F | Y | N | A | K | A |
! | 36| 37| 38| 39| 40| 41| 42| 43| 44| 45|
  |ATC|ATC|CGC|TAT|TTC|TAC|AAT|GCT|AAA|GC | -
!
! | G | L | C | Q | T | F | V | Y | G | G |
! | 46| 47| 48| 49| 50| 51| 52| 53| 54| 55|
 A|GGC|CTG|TGC|CAG|ACC|TTT|GTA|TAC|GGT|GGT| -
!| StuI |                 |  XcaI  |( & AccI)
!
! | C | R | A | K | R | N | N | F | K |
! | 56| 57| 58| 59| 60| 61| 62| 63| 64|
  |TGC|CGT|GCT|AAG|CGT|AAC|AAC|TTT|AAA| -
!         |  EspI   |
!
! | S | A | E | D | C | M | R | T | C | G |
! | 65| 66| 67| 68| 69| 70| 71| 72| 73| 74|
  |TCG|GCC|GAA|GAT|TGC|ATG|CGT|ACC|TGC|GGT| -
!   |XmaIII|       | SphI |
!
```

```
!           BPTI/M13 boundary
!                 ↓
! | G  | A  | A    E     (Residue numbers of mature III have had
! | 75 | 76 |119  120     118 added to the usual residue numbers.)
  |GGC |GCC |gct  gaa-
! | NarI    |  (& KasI)
!
! 121 122 123 124 125 126 127 128 129 130 131 132 133 134
!  T   V   E   S   C   L   A   K   P   H   T   E   N   S ...
  act gtt gaa agt tgt tta gca aaa ccc cat aca gaa aat tca...
!
```

! The remainder of the gene is idential to the corresponding part of *iii* in M13 mp18.

**Table 35:** *IIIsp::itiD1::matureIII* fusion gene.

DNA has SEQ ID NO. 003; amino-acid sequence has SEQ ID NO. 004.

The DNA is a linear segment and the amino-acid sequence is a protein that is processed *in vivo* and which contains disulfides.

```
   SEQ ID NO. 004
    m   k   k   l   l   f   a   I   p   l   v   v   p   f   y
   -18 -17 -16 -15 -14 -13 -12 -11 -10  -9  -8  -7  -6  -5  -4
5'-gtg aaa aaa tta tta ttc gca att cct tta gtt gtt cct ttc tat
   SEQ ID NO. 003
  |<---- gene III signal peptide ----------------------------

                 r cleavage site
    s   G   A   K   E   D   S   C   Q   L   G   Y   S   A   G
   -3  -2  -1   1   2   3   4   5   6   7   8   9  10  11  12
   tct GGc Gcc aaa gaa gaC tcT tGC CAG CTG GGC tac tCG GCC Ggt
    --------->|                 |  BglI        |     | EagI |
        | KasI |

   13  14  15  16  17  18  19  20  21  22  23  24  25  26
   P   C   M   G   M   T   S   R   Y   F   Y   N   G   T
  ccc tgc atg gga atg acc agc agg tat ttc tat aat ggt aca

   27  28  29  30  31  32  33  34  35  36  37  38  39  40  41
   S   M   A   C   E   T   F   Q   Y   G   G   C   M   G   N
  tCC ATG Gcc tgt gag act ttc cag tac ggc ggc tgc atg ggc aac
   | NcoI |
   | StyI |

   42  43  44  45  46  47  48  49  50  51  52  53  54  55  56
   G   N   N   F   V   T   E   K   E   C   L   Q   T   C   R
  ggt aac aac ttc gtc aca gaa aag gag tgt CTG CAG acc tgc cga
                                          | PstI  |

   57  58   101 102 119 120
   T   V     g   a   A   E
  act gtg   ggc gcc gct gaa
            | BbeI  |    (Residue numbers of mature
            | NarI  |    III have had 118 added to
            | KasI  |    the usual residue numbers.)

  121 122 123 124 125 126 127 128 129 130 131 132 133 134 135
   T   V   E   S   C   L   A   K   P   H   T   E   N   S   F ..
  act gtt gaa agt tgt tta gca aaa ccc cat aca gaa aat tca ttt..
```

The remainder of the gene is identical to the corresponding part of gene *iii* in phage M13mp18.

**Table 55:** Affinity Classes of ITI-D1-derived hNE inhibitors

| Affinity Class | Estimated $K_D$ | Fraction of Input bound | pH Elution Maximum | Protein |
|---|---|---|---|---|
| WEAK | $K_D$ > 10 nM | <0.005% | > 6.0 | ITI-D1 |

(continued)

| Affinity Class | Estimated $K_D$ | Fraction of Input bound | pH Elution Maximum | Protein |
|---|---|---|---|---|
| MODERATE | 1 to 10 nM | 0.01% to 0.03% | 5.5 to 5.0 | BITI<br>ITI-D1E7 |
| STRONG | 10 to 1000 pM | 0.03% to 0.06% | 5.0 to 4.5 | BITI-E7<br>BITI-E7-1222<br>AMINO1<br>AMINO2<br>MUTP1 |
| VERY STRONG | < 10 pM | > 0.1% | ≤ 4.0 | BITI-E7-141<br>MUTT26A<br>MUTQE<br>MUT1619 |

Table 65 : Definition of Class A, B and C mutations in PCT/US92/01501

| Classes : | | A | No major effect expected if molecular charge stays in range -1 to +1. |
|---|---|---|---|
| | | B | Major effects not expected, but are more likely than in "A". |
| | | C | Residue in the binding interface ; any change must be tested. |
| | | X | No substitution allowed. |

| Res. Id. | EpiNE1 | Substitutions | Class |
|---|---|---|---|
| 1 | R | any | A |
| 2 | P | any | A |
| 3 | D | any | A |
| 4 | F | Y, W, L | B |
| 5 | C | C | X |
| 6 | L | non-proline | A |
| 7 | E | L, S, T, D, N, K, R | A |
| 8 | P | any | A |
| 9 | P | any | A |
| 10 | Y | non-proline prefr'd | B |
| 11 | T | any | C |
| 12 | G | must be G | X |
| 13 | P | any | C |
| 14 | C | C strongly preferred, any non-proline | C |
| 15 | I | V, A | C |
| 16 | A | | C |
| 17 | F | L, I, M, Y, W, H, V | C |
| 18 | F | Y, W, H | C |
| 19 | P | any | C |
| 20 | R | non-proline prefr'd | C |
| 21 | Y | F & Y most prefr'd; W, I, L prefr' d; M, V allowed | C |
| 22 | F | Y & F most prefr'd; non-proline prefr'd Y, F | B |
| 23 | Y | Y & F strongly prefr'd F ,Y | B |
| 24 | N | non-proline prefr'd | A |
| 25 | A | any | A |

(continued)

| Res. Id. | EpiNE1 | Substitutions | Class |
|---|---|---|---|
| 26 | K | any | A |
| 27 | A | any | A |
| 28 | G | non-proline prefr'd | A |
| 29 | L | non-proline prefr'd | A |
| 30 | C | must be C | X |
| 31 | Q | non-proline prefr'd | B |
| 32 | T | non-proline prefr'd | B |
| 33 | F | F very strongly prefr'd; Y possible | X |
| 34 | V | any | C |
| 35 | Y | Y most prefr'd; W prefr'd; F allowed | B |
| 36 | G | G strongly prefr'd; S, A prefr'd; | C |
| 37 | G | must be G so long as 38 is C | X |
| 38 | C | C strongly prefr'd | X |
| 39 | M | any | C |
| 40 | G | A,S,N,D,T,P | C |
| 41 | N | K,Q,S,D,R,T,A,E | C |
| 42 | G | any | C |
| 43 | N | must be N | X |
| 44 | N | S, K, R, T, Q, D, E | B |
| 45 | F | Y | B |
| 46 | K | any non-proline | B |
| 47 | ST, N, A, G | | B |
| 48 | Aany | B | |
| 49 | E | any | A |
| 50 | D | any | A |
| 51 | C | must be C | X |
| 52 | M | any | A |
| 53 | R | any | A |
| 54 | T | any | A |
| 55 | C | must be C | X |
| 56 | G | any | A |
| 57 | G | any | A |
| 58 | A | any | A |

prefr'd stands for preferred.

Table 100: Sequences of Kunitz domains

| Name | Sequence | Parental domain | Seq Id No. |
|---|---|---|---|
| | 11111111112222222222333333333444 444444455555555<br>123456789a0123456789012345678901234567890 12ab345678901234 5678 | | |
| Consensus Kunitz Domain | RPDFCLLPA-ETGPCRAMIPRFYYNAKSGKCEPFIYGGCGGNA--NNFKTEEECRRTCGGA<br>1 3 5 7 9<br>2 4 6 8 10 | | 005 |
| BPTI (Genebank P00974) | RPDFCLEPP-YTGPCKARIIRYFYNAKAGLCQTFVYGGCRAKR--NNFKSAEDCMRTCGGA | BPTI | 006 |
| EPI-HNE-1 =EpiNE1 | rpdfclepp-ytgpcIaFFPryfynakaglcqtfvyggcMGNG--nnfksaedcmrtcgga | BPTI | 007 |
| EPI-HNE-2 | EAEArpdfclepp-ytgpcIaFFPryfynakaglcqtfvyggcMGNG--nnfksaedcmrtcgga | BPTI | 008 |
| EpiNE7 | rpdfclepp-ytgpcVaMFPryfynakaglcqtfvyggcMGNG--nnfksaedcmrtcgga | BPTI | 009 |
| EpiNE3 | rpdfclepp-ytgpcVGFFSryfynakaglcqtfvyggcMGNG--nnfksaedcmrtcgga | BPTI | 010 |
| EpiNE6 | rpdfclepp-ytgpcVGFFQryfynakaglcqtfvyggcMGNG--nnfksaedcmrtcgga | BPTI | 011 |

Table 100: Sequences of Kunitz domains

| Name | Sequence<br>1111111111222222222233333333333444 4444444555555555<br>123456789a0123456789012345678901234567890l2ab3456789012345678 | Parental domain | Seq Id No. |
|---|---|---|---|
| EpiNE4 | rpdfclepp-ytgpcVaIFPryfynakaglcqtfvyggcMGNG--nnfksaedcmrtcgga | BPTI | 012 |
| EpiNE8 | rpdfclepp-ytgpcVaFFKrsfynakaglcqtfvyggcMGNG--nnfksaedcmrtcgga | BPTI | 013 |
| EpiNE5 | rpdfclepp-ytgpcIaFFQryfynakaglcqtfvyggcMGNG--nnfksaedcmrtcgga | BPTI | 014 |
| EpiNE2 | rpdfclepp-ytgpcIaLFKryfynakaglcqtfvyggcMGNG--nnfksaedcmrtcgga | BPTI | 015 |
| ITI-D1 (Genebank P02760) | KEDSCQLGY-SAGPCMGMTSRYFYNGTSMACETFQYGGCMGNG--NNFVTEKDCLQTCRTV | ITI-D1 | 016 |
| BITI-E7-141 | RPdFcqlgy-sagpcVAmFPryfyngtsmacQtfVyggcmgng--nnfvtekdclqtcrga | ITI-D1 | 017 |
| MUTT26A | RPdFcqlgy-sagpcVAmFPryfyngAsmacQtfVyggcmgng--nnfvtekdclqtcrga | ITI-D1 | 018 |
| MUTQE | RPdFcqlgy-sagpcVAmFPryfyngtsmacetfVyggcmgng--nnfvtekdclqtcrga | ITI-D1 | 019 |
| MUT1619 | RPdFcqlgy-sagpcVgmFsryfyngtsmacQtfVyggcmgng--nnfvtekdclqtcrga | IDI-D1 | 020 |
| ITI-D1E7 | kedscqlgy-sagpcVAmFPryfyngtsmacetfqyggcmgng--nnfvtekdclqtcrga | ITI-D1 | 021 |

Table 100: Sequences of Kunitz domains

| Name | Sequence<br>1111111111222222222233333333333444 4444444555555555<br>123456789a012345678901234567890123456789012ab3456789012345678 | Parental domain | Seq Id No. |
|---|---|---|---|
| AMINO1 | kedFcqlgy-sagpcVAmFPryfyngtsmacetfqyggcmgng--nnfvtekdclqtcrga | ITI-D1 | 022 |
| AMINO2 | kPdscqlgy-sagpcVAmFPryfyngtsmacetfqyggcmgng--nnfvtekdclqtcrga | ITI-D1 | 023 |
| MUTP1 | RPdFcqlgy-sagpcIgmFsryfyngtsmacetfqyggcmgng--nnfvtekdclqtcrga | ITI-D1 | 024 |
| ITI-D2 (Genebank P02760) | TVAACNLPI-VRGPCRAFIQLWAFDAVKGKCVLFPYGGCQGNG--NKFYSEKECREYCGVP | ITI-D2 | 025 |
| EPI-HNE-3 | aacnlpi-vrgpcIafFPRwafdavkgkcvlfpyggcqgng--nkfysekecreycgvp | ITI-D2 | 026 |
| EPI-HNE-4 | Eacnlpi-vrgpcIafFPRwafdavkgkcvlfpyggcqgng--nkfysekecreycgvp | ITI-D2 | 027 |
| App-I (NCBI 105306) | VREVCSEQA-ETGPCRAMISRWYFDVTEGKCAPFFYGGCGGNR--NNFDTEEYCMAVCGSA | | 028 |
| DPI.1.1 | vrevcseqa-YtgpcIaFFPrYyfdvtegkcQTfVyggcMgnG--nnfdteeycmavcgsa | APP-I | 029 |
| DPI.1.2 | vrevcseqa-etgpcIamFsrwyfdvtegkcapfVyggcggnr--nnfdteeycmavcgsa | App-I | 030 |

Table 100: Sequences of Kunitz domains

| Name | Sequence<br>1111111111222222222233333333334444 4444444555555555<br>123456789a0123456789012345678901234567890l2ab345678901234567 8 | Parental domain | Seq Id No. |
|---|---|---|---|
| DPI.1.3 | vrevcseqa-etgpcIaFFsrwyfdvtegkcaTfVyggcMgnr--nnfdteeycmavcgsa | App-I | 031 |
| TFPI2-D1 (SPRE94) | NAEICLLPL-DYGPCRALLLRYYYDRYTQSCRQFLYGGCEGNA--NNFYTWEACDDACWRI | | 032 |
| DPI.2.1 | naeicllpl-YTgpcIaFFPryyydrytqscQTfVyggcMgna--nnfytweacddacwri | TFPI2-D1 | 033 |
| DPI.2.2 | naeicllpl-dygpcIalFlryyydrytqscrqfVyggcegna--nnfytweacddacwri | TFPI2-D1 | 034 |
| DPI.2.3 | naeicllpl-dTgpcIaFFlryyydrytqscQTfVyggcMgna--nnfytweacddacwri | TFPI2-D1 | 035 |
| TFPI2-D2 (SPRE94) | VPKVCRLQVSVDDQCEGSTEKYFFNLSSMTCEKFFSGGCHRNRIENRFPDEATCMGFCAPK | | 036 |
| DPI.3.1 | vpkvcrlqv-vRGPcIAFFPRWffnlssmtcVLfPYggcQGnG--nrfpdeatcmgfcapk | | 037 |
| DPI.3.2 | vpkvcrlqvsvddqcIgsFekyffnlAsmtceTfVsggchrnrienrfpdeatcmgfcapk | TFPI2-D2 | 038 |
| DPI.3.3 | vpkvcrlqv-vAGPcIgFFKRyffAlssmtceTfVsggchrnr--nrfpdeatcmgfcapk | TFPI2-D2 | 039 |
| TFPI2-D3 (SPRE94) | ipsfcyspk-deglcsanvtryyfnpryrtcdaftytgcggnd--nnfvsredckracaka | | 040 |
| DPI.4.1 | ipsfcyspk-SAgPcVaMFPryyfnpryrtcETfVyGgcMgnG--nnfvsredckracaka | TFPI2-D3 | 041 |

Table 100: Sequences of Kunitz domains

| Name | Sequence<br>111111111122222222223333333333444 4444444555555555<br>123456789a01234567890123456789012345678 9012ab3456789012345678 | Parental domain | Seq Id No. |
|---|---|---|---|
| DPI.4.2 | ipsfcyspk-deglcIaFFtryyfnpryrtcdaftytgcggnd--nnfvsredckracaka | TFPI2-D3 | 042 |
| DPI.4.3 | ipsfcyspk-dTgPcIaFFtryyfnpryrtcdTfVyGgcggnd--nnfvsredckracaka | TFPI2-D3 | 043 |
| LACI-D1 (Genebank P10646) | mhsfcafka-ddgpckaimkrfffniftrqceefiyggcegnq--nrfesleeckkmctrd | | 044 |
| DPI.5.1 | mhsfcafka-SAgpcVaMFPrYffniftrqceTfVyggcMgnG--nrfesleeckkmctrd | LACI-D1 | 045 |
| DPI.5.2 | mhsfcafka-ddgpcIaiFkrfffniftrqceefiyggcegnq--nrfesleeckkmctrd | LACI-D1 | 046 |
| DPI.5.3 | mhsfcafka-YTgpcIaFFkrfffniftrqceTfiyggcegnq--nrfesleeckkmctrd | LACI-D1 | 047 |
| LACI-D2 (Genebank P10646) | KPDFCFLEE-DPGICRGYITRYFYNNQTKQCERFKYGGCLGNM--NNFETLEECKNICEDG | | 048 |
| DPI.6.1 | kpdfcflee-SAgPcVAMFPryfynnqtkqceTfVyggcMgnG--nnfetleecknicedg | LACI-D2 | 049 |
| DPI.6.2 | kpdfcflee-dpgicVgyFtryfynnqtkqcerfkyggclgnm--nnfetleecknicedg | LACI-D2 | 050 |
| DPI.6.3 | kpdfcflee-dpgicVgFFtryfynnqtkqcerfVyggclgnm--nnfetleecknicedg | LACI-D2 | 051 |

| Table 100: Sequences of Kunitz domains | | | |
|---|---|---|---|
| Name | Sequence<br>1111111111222222222233333333333444  4444444555555555<br>123456789a0123456789012345678901234567890l2ab345678901234 5678 | Parental domain | Seq Id No. |
| DPI.6.4 | kpdfcflee-dpgicVgFFtryfynAqtkqcerfVyggclgnm--nnfetleeckniced g | LACI-D2 | 052 |
| DPI.6.5 | kpdfcflee-dpgPcVgFFQryfynAqtkqcerfVyggcQgnm--nnfetleecknicedg | LACI-D2 | 053 |
| DPI.6.6 | kpdfcflee-dpgPcVgFFtryfynnqtkqcerfVyggcQgnm--nnfetleecknicedg | LACI-D2 | 054 |
| DPI.6.7 | kpdfcflee-dpgPcIgFFPryfynnqtkqcerfVyggcQgnm--nnfetleecknicedg | LACI-D2 | 055 |
| LACI-D3 (Genebank P10646) | GPSWCLTPA-DRGLCRANENRFYYNSVIGKCRPFKYSGCGGNE--NNFTSKQECLRACKKG | | 056 |
| DPI.7.1 | gpswcltpa-VrgPcIaFFPrWyynsvigkcVLfPyGgcQgnG--nnftskqeclrackkg | LACI-D3 | 057 |
| DPI.7.2 | gpswcltpa-drglcVanFnrfyynsvigkcrpfkysgcggne--nnftskqeclrackkg | LACI-D3 | 058 |
| DPI.7.3 | gpswcltpa-drglcVaFFnrfyynsvigkcrpfkysgcggne--nnfKskqeclrackkg | LACI-D3 | 059 |
| DPI.7.4 | gpswcltpa-VrgPcVaFFnrfyynsvigkcrpfkyGgcggne--nnfKskqeclrackkg | LACI-D3 | 060 |
| DPI.7.5 | gpswcltpa-drgPcIaFFPrWyynsvigkcQTfVyGgcggne--nnfAskqeclrackkg | LACI-D3 | 061 |

Table 100: Sequences of Kunitz domains

| Name | Sequence<br>11111111112222222222333333333444 4444444555555555<br>123456789a012345678901234567890123456789012ab3456789012345678 | Parental domain | Seq Id No. |
|---|---|---|---|
| A3 collagen (WO93/ 14119) | ETDICKLPK-DEGTCRDFILKWYYDPNTKSCARFWYGGCGGNE--NKFGSQKECEKVCAPV | | 062 |
| DPI.8.1 | etdicklpk-VRgPcIAfFPRwyydpntkscVLfPyggcQgnG--nkfgsqkecekvcapv | A3 | 063 |
| DPI.8.2 | etdicklpk-degtcIAfFlkwyydpntkscarfVyggcggne--nkfgsqkecekvcapv | A3 collagen | 064 |
| DPI.8.3 | etdicklpk-degPcIAfFlRwyydpntkscarfVyggcggne--nkfgsqkecekvcapv | A3 | 065 |
| HKI B9 Domain (NORR93) | LPNVCAFPM-EKGPCQTYMTRWFFNFETGECELFAYGGCGGNS--NNFLRKEKCEKFCKFT | | 066 |
| DPI.9.1 | lpnvcafpm-VRgpcIAFFPrwffnfetgecVlfVyggcQgnG--nnflrkekcekfckft | HKI B9 | 067 |
| DPI.9.2 | lpnvcafpm-ekgpcIAyFtrwffnfetgecelfayggcggns--nnflrkekcekfckft | HKI B9 | 068 |
| DPI.9.3 | lpnvcafpm-ekgpcIAyFPrwffnfetgecVlfVyggcggns--nnflrkekcekfckft | HKI B9 | 069 |

Sequences listed in Table 100 that strongly inhibit hNE are EPI-HNE-1(=EpiNE1), EPI-HNE-2, EpiNE7, EpiNE3, EpiNE6, EpiNF4, EpiNE8, EpiNE5, EpiNE2, BITI-E7-141, MUTT26A, MUTQE, MUT1619, ITI-D1E7, AMINO1, AMINO2, MUTP1, and EPI-HNE-3, and EPI-HNE-4. Sequences listed in Table 100 that are highly likely to strongly inhibit hNE are DPI.1.1, DPI.1.2, DPI.1.3, DPL2.1, DPL2.2, DPI.2.3, DPI.3.1, DPI.3.2, DPI.3.3, DPI.4.1, DPI.4.2, DPI.4.3, DPI.5.1, DPI.5.2, DPI.5.3, DPI.6.1, DPI.6.2, DPI.6.3, DPI.6.4, DPI.6.5, DPI.6.6, DPI.6.7, DPI.7.1, DPI.7.2, DPI.7.3, DPI.7.4, DPI. 7.5, DPI.8.1, DPI.8.2, DPI.8.3, DPI.9.1, DPI.9.2, and DPI.9.3. Human Kunitz domains listed in Table 100: ITI-D1, ITI-D2, App-I, TFPI2-D1, TFPI2-D2, TFPI2-D3, LACI-D1, LACI-D2, LACI-D3, A3 collagen Kunitz domain, and HKI B9 Domain.

Table 111: Restriction sites in plasmid pHIL-D2

pHIL-D2, 93-01-02
Ngene = 8157

| <u>Non-cutters</u> | | | | | | |
|---|---|---|---|---|---|---|
| AflII | ApaI | AscI | AvaI | AvrII | BamHI | BglII |
| Bsp120I | BsrGI | BssHII | BstEII | FseI | MluI | NruI |
| PacI | PmlI | RsrII | SacII | SexAI | SfiI | SgfI |
| SnaBI | SpeI | Sse8387I | | XhoI(PaeR7I) | | XmaI(SmaI) |

| <u>Cutters</u> | | | | |
|---|---|---|---|---|
| AatII GACGTc | | 1 | 5498 | |
| AflIII Acrygt | | 1 | 7746 | |
| AgeI Accggt | | 1 | 1009 | |
| BlpI GCtnagc | | 1 | 597 | |
| BspEI(BspMII,AccIII) Tccgga | | 1 | 3551 | |
| BspMI gcaggt | | 1 | 4140 | |
| Bst1107I GTAtac | | 1 | 7975 | |
| BstBI(AsuII) TTcgaa | | 2 | 945 | 4780 |
| Bsu36I CCtnagg | | 1 | 1796 | |
| Ecl136I GAGctc | 1 | | 216 | |
| EcoRI Gaattc | 1 | | 956 | |
| EspI(Bpu1102I) GCtnagc | 1 | | 597 | |
| HpaI GTTaac | 1 | | 1845 | |
| NcoI Ccatgg | 1 | | 3339 | |
| NdeI CAtatg | 1 | | 7924 | |
| NsiI(Ppul0I) ATGCAt | 1 | | 684 | |
| PflMI CCANNNNntgg | 1 | | 196 | |
| PmeI GTTTaaac | 1 | | 420 | |
| PstI CTGCAg | 1 | | 6175 | |
| PvuI CGATcg | 1 | | 6049 | |
| SapI gaagagc | 1 | | 7863 | |
| SacI GAGCTc | 1 | | 216 | |
| Sail Gtcgac | 1 | | 2885 | |
| Seal AGTact | 1 | | 5938 | |
| SphI GCATGc | 1 | | 4436 | |
| StuI AGGcct | 1 | | 2968 | |
| SwaI ATTTaaat | 1 | | 6532 | |
| TthlllI GACNnngtc | 1 | | 7999 | |
| XbaI Tctaga | 1 | | 1741 | |
| XcmI CCANNNNNnnnntgg | 1 | | 711 | |

| | |
|---|---|
| Aox1 5' | 1 to about 950 |
| Aox1 3' | 950 to about 1250 |

(continued)

| Cutters | |
|---|---|
| His4 | 1700 to about 4200 |
| Aox1 3' | 4500 to 5400 |
| bla | 5600 to 6400 |
| f1 ori | 6500 to 6900 |

**TABLE 212**: Fractionation of EpiNE-7 and MA-ITI-D1 phage on hNE beads

| | | EpiNE-7 | | MA-ITI-D1 | |
|---|---|---|---|---|---|
| | | pfu | pfu/INPUT | pfu | pfu/INPUT |
| INPUT | | $3.3 \cdot 10^9$ | 1.00 | $3.4 \cdot 10^{11}$ | 1.00 |
| Final TBS-TWEEN Wash | | $3.8 \cdot 10^5$ | $1.2 \cdot 10^{-4}$ | $1.8 \cdot 10^6$ | $5.3 \cdot 10^{-6}$ |
| pH | 7.0 | $6.2 \cdot 10^5$ | $1.8 \cdot 10^{-4}$ | $1.6 \cdot 10^6$ | $4.7 \cdot 10^{-6}$ |
| | 6.0 | $1.4 \cdot 10^6$ | $4.1 \cdot 10^{-4}$ | $1.0 \cdot 10^6$ | $2.9 \cdot 10^{-6}$ |
| | 5.5 | $9.4 \cdot 10^5$ | $2.8 \cdot 10^{-4}$ | $1.6 \cdot 10^6$ | $4.7 \cdot 10^{-6}$ |
| | 5.0 | $9.5 \cdot 10^5$ | $2.9 \cdot 10^{-4}$ | $3.1 \cdot 10^5$ | $9.1 \cdot 10^{-7}$ |
| | 4.5 | $1.2 \cdot 10^6$ | $3.5 \cdot 10^{-4}$ | $1.2 \cdot 10^5$ | $3.5 \cdot 10^{-7}$ |
| | 4.0 | $1.6 \cdot 10^6$ | $4.8 \cdot 10^{-4}$ | $7.2 \cdot 10^4$ | $2.1 \cdot 10^{-7}$ |
| | 3.5 | $9.5 \cdot 10^5$ | $2.9 \cdot 10^{-4}$ | $4.9 \cdot 10^4$ | $1.4 \cdot 10^{-7}$ |
| | 3.0 | $6.6 \cdot 10^5$ | $2.0 \cdot 10^{-4}$ | $2.9 \cdot 10^4$ | $8.5 \cdot 10^{-8}$ |
| | 2.5 | $1.6 \cdot 10^5$ | $4.8 \cdot 10^{-5}$ | $1.4 \cdot 10^4$ | $4.1 \cdot 10^{-8}$ |
| | 2.0 | $3.0 \cdot 10^5$ | $9.1 \cdot 10^{-5}$ | $1.7 \cdot 10^4$ | $5.0 \cdot 10^{-8}$ |
| SUM | | $6.4 \cdot 10^6$ | $3 \cdot 10^{-3}$ | $5.7 \cdot 10^6$ | $2 \cdot 10^{-5}$ |
| * SUM is the total pfu (or fraction of input) obtained from all pH elution fractions | | | | | |

**TABLE 214**: Abbreviated fractionation of display phage on hNE beads

| | Display phage | | | |
|---|---|---|---|---|
| | EpiNE-7 | MA-ITI-D1 2 | MA-ITI-D1E7 1 | MA-ITI-D1E7 2 |
| INPUT (pfu) | 1.00 ($1.8 \times 10^9$) | 1.00 ($1.2 \times 10^{10}$ | 1.00 ($3.3 \times 10^9$) | 1.00 ($1.1 \times 10^9$) |
| Wash | $6 \cdot 10^{-5}$ | $1 \cdot 10^{-5}$ | $2 \cdot 10^{-5}$ | $2 \cdot 10^{-5}$ |
| pH 7.0 | $3 \cdot 10^{-4}$ | $1 \cdot 10^{-5}$ | $2 \cdot 10^{-5}$ | $4 \cdot 10^{-5}$ |
| pH 3.5 | $3 \cdot 10^{-3}$ | $3 \cdot 10^{-6}$ | $8 \cdot 10^{-5}$ | $8 \cdot 10^{-5}$ |
| pH 2.0 | $1 \cdot 10^{-3}$ | $1 \cdot 10^{-6}$ | $6 \cdot 10^{-6}$ | $2 \cdot 10^{-5}$ |
| SUM | $4.3 \cdot 10^{-3}$ | $1.4 \cdot 10^{-5}$ | $1.1 \cdot 10^{-4}$ | $1.4 \cdot 10^{-4}$ |

Each entry is the fraction of input obtained in that component.
SUM is the total fraction of input pfu obtained from all pH elution fractions

**TABLE 215**: Fractionation of EpiNE-7 and MA-ITI-D1E7 phage on hNE beads

| | EpiNE-7 | | MA-ITI-D1E7 | |
|---|---|---|---|---|
| | Total pfu | Fraction of Input | Total pfu | Fraction of Input |
| INPUT | $1.8 \cdot 10^9$ | 1.00 | $3.0 \cdot 10^9$ | 1.00 |
| pH 7.0 | $5.2 \cdot 10^5$ | $2.9 \cdot 10^{-4}$ | $6.4 \cdot 10^4$ | $2.1 \cdot 10^{-5}$ |
| pH 6.0 | $6.4 \cdot 10^5$ | $3.6 \cdot 10^{-4}$ | $4.5 \cdot 10^4$ | $1.5 \cdot 10^{-5}$ |
| pH 5.5 | $7.8 \cdot 10^5$ | $4.3 \cdot 10^{-4}$ | $5.0 \cdot 10^4$ | $1.7 \cdot 10^{-5}$ |
| pH 5.0 | $8.4 \cdot 10^5$ | $4.7 \cdot 10^{-4}$ | $5.2 \cdot 10^4$ | $1.7 \cdot 10^{-5}$ |
| pH 4.5 | $1.1 \cdot 10^6$ | $6.1 \cdot 10^{-4}$ | $4.4 \cdot 10^4$ | $1.5 \cdot 10^{-5}$ |
| pH 4.0 | $1.7 \cdot 10^6$ | $9.4 \cdot 10^{-4}$ | $2.6 \cdot 10^4$ | $8.7 \cdot 10^{-6}$ |
| pH 3.5 | $1.1 \cdot 10^6$ | $6.1 \cdot 10^{-4}$ | $1.3 \cdot 10^4$ | $4.3 \cdot 10^{-6}$ |
| pH 3.0 | $3.8 \cdot 10^5$ | $2.1 \cdot 10^{-4}$ | $5.6 \cdot 10^3$ | $1.9 \cdot 10^{-6}$ |
| pH 2.5 | $2.8 \cdot 10^5$ | $1.6 \cdot 10^{-4}$ | $4.9 \cdot 10^3$ | $1.6 \cdot 10^{-6}$ |
| pH 2.0 | $2.9 \cdot 10^5$ | $1.6 \cdot 10^{-4}$ | $2.2 \cdot 10^3$ | $7.3 \cdot 10^{-7}$ |
| SUM | $7.6 \cdot 10^6$ | $4.1 \cdot 10^{-3}$ | $3.1 \cdot 10^5$ | $1.1 \cdot 10^{-4}$ |

* SUM is the total pfu (or fraction of input) obtained from all pH elution fractions.

**TABLE 216:** Fractionation of MA-EpiNE-7, MA-BITI and MA-BITI-E7 on hNE beads

| | | MA-BITI | | MA-BITI-E7 | | MA-EpiNE7 | |
|---|---|---|---|---|---|---|---|
| | | pfu | pfu/Input | pfu | pfu/Input | pfu | pfu/Input |
| INPUT | | $2.0 \cdot 10^{10}$ | 1.00 | $6.0 \cdot 10^{9}$ | 1.00 | $1.5 \cdot 10^{9}$ | 1.00 |
| pH | 7.0 | $2.4 \cdot 10^{5}$ | $1.2 \cdot 10^{-5}$ | $2.8 \cdot 10^{5}$ | $4.7 \cdot 10^{-5}$ | $2.9 \cdot 10^{5}$ | $1.9 \cdot 10^{-4}$ |
| | 6.0 | $2.5 \cdot 10^{5}$ | $1.2 \cdot 10^{-5}$ | $2.8 \cdot 10^{5}$ | $4.7 \cdot 10^{-5}$ | $3.7 \cdot 10^{5}$ | $2.5 \cdot 10^{-4}$ |
| | 5.0 | $9.6 \cdot 10^{4}$ | $4.8 \cdot 10^{-6}$ | $3.7 \cdot 10^{5}$ | $6.2 \cdot 10^{-5}$ | $4.9 \cdot 10^{5}$ | $3.3 \cdot 10^{-4}$ |
| | 4.5 | $4.4 \cdot 10^{4}$ | $2.2 \cdot 10^{-6}$ | $3.8 \cdot 10^{5}$ | $6.3 \cdot 10^{-5}$ | $6.0 \cdot 10^{5}$ | $4.0 \cdot 10^{-4}$ |
| | 4.0 | $3.1 \cdot 10^{4}$ | $1.6 \cdot 10^{-6}$ | $2.4 \cdot 10^{5}$ | $4.0 \cdot 10^{-5}$ | $6.4 \cdot 10^{5}$ | $4.3 \cdot 10^{-4}$ |
| | 3.5 | $8.6 \cdot 10^{4}$ | $4.3 \cdot 10^{-6}$ | $9.0 \cdot 10^{4}$ | $1.5 \cdot 10^{-5}$ | $5.0 \cdot 10^{5}$ | $3.3 \cdot 10^{-4}$ |
| | 3.0 | $2.2 \cdot 10^{4}$ | $1.1 \cdot 10^{-6}$ | $8.9 \cdot 10^{4}$ | $1.5 \cdot 10^{-5}$ | $1.9 \cdot 10^{5}$ | $1.3 \cdot 10^{-4}$ |
| | 2.5 | $2.2 \cdot 10^{4}$ | $1.1 \cdot 10^{-6}$ | $2.3 \cdot 10^{4}$ | $3.8 \cdot 10^{-6}$ | $7.7 \cdot 10^{4}$ | $5.1 \cdot 10^{-5}$ |
| | 2.0 | $7.7 \cdot 10^{3}$ | $3.8 \cdot 10^{-7}$ | $8.7 \cdot 10^{3}$ | $1.4 \cdot 10^{-6}$ | $9.7 \cdot 10^{4}$ | $6.5 \cdot 10^{-5}$ |
| SUM | | $8.0 \cdot 10^{5}$ | $3.9 \cdot 10^{-5}$ | $1.8 \cdot 10^{6}$ | $2.9 \cdot 10^{-4}$ | $3.3 \cdot 10^{6}$ | $2.2 \cdot 10^{-3}$ |

* SUM is the total pfu (or fraction of input) obtained from all pH elution fractions

**TABLE 217**: Fractionation of MA-BITI-E7 and MA-BITI-E7-1222 on hNE beads

| | | MA-BITI-E7 | | MA-BITI-E7-1222 | |
|---|---|---|---|---|---|
| | | pfu | pfu/INPUT | pfu | pfu/INPUT |
| INPUT | | $1.3 \cdot 10^9$ | 1.00 | $1.2 \cdot 10^9$ | 1.00 |
| pH | 7.0 | $4.7 \cdot 10^4$ | $3.6 \cdot 10^{-5}$ | $4.0 \cdot 10^4$ | $3.3 \cdot 10^{-5}$ |
| | 6.0 | $5.3 \cdot 10^4$ | $4.1 \cdot 10^{-5}$ | $5.5 \cdot 10^4$ | $4.6 \cdot 10^{-5}$ |
| | 5.5 | $7.1 \cdot 10^4$ | $5.5 \cdot 10^{-5}$ | $5.4 \cdot 10^4$ | $4.5 \cdot 10^{-5}$ |
| | 5.0 | $9.0 \cdot 10^4$ | $6.9 \cdot 10^{-5}$ | $6.7 \cdot 10^4$ | $5.6 \cdot 10^{-5}$ |
| | 4.5 | $6.2 \cdot 10^4$ | $4.8 \cdot 10^{-5}$ | $6.7 \cdot 10^4$ | $5.6 \cdot 10^{-5}$ |
| | 4.0 | $3.4 \cdot 10^4$ | $2.6 \cdot 10^{-5}$ | $2.7 \cdot 10^4$ | $2.2 \cdot 10^{-5}$ |
| | 3.5 | $1.8 \cdot 10^4$ | $1.4 \cdot 10^{-5}$ | $2.3 \cdot 10^4$ | $1.9 \cdot 10^{-5}$ |
| | 3.0 | $2.5 \cdot 10^3$ | $1.9 \cdot 10^{-6}$ | $6.3 \cdot 10^3$ | $5.2 \cdot 10^{-6}$ |
| | 2.5 | $< 1.3 \cdot 10^3$ | $< 1.0 \cdot 10^{-6}$ | $< 1.3 \cdot 10^3$ | $< 1.0 \cdot 10^{-6}$ |
| | 2.0 | $1.3 \cdot 10^3$ | $1.0 \cdot 10^{-6}$ | $1.3 \cdot 10^3$ | $1.0 \cdot 10^{-6}$ |
| SUM | | $3.8 \cdot 10^5$ | $2.9 \cdot 10^{-4}$ | $3.4 \cdot 10^5$ | $2.8 \cdot 10^{-4}$ |
| SUM is the total pfu (or fraction of input) obtained from all pH elution fractions | | | | | |

**TABLE 218**: Fractionation of MA-EpiNE7 and MA-BITI-E7-141 on hNE beads

| | | MA-EpiNE7 | | MA-BITI-E7-141 | |
|---|---|---|---|---|---|
| | | pfu | pfu/INPUT | pfu | pfu/INPUT |
| INPUT | | $6.1 \cdot 10^8$ | 1.00 | $2.0 \cdot 10^9$ | 1.00 |
| pH | 7.0 | $5.3 \cdot 10^4$ | $8.7 \cdot 10^{-5}$ | $4.5 \cdot 10^5$ | $2.2 \cdot 10^{-4}$ |
| | 6.0 | $9.7 \cdot 10^4$ | $1.6 \cdot 10^{-4}$ | $4.4 \cdot 10^5$ | $2.2 \cdot 10^{-4}$ |
| | 5.5 | $1.1 \cdot 10^5$ | $1.8 \cdot 10^{-4}$ | $4.4 \cdot 10^5$ | $2.2 \cdot 10^{-4}$ |
| | 5.0 | $1.4 \cdot 10^5$ | $2.3 \cdot 10^{-4}$ | $7.2 \cdot 10^5$ | $3.6 \cdot 10^{-4}$ |
| | 4.5 | $1.0 \cdot 10^5$ | $1.6 \cdot 10^{-4}$ | $1.3 \cdot 10^6$ | $6.5 \cdot 10^{-4}$ |
| | 4.0 | $2.0 \cdot 10^5$ | $3.3 \cdot 10^{-4}$ | $1.1 \cdot 10^6$ | $5.5 \cdot 10^{-4}$ |
| | 3.5 | $9.7 \cdot 10^4$ | $1.6 \cdot 10^{-4}$ | $5.9 \cdot 10^5$ | $3.0 \cdot 10^{-4}$ |
| | 3.0 | $3.8 \cdot 10^4$ | $6.2 \cdot 10^{-5}$ | $2.3 \cdot 10^5$ | $1.2 \cdot 10^{-4}$ |
| | 2.5 | $1.3 \cdot 10^4$ | $2.1 \cdot 10^{-5}$ | $1.2 \cdot 10^5$ | $6.0 \cdot 10^{-5}$ |
| | 2.0 | $1.6 \cdot 10^4$ | $2.6 \cdot 10^{-5}$ | $1.0 \cdot 10^5$ | $5.0 \cdot 10^{-5}$ |
| SUM | | $8.6 \cdot 10^5$ | $1.4 \cdot 10^{-3}$ | $5.5 \cdot 10^6$ | $2.8 \cdot 10^{-3}$ |
| SUM is the total pfu (or fraction of input) obtained from all pH elution fractions. | | | | | |

**TABLE 219:** pH Elution Analysis of hNE Binding by BITI-E7-141 Varient Display Phage

| Displayed protein | Input | Fraction of Input recovered at pH | | | Recovery | |
|---|---|---|---|---|---|---|
| | PFU ($x10^9$) | pH7.0 | pH3.5 $x10^{-4}$ | pH2.0 $x10^{-4}$ | Total $x10^{-4}$ | Relative |
| AMINO1 (EE) | 0.96 | 0.24 | 2.3 | 0.35 | 2.9 | 0.11 |
| AMINO2 (AE) | 6.1 | 0.57 | 2.1 | 0.45 | 3.1 | 0.12 |

(continued)

| Displayed protein | Input | Fraction of Input recovered at pH | | | Recovery | |
|---|---|---|---|---|---|---|
| | PFU ($x10^9$) | pH7.0 | pH3.5 $x10^{-4}$ | pH2.0 $x10^{-4}$ | Total $x10^{-4}$ | Relative |
| BITI-E7-1222 (EE) | 1.2 | 0.72 | 4.0 | 0.64 | 5.4 | 0.21 |
| EpiNE7 (EE) | 0.72 | 0.44 | 6.4 | 2.2 | 9.0 | 0.35 |
| MUTP1 (AE) | 3.9 | 1.8 | 9.2 | 1.2 | 12.0 | 0.46 |
| MUT1619 (EE) | 0.78 | 0.82 | 9.9 | 0.84 | 12.0 | 0.46 |
| MUTQE (AE) | 4.7 | 1.2 | 16. | 5.3 | 22.0 | 0.85 |
| MUTT26A (EE) | 0.51 | 2.5 | 19.0 | 3.3 | 25.0 | 0.96 |
| BITI-E7-141 (AE) | 1.7 | 2.2 | 18.0 | 5.4 | 26.0 | 1.00 |
| BITI-E7-141 (EE) | 0.75 | 2.1 | 21. | 3.2 | 26.0 | 1.00 |

Notes:
EE Extended pH elution protocol
AE Abbreviated pH elution protocol
Total Total fraction of input = Sum of fractions collected at pH 7.0, pH 3.5, and pH 2.0.
Relative Total fraction of input recovered divided by total fraction of input recovered for BITI-E7-141

**Table 250**: Plasmid pHIL-D2 SEQ ID NO. 070
8157 base pairs. Only one strand is shown, but the DNA exists as double-stranded circular DNA *in vivo*.

```
              1          2          3          4          5
     1234567890 1234567890 1234567890 1234567890 1234567890
   1 AgATCgCggC CgCgATCTAA CATCCAAAgA CgAAAggTTg AATgAAACCT
  51 TTTTgCCATC CgACATCCAC AggTCCATTC TCACACATAA gTgCCAAACg
 101 CAACAggAgg ggATACACTA gCAgCAgACC gTTgCAAACg CAggACCTCC
 151 ACTCCTCTTC TCCTCAACAC CCACTTTTgC CATCgAAAAA CCAgCCCAgT
 201 TATTgggCTT gATTggAgCT CgCTCATTCC AATTCCTTCT ATTAggCTAC
 251 TAACACCATg ACTTTATTAg CCTgTCTATC CTggCCCCCC TggCgAggTC
 301 ATgTTTgTTT ATTTCCgAAT gCAACAAgCT CCgCATTACA CCCgAACATC
 351 ACTCCAgATg AgggCTTTCT gAgTgTgggg TCAAATAgTT TCATgTTCCC
 401 AAATggCCCA AAACTgACAg TTTAAACgCT gTCTTggAAC CTAATATgAC
 451 AAAAgCgTgA TCTCATCCAA gATgAACTAA gTTTggTTCg TTgAAATgCT
 501 AACggCCAgT TggTCAAAAA gAAACTTCCA AAAgTCgCCA TACCgTTTgT
 551 CTTgTTTggT ATTgATTgAC gAATgCTCAA AAATAATCTC ATTAATgCTT
 601 AgCgCAgTCT CTCTATCgCT TCTgAACCCg gTggCACCTg TgCCgAAACg
 651 CAAATggggA AACAACCCgC TTTTTggATg ATTATgCATT gTCCTCCACA
 701 TTgTATgCTT CCAAgATTCT ggTgggAATA CTgCTgATAg CCTAACgTTC
 751 ATgATCAAAA TTTAACTgTT CTAACCCCTA CTTgACAggC AATATATAAA
 801 CAgAAggAAg CTgCCCTgTC TTAAACCTTT TTTTTTATCA TCATTATTAg
 851 CTTACTTTCA TAATTgCgAC TggTTCCAAT TgACAAgCTT TTgATTTTAA
 901 CgACTTTTAA CgACAACTTg AgAAgATCAA AAAACAACTA ATTATTCgAA
                                                  BstBI
 951 ACgAggAATT CgCCTTAgAC ATgACTgTTC CTCAgTTCAA gTTgggCATT
           EcoRI
1001 ACgAgAAgAC CggTCTTgCT AgATTCTAAT CAAgAggATg TCAgAATgCC
1051 ATTTgCCTgA gAgATgCAgg CTTCATTTTT gATACTTTTT TATTTgTAAC
1101 CTATATAgTA TAggATTTTT TTTgTCATTT TgTTTCTTCT CgTACgAgCT
1151 TgCTCCTgAT CAgCCTATCT CgCAgCTgAT gAATATCTTg TggTAggggT
1201 TTgggAAAAT CATTCgAgTT TgATgTTTTT CTTggTATTT CCCACTCCTC
1251 TTCAgAgTAC AgAAgATTAA gTgAgAAgTT CgTTTgTgCA AgCTTATCgA
1301 TAAgCTTTAA TgCggTAgTT TATCACAgTT AAATTgCTAA CgCAgTCAgg
1351 CACCgTgTAT gAAATCTAAC AATgCgCTCA TCgTCATCCT CggCACCgTC
1401 ACCCTggATg CTgTAggCAT AggCTTggTT ATgCCggTAC TgCCgggCCT
1451 CTTgCgggAT ATCgTCCATT CCgACAgCAT CgCCAgTCAC TATggCgTgC
1501 TgCTAgCgCT ATATgCgTTg ATgCAATTTC TATgCgCACC CgTTCTCggA
```

Table 250, continued

1551 gCACTgTCCg ACCgCTTTgg CCgCCgCCCA gTCCTgCTCg CTTCgCTACT
1601 TggAgCCACT ATCgACTACg CgATCATggC gACCACACCC gTCCTgTggA
1651 TCTATCgAAT CTAAATgTAA gTTAAAATCT CTAAATAATT AAATAAgTCC
1701 CAgTTTCTCC ATACgAACCT TAACAgCATT gCggTgAgCA TCTAgACCTT
1751 CAACAgCAgC CAgATCCATC ACTgCTTggC CAATATgTTT CAgTCCCTCA
1801 ggAgTTACgT CTTgTgAAgT gATgAACTTC TggAAggTTg CAgTgTTAAC
1851 TCCgCTgTAT TgACgggCAT ATCCgTACgT TggCAAAgTg TggTTggTAC
1901 CggAggAgTA ATCTCCACAA CTCTCTggAg AgTAggCACC AACAAACACA
1951 gATCCAgCgT gTTgTACTTg ATCAACATAA gAAgAAgCAT TCTCgATTTg
2001 CAggATCAAg TgTTCAggAg CgTACTgATT ggACATTTCC AAAgCCTgCT
2051 CgTAggTTgC AACCgATAgg gTTgTAgAgT gTgCAATACA CTTgCgTACA
2101 ATTTCAACCC TTggCAACTg CACAgCTTgg TTgTgAACAg CATCTTCAAT
2151 TCTggCAAgC TCCTTgTCTg TCATATCgAC AgCCAACAgA ATCACCTggg
2201 AATCAATACC ATgTTCAgCT TgAgCAgAAg gTCTgAggCA ACgAAATCTg
2251 gATCAgCgTA TTTATCAgCA ATAACTAgAA CTTCAgAAgg CCCAgCAggC
2301 ATgTCAATAC TACACAgggC TgATgTgTCA TTTTgAACCA TCATCTTggC
2351 AgCAgTAACg AACTggTTTC CTggACCAAA TATTTTgTCA CACTTAggAA
2401 CAgTTTCTgT TCCgTAAgCC ATAgCAgCTA CTgCCTgggC gCCTCCTgCT
2451 AgCACgATAC ACTTAgCACC AACCTTgTgg gCAACgTAgA TgACTTCTgg
2501 ggTAAgggTA CCATCCTTCT TAggTggAgA TgCAAAAACA ATTTCTTTgC
2551 AACCAgCAAC TTTggCAggA ACACCCAgCA TCAgggAAgT ggAAggCAgA
2601 ATTgCggTTC CACCAggAAT ATAgAggCCA ACTTTCTCAA TAggTCTTgC
2651 AAAACgAgAg CAgACTACAC CAgggCAAgT CTCAACTTgC AACgTCTCCg
2701 TTAgTTgAgC TTCATggAAT TTCCTgACgT TATCTATAgA gAgATCAATg
2751 gCTCTCTTAA CgTTATCTgg CAATTgCATA AgTTCCTCTg ggAAAggAgC
2801 TTCTAACACA ggTgTCTTCA AAgCgACTCC ATCAAACTTg gCAgTTAgTT
2851 CTAAAAgggC TTTgTCACCA TTTTgACgAA CATTgTCgAC AATTggTTTg
2901 ACTAATTCCA TAATCTgTTC CgTTTTCTgg ATAggACgAC gAAgggCATC
2951 TTCAATTTCT TgTgAggAgg CCTTAgAAAC gTCAATTTTg CACAATTCAA
3001 TACgACCTTC AgAAgggACT TCTTTAggTT TggATTCTTC TTTAggTTgT
3051 TCCTTggTgT ATCCTggCTT ggCATCTCCT TTCCTTCTAg TgACCTTTAg
3101 ggACTTCATA TCCAggTTTC TCTCCACCTC gTCCAACgTC ACACCgTACT
3151 TggCACATCT AACTAATgCA AAATAAAATA AgTCAgCACA TTCCCAggCT
3201 ATATCTTCCT TggATTTAgC TTCTgCAAgT TCATCAgCTT CCTCCCTAAT
3251 TTTAgCgTTC AACAAAACTT CgTCgTCAAA TAACCgTTTg gTATAAgAAC
3301 CTTCTggAgC ATTgCTCTTA CgATCCCACA AggTgCTTCC ATggCTCTAA
3351 gACCCTTTgA TTggCCAAAA CAggAAgTgC gTTCCAAgTg ACAgAAACCA
3401 ACACCTgTTT gTTCAACCAC AAATTTCAAg CAgTCTCCAT CACAATCCAA

Table 250, continued

| | | | | | |
|---|---|---|---|---|---|
| 3451 | TTCgATACCC | AgCAACTTTT | gAgTTCgTCC | AgATgTAgCA | CCTTTATACC |
| 3501 | ACAAACCgTg | ACgACgAgAT | TggTAgACTC | CAgTTTgTgT | CCTTATAgCC |
| 3551 | TCCggAATAg | ACTTTTTggA | CgAgTACACC | AggCCCAACg | AgTAATTAgA |
| 3601 | AgAgTCAgCC | ACCAAAgTAg | TgAATAgACC | ATCggggCgg | TCAgTAgTCA |
| 3651 | AAgACgCCAA | CAAAATTTCA | CTgACAgggA | ACTTTTTgAC | ATCTTCAgAA |
| 3701 | AgTTCgTATT | CAgTAgTCAA | TTgCCgAgCA | TCAATAATgg | ggATTATACC |
| 3751 | AgAAgCAACA | gTggAAgTCA | CATCTACCAA | CTTTgCggTC | TCAgAAAAAg |
| 3801 | CATAAACAgT | TCTACTACCg | CCATTAgTgA | AACTTTTCAA | ATCgCCCAgT |
| 3851 | ggAgAAgAAA | AAggCACAgC | gATACTAgCA | TTAgCgggCA | AggATgCAAC |
| 3901 | TTTATCAACC | AgggTCCTAT | AgATAACCCT | AgCgCCTggg | ATCATCCTTT |
| 3951 | ggACAACTCT | TTCTgCCAAA | TCTAggTCCA | AAATCACTTC | ATTgATACCA |
| 4001 | TTATACggAT | gACTCAACTT | gCACATTAAC | TTgAAgCTCA | gTCgATTgAg |
| 4051 | TgAACTTgAT | CAggTTgTgC | AgCTggTCAg | CAgCATAggg | AAACACggCT |
| 4101 | TTTCCTACCA | AACTCAAggA | ATTATCAAAC | TCTgCAACAC | TTgCgTATgC |
| 4151 | AggTAgCAAg | ggAAATgTCA | TACTTgAAgT | CggACAgTgA | gTgTAgTCTT |
| 4201 | gAgAAATTCT | gAAgCCgTAT | TTTTATTATC | AgTgAgTCAg | TCATCAggAg |
| 4251 | ATCCTCTACg | CCggACgCAT | CgTggCCggC | ATCACCggCg | CCACAggTgC |
| 4301 | ggTTgCTggC | gCCTATATCg | CCgACATCAC | CgATggggAA | gATCgggCTC |
| 4351 | gCCACTTCgg | gCTCATgAgC | gCTTgTTTCg | gCgTgggTAT | ggTggCAggC |
| 4401 | CCCgTggCCg | ggggACTgTT | gggCgCCATC | TCCTTgCATg | CACCATTCCT |
| 4451 | TgCggCggCg | gTgCTCAACg | gCCTCAACCT | ACTACTgggC | TgCTTCCTAA |
| 4501 | TgCAggAgTC | gCATAAgggA | gAgCgTCgAg | TATCTATgAT | TggAAgTATg |
| 4551 | ggAATggTgA | TACCCgCATT | CTTCAgTgTC | TTgAggTCTC | CTATCAgATT |
| 4601 | ATgCCCAACT | AAAgCAACCg | gAggAggAgA | TTTCATggTA | AATTTCTCTg |
| 4651 | ACTTTTggTC | ATCAgTAgAC | TCgAACTgTg | AgACTATCTC | ggTTATgACA |
| 4701 | gCAgAAATgT | CCTTCTTggA | gACAgTAAAT | gAAgTCCCAC | CAATAAAgAA |
| 4751 | ATCCTTgTTA | TCAggAACAA | ACTTCTTgTT | TCgAACTTTT | TCggTgCCTT |
| 4801 | gAACTATAAA | ATgTAgAgTg | gATATgTCgg | gTAggAATgg | AgCgggCAAA |
| 4851 | TgCTTACCTT | CTggACCTTC | AAgAggTATg | TAgggTTTgT | AgATACTgAT |
| 4901 | gCCAACTTCA | gTgACAACgT | TgCTATTTCg | TTCAAACCAT | TCCgAATCCA |
| 4951 | gAgAAATCAA | AgTTgTTTgT | CTACTATTgA | TCCAAgCCAg | TgCggTCTTg |
| 5001 | AAACTgACAA | TAgTgTgCTC | gTgTTTTgAg | gTCATCTTTg | TATgAATAAA |
| 5051 | TCTAgTCTTT | gATCTAAATA | ATCTTgACgA | gCCAAggCgA | TAAATACCCA |
| 5101 | AATCTAAAAC | TCTTTTAAAA | CgTTAAAAgg | ACAAgTATgT | CTgCCTgTAT |
| 5151 | TAAACCCCAA | ATCAgCTCgT | AgTCTgATCC | TCATCAACTT | gAggggCACT |
| 5201 | ATCTTgTTTT | AgAgAAATTT | gCggAgATgC | gATATCgAgA | AAAAggTACg |
| 5251 | CTgATTTTAA | ACgTgAAATT | TATCTCAAgA | TCgCggCCgC | gATCTCgAAT |
| 5301 | AATAACTgTT | ATTTTTCAgT | gTTCCCgATC | TgCgTCTATT | TCACAATACC |

Table 250, continued

5351 AACATgAgTC AgCTTATCgA TgATAAgCTg TCAAACATgA gAATTAATTC
5401 gATgATAAgC TgTCAAACAT gAgAAATCTT gAAgACgAAA gggCCTCgTg
5451 ATACgCCTAT TTTTATAggT TAATgTCATg ATAATAATgg TTTCTTAgAC
5501 gTCAggTggC ACTTTTCggg gAAATgTgCg CggAACCCCT ATTTgTTTAT
5551 TTTTCTAAAT ACATTCAAAT ATgTATCCgC TCATgAgACA ATAACCCTgA
5601 TAAATgCTTC AATAATATTg AAAAAggAAg AgTATgAgTA TTCAACATTT
5651 CCgTgTCgCC CTTATTCCCT TTTTTgCggC ATTTTgCCTT CCTgTTTTTg
5701 CTCACCCAgA AACgCTggTg AAAgTAAAAg ATgCTgAAgA TCAgTTgggT
5751 gCACgAgTgg gTTACATCgA ACTggATCTC AACAgCggTA AgATCCTTgA
5801 gAgTTTTCgC CCCgAAgAAC gTTTTCCAAT gATgAgCACT TTTAAAgTTC
5851 TgCTATgTgg CgCggTATTA TCCCgTgTTg ACgCCgggCA AgAgCAACTC
5901 ggTCgCCgCA TACACTATTC TCAgAATgAC TTggTTgAgT ACTCACCAgT
5951 CACAgAAAAg CATCTTACgg ATggCATgAC AgTAAgAgAA TTATgCAgTg
6001 CTgCCATAAC CATgAgTgAT AACACTgCgg CCAACTTACT TCTgACAACg
6051 ATCggAggAC CgAAggAgCT AACCgCTTTT TTgCACAACA TgggggATCA
6101 TgTAACTCgC CTTgATCgTT gggAACCggA gCTgAATgAA gCCATACCAA
6151 ACgACgAgCg TgACACCACg ATgCCTgCAg CAATggCAAC AACgTTgCgC
6201 AAACTATTAA CTggCgAACT ACTTACTCTA gCTTCCCggC AACAATTAAT
6251 AgACTggATg gAggCggATA AAgTTgCAgg ACCACTTCTg CgCTCggCCC
6301 TTCCggCTgg CTggTTTATT gCTgATAAAT CTggAgCCgg TgAgCgTggg
6351 TCTCgCggTA TCATTgCAgC ACTggggCCA gATggTAAgC CCTCCCgTAT
6401 CgTAgTTATC TACACgACgg ggAgTCAggC AACTATggAT gAACgAAATA
6451 gACAgATCgC TgAgATAggT gCCTCACTgA TTAAgCATTg gTAACTgTCA
6501 gACCAAgTTT ACTCATATAT ACTTTAgATT gATTTAAATT gTAAACgTTA
6551 ATATTTTgTT AAAATTCgCg TTAAATTTTT gTTAAATCAg CTCATTTTTT
6601 AACCAATAgg CCgAAATCgg CAAAATCCCT TATAAATCAA AAgAATAgAC
6651 CgAgATAggg TTgAgTgTTg TTCCAgTTTg gAACAAgAgT CCACTATTAA
6701 AgAACgTggA CTCCAACgTC AAAgggCgAA AAACCgTCTA TCAgggCgAT
6751 ggCCCACTAC gTgAACCATC ACCCTAATCA AgTTTTTTgg ggTCgAggTg
6801 CCgTAAAgCA CTAAATCggA ACCCTAAAgg gAgCCCCCgA TTTAgAgCTT
6851 gACggggAAA gCCggCgAAC gTggCgAgAA AggAAgggAA gAAAgCgAAA
6901 ggAgCgggCg CTAgggCgCT ggCAAgTgTA gCggTCACgC TgCgCgTAAC
6951 CACCACACCC gCCgCgCTTA ATgCgCCgCT ACAgggCgCg TAAAAggATC
7001 TAggTgAAgA TCCTTTTTgA TAATCTCATg ACCAAAATCC CTTAACgTgA
7051 gTTTTCgTTC CACTgAgCgT CAgACCCCgT AgAAAAgATC AAAggATCTT
7101 CTTgAgATCC TTTTTTTCTg CgCgTAATCT gCTgCTTgCA AACAAAAAAA
7151 CCACCgCTAC CAgCggTggT TTgTTTgCCg gATCAAgAgC TACCAACTCT
7201 TTTTCCgAAg gTAACTggCT TCAgCAgAgC gCAgATACCA AATACTgTCC

Table 250, continued

7251 TTCTAgTgTA gCCgTAgTTA ggCCACCACT TCAAgAACTC TgTAgCACCg
7301 CCTACATACC TCgCTCTgCT AATCCTgTTA CCAgTggCTg CTgCCAgTgg
7351 CgATAAgTCg TgTCTTACCg ggTTggACTC AAgACgATAg TTACCggATA
7401 AggCgCAgCg gTCgggCTgA ACggggggTT CgTgCACACA gCCCAgCTTg
7451 gAgCgAACgA CCTACACCgA ACTgAgATAC CTACAgCgTg AgCATTgAgA
7501 AAgCgCCACg CTTCCCgAAg ggAgAAAggC ggACAggTAT CCggTAAgCg
7551 gCAgggTCgg AACAggAgAg CgCACgAggg AgCTTCCAgg gggAAACgCC
7601 TggTATCTTT ATAgTCCTgT CgggTTTCgC CACCTCTgAC TTgAgCgTCg
7651 ATTTTTgTgA TgCTCgTCAg gggggCggAg CCTATggAAA AACgCCAgCA
7701 ACgCggCCTT TTTACggTTC CTggCCTTTT gCTggCCTTT TgCTCACATg
7751 TTCTTTCCTg CgTTATCCCC TgATTCTgTg gATAACCgTA TTACCgCCTT
7801 TgAgTgAgCT gATACCgCTC gCCgCAgCCg AAgACCgAg CgCAgCgAgT
7851 CAgTgAgCgA ggAAgCggAA gAgCgCCTgA TgCggTATTT TCTCCTTACg
7901 CATCTgTgCg gTATTTCACA CCgCATATgg TgCACTCTCA gTACAATCTg
7951 CTCTgATgCC gCATAgTTAA gCCAgTATAC ACTCCgCTAT CgCTACgTgA
8001 CTgggTCATg gCTgCgCCCC gACACCCgCC AACACCCgCT gACgCgCCCT
8051 gACgggCTTg TCTgCTCCCg gCATCCgCTT ACAgACAAgC TgTgACCgTC
8101 TCCgggAgCT gCATgTgTCA gAggTTTTCA CCgTCATCAC CgAAACgCgC
8151 gAggCAg

Table 251: pHIL-D2(MFαPrePro::EPI-HNE-3) 8584 b.p.
DNA has SEQ ID NO. 071; Encoded polypeptide has SEQ ID NO. 072. DNA is circular and double stranded, only one strand is shown. Translation of the protein to be expressed is shown.

```
                1          2          3          4          5
         1234567890 1234567890 1234567890 1234567890 1234567890
       1 AgATCgCggC CgCgATCTAA CATCCAAAgA CgAAAggTTg AATgAAACCT
      51 TTTTgCCATC CgACATCCAC AggTCCATTC TCACACATAA gTgCCAAACg
     101 CAACAggAgg ggATACACTA gCAgCAgACC gTTgCAAACg CAggACCTCC
     151 ACTCCTCTTC TCCTCAACAC CCACTTTTgC CATCgAAAAA CCAgCCCAgT
     201 TATTgggCTT gATTggAgCT CgCTCATTCC AATTCCTTCT ATTAggCTAC
     251 TAACACCATg ACTTTATTAg CCTgTCTATC CTggCCCCCC TggCgAggTC
     301 ATgTTTgTTT ATTTCCgAAT gCAACAAgCT CCgCATTACA CCCgAACATC
     351 ACTCCAgATg AgggCTTTCT gAgTgTgggg TCAAATAgTT TCATgTTCCC
     401 AAATggCCCA AAACTgACAg TTTAAACgCT gTCTTggAAC CTAATATgAC
     451 AAAAgCgTgA TCTCATCCAA gATgAACTAA gTTTggTTCg TTgAAATgCT
     501 AACggCCAgT TggTCAAAAA gAAACTTCCA AAAgTCgCCA TACCgTTTgT
     551 CTTgTTTggT ATTgATTgAC gAATgCTCAA AAATAATCTC ATTAATgCTT
     601 AgCgCAgTCT CTCTATCgCT TCTgAACCCg gTggCACCTg TgCCgAAACg
     651 CAAATggggA AACAACCCgC TTTTTggATg ATTATgCATT gTCCTCCACA
     701 TTgTATgCTT CCAAgATTCT ggTgggAATA CTgCTgATAg CCTAACgTTC
     751 ATgATCAAAA TTTAACTgTT CTAACCCCTA CTTgACAggC AATATATAAA
     801 CAgAAggAAg CTgCCCTgTC TTAAACCTTT TTTTTTATCA TCATTATTAg
     851 CTTACTTTCA TAATTgCgAC TggTTCCAAT TgACAAgCTT TTgATTTTAA
     901 CgACTTTTAA CgACAACTTg AgAAgATCAA AAAACAACTA ATTATTCgAA
!                                                     BstBI
       ACg
!         M   R   F   P   S   I   F   T   A   V   L   F   A   13
         ATg AgA TTC CCA TCT ATC TTC ACT gCT gTT TTg TTC gCT
!            |  BsaBI   |
!
! A   S   S   A   L   A   A   P   V   N   T   T   T   E       27
 gCT TCC TCT gCT TTg gCT gCT CCA gTT AAC ACC ACT ACT gAA
!                            BpmI    HpaI                BbsI
!
! D   E   T   A   Q   I   P   A   E   A   V   I   G   Y       41
 gAC gAg ACT gCT CAA ATT CCT gCT gAg gCT gTC ATC ggT TAC
!BbsI
!
! S   D   L   E   G   D   F   D   V   A   V   L   P   F       55
```

Table 251, continued

```
   TCT gAC TTg gAA ggT gAC TTC gAC gTC gCT gTT TTg CCA TTC
!                              AatII
!
!  S   N   S   T   N   N   G   L   L   F   I   N   T   T   69
   TCT AAC TCT ACT AAC AAC ggT TTg TTg TTC ATC AAC ACT ACC
!
!  I   A   S   I   A   A   K   E   E   G   V   S   L   D   83
   ATC gCT TCT ATC gCT gCT AAg gAg gAA ggT gTT TCC TTg gAC
!
!  K   R       A   A   C   N   L   P                       91
   AAg AgA     gCT gCT TgT AAC TTg CCA
          L________ Site of cleavage
!
!  I   V   R   G   P   C   I   A   F   F   P   R   W   A  105
   ATC gTC AgA ggT CCA TgC ATT gCT TTC TTC CCA AgA Tgg gCT
!                     NsiI
!
!  F   D   A   V   K   G   K   C   V   L   F   P   Y   G  119
   TTC gAC gCT gTT AAg ggT AAg TgC gTC TTg TTC CCA TAC ggT
!                                              | PflMI
!
!  G   C   Q   G   N   G   N   K   F   Y   S   E   K   E  133
   ggT TgT CAA ggT AAC ggT AAC AAg TTC TAC TCT gAg AAg gAg
!  PflMI
!
!  C   R   E   Y   C   G   V   P   .   .                  141
   TgT AgA gAg TAC TgT ggT gTT CCA TAg TAA gAATTCgCCT
!                                          EcoRI

                                                     TAgACATg
1401 ACTgTTCCTC AgTTCAAgTT gggCATTACg AgAAgACCgg TCTTgCTAgA
1451 TTCTAATCAA gAggATgTCA gAATgCCATT TgCCTgAgAg ATgCAggCTT
1501 CATTTTTgAT ACTTTTTTAT TTgTAACCTA TATAgTATAg gATTTTTTTT
1551 gTCATTTTgT TTCTTCTCgT ACgAgCTTgC TCCTgATCAg CCTATCTCgC
1601 AgCTgATgAA TATCTTgTgg TAggggTTTg ggAAAATCAT TCgAgTTTgA
1651 TgTTTTTCTT ggTATTTCCC ACTCCTCTTC AgAgTACAgA AgATTAAgTg
1701 AgAAgTTCgT TTgTgCAAgC TTATCgATAA gCTTTAATgC ggTAgTTTAT
1751 CACAgTTAAA TTgCTAACgC AgTCAggCAC CgTgTATgAA ATCTAACAAT
1801 gCgCTCATCg TCATCCTCgg CACCgTCACC CTggATgCTg TAggCATAgg
1851 CTTggTTATg CCggTACTgC CgggCCTCTT gCgggATATC gTCCATTCCg
1901 ACAgCATCgC CAgTCACTAT ggCgTgCTgC TAgCgCTATA TgCgTTgATg
1951 CAATTTCTAT gCgCACCCgT TCTCggAgCA CTgTCCgACC gCTTTggCCg
2001 CCgCCCAgTC CTgCTCgCTT CgCTACTTgg AgCCACTATC gACTACgCgA
2051 TCATggCgAC CACACCCgTC CTgTggATCT ATCgAATCTA AATgTAAgTT
2101 AAAATCTCTA AATAATTAAA TAAgTCCCAg TTTCTCCATA CgAACCTTAA
2151 CAgCATTgCg gTgAgCATCT AgACCTTCAA CAgCAgCCAg ATCCATCACT
2201 gCTTggCCAA TATgTTTCAg TCCCTCAggA gTTACgTCTT gTgAAgTgAT
```

Table 251, continued

2251 gAACTTCTgg AAggTTgCAg TgTTAACTCC gCTgTATTgA CgggCATATC
2301 CgTACgTTgg CAAAgTgTgg TTggTACCgg AggAgTAATC TCCACAACTC
2351 TCTggAgAgT AggCACCAAC AAACACAgAT CCAgCgTgTT gTACTTgATC
2401 AACATAAgAA gAAgCATTCT CgATTTgCAg gATCAAgTgT TCAggAgCgT
2451 ACTgATTggA CATTTCCAAA gCCTgCTCgT AggTTgCAAC CgATAgggTT
2501 gTAgAgTgTg CAATACACTT gCgTACAATT TCAACCCTTg gCAACTgCAC
2551 AgCTTggTTg TgAACAgCAT CTTCAATTCT ggCAAgCTCC TTgTCTgTCA
2601 TATCgACAgC CAACAgAATC ACCTgggAAT CAATACCATg TTCAgCTTgA
2651 gCAgAAggTC TgAggCAACg AAATCTggAT CAgCgTATTT ATCAgCAATA
2701 ACTAgAACTT CAgAAggCCC AgCAggCATg TCAATACTAC ACAgggCTgA
2751 TgTgTCATTT TgAACCATCA TCTTggCAgC AgTAACgAAC TggTTTCCTg
2801 gACCAAATAT TTTgTCACAC TTAggAACAg TTTCTgTTCC gTAAgCCATA
2851 gCAgCTACTg CCTgggCgCC TCCTgCTAgC ACgATACACT TAgCACCAAC
2901 CTTgTgggCA ACgTAgATgA CTTCTggggT AAgggTACCA TCCTTCTTAg
2951 gTggAgATgC AAAAACAATT TCTTTgCAAC CAgCAACTTT ggCAggAACA
3001 CCCAgCATCA gggAAgTggA AggCAgAATT gCggTTCCAC CAggAATATA
3051 gAggCCAACT TTCTCAATAg gTCTTgCAAA ACgAgAgCAg ACTACACCAg
3101 ggCAAgTCTC AACTTgCAAC gTCTCCgTTA gTTgAgCTTC ATggAATTTC
3151 CTgACgTTAT CTATAgAgAg ATCAATggCT CTCTTAACgT TATCTggCAA
3201 TTgCATAAgT TCCTCTgggA AAggAgCTTC TAACACAggT gTCTTCAAAg
3251 CgACTCCATC AAACTTggCA gTTAgTTCTA AAAgggCTTT gTCACCATTT
3301 TgACgAACAT TgTCgACAAT TggTTTgACT AATTCCATAA TCTgTTCCgT
3351 TTTCTggATA ggACgACgAA gggCATCTTC AATTTCTTgT gAggAggCCT
3401 TAgAAACgTC AATTTTgCAC AATTCAATAC gACCTTCAgA AgggACTTCT
3451 TTAggTTTgg ATTCTTCTTT AggTTgTTCC TTggTgTATC CTggCTTggC
3501 ATCTCCTTTC CTTCTAgTgA CCTTTAgggA CTTCATATCC AggTTTCTCT
3551 CCACCTCgTC CAACgTCACA CCgTACTTgg CACATCTAAC TAATgCAAAA
3601 TAAAATAAgT CAgCACATTC CCAggCTATA TCTTCCTTgg ATTTAgCTTC
3651 TgCAAgTTCA TCAgCTTCCT CCCTAATTTT AgCgTTCAAC AAAACTTCgT
3701 CgTCAAATAA CCgTTTggTA TAAgAACCTT CTggAgCATT gCTCTTACgA
3751 TCCCACAAgg TgCTTCCATg gCTCTAAgAC CCTTTgATTg gCCAAAACAg
3801 gAAgTgCgTT CCAAgTgACA gAAACCAACA CCTgTTTgTT CAACCACAAA
3851 TTTCAAgCAg TCTCCATCAC AATCCAATTC gATACCCAgC AACTTTTgAg
3901 TTCgTCCAgA TgTAgCACCT TTATACCACA AACCgTgACg ACgAgATTgg
3951 TAgACTCCAg TTTgTgTCCT TATAgCCTCC ggAATAgACT TTTTggACgA
4001 gTACACCAgg CCCAACgAgT AATTAgAAgA gTCAgCCACC AAAgTAgTgA
4051 ATAgACCATC ggggCggTCA gTAgTCAAAg ACgCCAACAA AATTTCACTg

Table 251, continued

```
4101 ACAgggAACT TTTTgACATC TTCAgAAAgT TCgTATTCAg TAgTCAATTg
4151 CCgAgCATCA ATAATggggA TTATACCAgA AgCAACAgTg gAAgTCACAT
4201 CTACCAACTT TgCggTCTCA gAAAAAgCAT AAACAgTTCT ACTACCgCCA
4251 TTAgTgAAAC TTTTCAAATC gCCCAgTggA gAAgAAAAAg gCACAgCgAT
4301 ACTAgCATTA gCgggCAAgg ATgCAACTTT ATCAACCAgg gTCCTATAgA
4351 TAACCCTAgC gCCTgggATC ATCCTTTggA CAACTCTTTC TgCCAAATCT
4401 AggTCCAAAA TCACTTCATT gATACCATTA TACggATgAC TCAACTTgCA
4451 CATTAACTTg AAgCTCAgTC gATTgAgTgA ACTTgATCAg gTTgTgCAgC
4501 TggTCAgCAg CATAgggAAA CACggCTTTT CCTACCAAAC TCAAggAATT
4551 ATCAAACTCT gCAACACTTg CgTATgCAgg TAgCAAgggA AATgTCATAC
4601 TTgAAgTCgg ACAgTgAgTg TAgTCTTgAg AAATTCTgAA gCCgTATTTT
4651 TATTATCAgT gAgTCAgTCA TCAggAgATC CTCTACgCCg gACgCATCgT
4701 ggCCggCATC ACCggCgCCA CAggTgCggT TgCTggCgCC TATATCgCCg
4751 ACATCACCgA TggggAAgAT CgggCTCgCC ACTTCgggCT CATgAgCgCT
4801 TgTTTCggCg TgggTATggT ggCAggCCCC gTggCCgggg gACTgTTggg
4851 CgCCATCTCC TTgCATgCAC CATTCCTTgC ggCggCggTg CTCAACggCC
4901 TCAACCTACT ACTgggCTgC TTCCTAATgC AggAgTCgCA TAAgggAgAg
4951 CgTCgAgTAT CTATgATTgg AAgTATgggA ATggTgATAC CCgCATTCTT
5001 CAgTgTCTTg AggTCTCCTA TCAgATTATg CCCAACTAAA gCAACCggAg
5051 gAggAgATTT CATggTAAAT TTCTCTgACT TTTggTCATC AgTAgACTCg
5101 AACTgTgAgA CTATCTCggT TATgACAgCA gAAATgTCCT TCTTggAgAC
5151 AgTAAATgAA gTCCCACCAA TAAAgAAATC CTTgTTATCA ggAACAAACT
5201 TCTTgTTTCg AACTTTTTCg gTgCCTTgAA CTATAAAATg TAgAgTggAT
                BstBI
5251 ATgTCgggTA ggAATggAgC gggCAAATgC TTACCTTCTg gACCTTCAAg
5301 AggTATgTAg ggTTTgTAgA TACTgATgCC AACTTCAgTg ACAACgTTgC
5351 TATTTCgTTC AAACCATTCC gAATCCAgAg AAATCAAAgT TgTTTgTCTA
5401 CTATTgATCC AAgCCAgTgC ggTCTTgAAA CTgACAATAg TgTgCTCgTg
5451 TTTTgAggTC ATCTTTgTAT gAATAAATCT AgTCTTTgAT CTAAATAATC
5501 TTgACgAgCC AAggCgATAA ATACCCAAAT CTAAAACTCT TTTAAAACgT
5551 TAAAAggACA AgTATgTCTg CCTgTATTAA ACCCCAAATC AgCTCgTAgT
5601 CTgATCCTCA TCAACTTgAg gggCACTATC TTgTTTTAgA gAAATTTgCg
5651 gAgATgCgAT ATCgAgAAAA AggTACgCTg ATTTTAAACg TgAAATTTAT
5701 CTCAAgATCg CggCCgCgAT CTCgAATAAT AACTgTTATT TTTCAgTgTT
5751 CCCgATCTgC gTCTATTTCA CAATACCAAC ATgAgTCAgC TTATCgATgA
5801 TAAgCTgTCA AACATgAgAA TTAATTCgAT gATAAgCTgT CAAACATgAg
5851 AAATCTTgAA gACgAAAggg CCTCgTgATA CgCCTATTTT TATAggTTAA
```

Table 251, continued

```
5901 TgTCATgATA ATAATggTTT CTTAgACgTC AggTggCACT TTTCggggAA
                                AatII
5951 ATgTgCgCgg AACCCCTATT TgTTTATTTT TCTAAATACA TTCAAATATg
6001 TATCCgCTCA TgAgACAATA ACCCTgATAA ATgCTTCAAT AATATTgAAA
6051 AAggAAgAgT ATgAgTATTC AACATTTCCg TgTCgCCCTT ATTCCCTTTT
6101 TTgCggCATT TTgCCTTCCT gTTTTTgCTC ACCCAgAAAC gCTggTgAAA
6151 gTAAAAgATg CTgAAgATCA gTTgggTgCA CgAgTgggTT ACATCgAACT
6201 ggATCTCAAC AgCggTAAgA TCCTTgAgAg TTTTCgCCCC gAAgAACgTT
6251 TTCCAATgAT gAgCACTTTT AAAgTTCTgC TATgTggCgC ggTATTATCC
6301 CgTgTTgACg CCgggCAAgA gCAACTCggT CgCCgCATAC ACTATTCTCA
6351 gAATgACTTg gTTgAgTACT CACCAgTCAC AgAAAAgCAT CTTACggATg
6401 gCATgACAgT AAgAgAATTA TgCAgTgCTg CCATAACCAT gAgTgATAAC
6451 ACTgCggCCA ACTTACTTCT gACAACgATC ggAggACCgA AggAgCTAAC
6501 CgCTTTTTTg CACAACATgg gggATCATgT AACTCgCCTT gATCgTTggg
6551 AACCggAgCT gAATgAAgCC ATACCAAACg ACgAgCgTgA CACCACgATg
6601 CCTgCAgCAA TggCAACAAC gTTgCgCAAA CTATTAACTg gCgAACTACT
6651 TACTCTAgCT TCCCggCAAC AATTAATAgA CTggATggAg gCggATAAAg
6701 TTgCAggACC ACTTCTgCgC TCggCCCTTC CggCTggCTg gTTTATTgCT
6751 gATAAATCTg gAgCCggTgA gCgTgggTCT CgCggTATCA TTgCAgCACT
6801 ggggCCAgAT ggTAAgCCCT CCCgTATCgT AgTTATCTAC ACgACggggA
6851 gTCAggCAAC TATggATgAA CgAAATAgAC AgATCgCTgA gATAggTgCC
6901 TCACTgATTA AgCATTggTA ACTgTCAgAC CAAgTTTACT CATATATACT
6951 TTAgATTgAT TTAAATTgTA AACgTTAATA TTTTgTTAAA ATTCgCgTTA
7001 AATTTTTgTT AAATCAgCTC ATTTTTTAAC CAATAggCCg AAATCggCAA
7051 AATCCCTTAT AAATCAAAAg AATAgACCgA gATAgggTTg AgTgTTgTTC
7101 CAgTTTggAA CAAgAgTCCA CTATTAAAgA ACgTggACTC CAACgTCAAA
7151 gggCgAAAAA CCgTCTATCA gggCgATggC CCACTACgTg AACCATCACC
7201 CTAATCAAgT TTTTTggggT CgAggTgCCg TAAAgCACTA AATCggAACC
7251 CTAAAgggAg CCCCCgATTT AgAgCTTgAC ggggAAAgCC ggCgAACgTg
7301 gCgAgAAAgg AAgggAAgAA AgCgAAAggA gCgggCgCTA gggCgCTggC
7351 AAgTgTAgCg gTCACgCTgC gCgTAACCAC CACACCCgCC gCgCTTAATg
7401 CgCCgCTACA gggCgCgTAA AAggATCTAg gTgAAgATCC TTTTTgATAA
7451 TCTCATgACC AAAATCCCTT AACgTgAgTT TTCgTTCCAC TgAgCgTCAg
7501 ACCCCgTAgA AAAgATCAAA ggATCTTCTT gAgATCCTTT TTTTCTgCgC
7551 gTAATCTgCT gCTTgCAAAC AAAAAAACCA CCgCTACCAg CggTggTTTg
7601 TTTgCCggAT CAAgAgCTAC CAACTCTTTT TCCgAAggTA ACTggCTTCA
7651 gCAgAgCgCA gATACCAAAT ACTgTCCTTC TAgTgTAgCC gTAgTTAggC
```

Table 251, continued

```
7701 CACCACTTCA AgAACTCTgT AgCACCgCCT ACATACCTCg CTCTgCTAAT
7751 CCTgTTACCA gTggCTgCTg CCAgTggCgA TAAgTCgTgT CTTACCgggT
7801 TggACTCAAg ACgATAgTTA CCggATAAgg CgCAgCggTC gggCTgAACg
7851 gggggTTCgT gCACACAgCC CAgCTTggAg CgAACgACCT ACACCgAACT
7901 gAgATACCTA CAgCgTgAgC ATTgAgAAAg CgCCACgCTT CCCgAAgggA
7951 gAAAggCggA CAggTATCCg gTAAgCggCA gggTCggAAC AggAgAgCgC
8001 ACgAgggAgC TTCCAggggg AAACgCCTgg TATCTTTATA gTCCTgTCgg
8051 gTTTCgCCAC CTCTgACTTg AgCgTCgATT TTTgTgATgC TCgTCAgggg
8101 ggCggAgCCT ATggAAAAAC gCCAgCAACg CggCCTTTTT ACggTTCCTg
8151 gCCTTTTgCT ggCCTTTTgC TCACATgTTC TTTCCTgCgT TATCCCCTgA
8201 TTCTgTggAT AACCgTATTA CCgCCTTTgA gTgAgCTgAT ACCgCTCgCC
8251 gCAgCCgAAC gACCgAgCgC AgCgAgTCAg TgAgCgAggA AgCggAAgAg
8301 CgCCTgATgC ggTATTTTCT CCTTACgCAT CTgTgCggTA TTTCACACCg
8351 CATATggTgC ACTCTCAgTA CAATCTgCTC TgATgCCgCA TAgTTAAgCC
8401 AgTATACACT CCgCTATCgC TACgTgACTg ggTCATggCT gCgCCCCgAC
8451 ACCCgCCAAC ACCCgCTgAC gCgCCCTgAC gggCTTgTCT gCTCCCggCA
8501 TCCgCTTACA gACAAgCTgT gACCgTCTCC gggAgCTgCA TgTgTCAgAg
8551 gTTTTCACCg TCATCACCgA AACgCgCgAg gCAg
```

**Restriction map of pHIL-D2(MFαPrePro::EPI-HNE-3)**

Non-cutters

| | | | | |
|---|---|---|---|---|
| *Afl*II | *Apa*I | *Asc*I | *Ava*I | *Avr*II |
| *Bam*HI | *Bgl*II | *Bss*HII | *Bst*EII | *Mlu*I |
| *Nru*I | *Pac*I | *Pml*I | *Rsr*II | *Sac*II |
| *Sfi*I | *Sna*BI | *Spe*I | *Xho*I | *Xma*I |

Cutters, 3 or fewer sites

| | | | | | | | | | |
|---|---|---|---|---|---|---|---|---|---|
| *Aat*II | 2 | 1098 | 5925 | | *Bsi*WI | 2 | 1568 | 2301 | |
| *Afl*III | 1 | 8173 | | | *Bsp*DI | 2 | 1723 | 5793 | |
| *Age*I | 1 | 1436 | | | *Bsp*EI | 1 | 3978 | | |
| *Alw*NI | 3 | 2828 | 2852 | 7759 | *Bsp*MI | 1 | 4576 | | |
| *Apa*LI | 3 | 6176 | 7859 | 8357 | *Bst*1107I | 1 | 8402 | | |
| *Ase*I | 3 | 591 | 5820 | 6672 | *Bst*BI(*Asu*II) | 2 | 945 | 5207 | |
| *Bgl*I | 3 | 284 | 2717 | 6724 | *Bst*XI | 3 | 711 | 2765 | 2896 |
| *Bsa*AI | 2 | 7185 | 8421 | | *Bsu*36I | 1 | 2223 | | |
| *Bsg*I | 2 | 2545 | 4494 | | *Dra*III | 2 | 3754 | 7182 | |

Table 251, continued

| | | | | |
|---|---|---|---|---|
| *Eag*I | 3 | 7 | 5711 | 8591 |
| *Eam*1105I | 2 | 5077 | 6843 | |
| *Ecl*136I | 1 | 216 | | |
| *Eco*47III | 2 | 1932 | 4795 | |
| *Eco*NI | 3 | 3433 | 4923 | 5293 |
| *Eco*RI | 1 | 1383 | | |
| *Eco*RV | 2 | 1885 | 5658 | |
| *Esp*3I(*Bsa*I) | 2 | 3120 | 8524 | |
| *Esp*I(*Bpu*1102I) | 1 | 597 | | |
| *Fsp*I | 2 | 1960 | 6623 | |
| *Hind*III | 3 | 885 | 1717 | 1729 |
| *Hpa*I | 2 | 1017 | 2272 | |
| *Kpn*I | 2 | 2323 | 2934 | |
| *Msc*I | 2 | 2204 | 3789 | |
| *Nco*I | 1 | 3766 | | |
| *Nde*I | 1 | 8351 | | |
| *Ngo*MI | 2 | 4702 | 7288 | |
| *Nhe*I | 2 | 1929 | 2875 | |
| *Not*I | 3 | 6 | 5710 | 8590 |
| *Nsi*I | 2 | 684 | 1241 | |
| *Pfl*MI | 2 | 196 | 1302 | |
| *Pme*I | 1 | 420 | | |
| *Ppu*MI | 2 | 142 | 4339 | |
| *Pst*I | 1 | 6602 | | |
| *Pvu*I | 1 | 6476 | | |
| *Pvu*II | 2 | 1600 | 4497 | |
| *Sac*I | 1 | 216 | | |
| *Sal*I | 1 | 3312 | | |
| *Sca*I | 2 | 1360 | 6365 | |
| *Sph*I | 1 | 4863 | | |
| *Ssp*I | 3 | 2806 | 6041 | 6977 |
| *Stu*I | 1 | 3395 | | |
| *Tth*111I | 1 | 8426 | | |
| *Xba*I | 1 | 2168 | | |
| *Xcm*I | 1 | 711 | | |

Table 251, continued

**Table 252:** *Bst*BI-*Aat*II-*Eco*RI cassette for expression of EPI-HNE-4

DNA has SEQ ID NO. 073; amino-acid sequence has SEQ ID NO. 074

```
!                 M   R   F   P   S   I   F   T
 5'TTCgAA ACg ATg AgA TTC CCA TCT ATC TTC ACT
    BstBI           |  BsaBI    |

!              A   V   L   F   A    13
              gCT gTT TTg TTC gCT
!
! A   S   S   A   L   A   A   P   V   N   T   T   T   E    27
 gCT TCC TCT gCT TTg gCT gCT CCA gTT AAC ACC ACT ACT gAA
!                           BpmI  HpaI                BbsI
!
! D   E   T   A   Q   I   P   A   E   A   V   I   G   Y    41
 gAC gAg ACT gCT CAA ATT CCT gCT gAg gCT gTC ATC ggT TAC
! BbsI
!
! S   D   L   E   G   D   F   D   V   A   V   L   P   F    55
 TCT gAC TTg gAA ggT gAC TTC gAC gTC gCT gTT TTg CCA TTC
!                             AatII
!
! S   N   S   T   N   N   G   L   L   F   I   N   T   T    69
 TCT AAC TCT ACT AAC AAC ggT TTg TTg TTC ATC AAC ACT ACC
!
! I   A   S   I   A   A   K   E   E   G   V   S   L   D    83
 ATC gCT TCT ATC gCT gCT AAg gAg gAA ggT gTT TCC TTg gAC
!
! K   R   E   A   C   N   L   P                            91
 AAg AgA gAg gCT TgT AAC TTg CCA
!
! I   V   R   G   P   C   I   A   F   F   P   R   W   A   105
 ATC gTC AgA ggT CCA TgC ATT gCT TTC TTC CCA AgA Tgg gCT
!                     NsiI
!
! F   D   A   V   K   G   K   C   V   L   F   P   Y   G   119
 TTC gAC gCT gTT AAg ggT AAg TgC gTC TTg TTC CCA TAC ggT
!                                            |  PflMI
!
! G   C   Q   G   N   G   N   K   F   Y   S   E   K   E   133
 ggT TgT CAA ggT AAC ggT AAC AAg TTC TAC TCT gAg AAg gAg
! PflMI
!
! C   R   E   Y   C   G   V   P   .   .                   141
 TgT AgA gAg TAC TgT ggT gTT CCA TAg TAA gAATTC
!                                         EcoRI
```

The DNA is a linear fragment that is double stranded *in vivo*, only one strand is shown. The amino acid sequence is that of a disulfide-containing protein that is processed *in vivo*.

**Table 253**: pD2pick(MFαPrePro::EPI-HNE-3), 8590 bp, CIRCULAR dsDNA, one strand shown. pD2pick(MFαPrePro::EPI-HNE-3) DNA has SEQ ID NO. 075 Encoded protein has SEQ ID NO. 076

```
              1          2          3          4          5
     1234567890 1234567890 1234567890 1234567890 1234567890
   1 AgATCgCggC CgCgATCTAA CATCCAAAgA CgAAAggTTg AATgAAACCT
  51 TTTTgCCATC CgACATCCAC AggTCCATTC TCACACATAA gTgCCAAACg
 101 CAACAggAgg ggATACACTA gCAgCAgACC gTTgCAAACg CAggACCTCC
 151 ACTCCTCTTC TCCTCAACAC CCACTTTTgC CATCgAAAAA CCAgCCCAgT
 201 TATTgggCTT gATTggAgCT CgCTCATTCC AATTCCTTCT ATTAggCTAC
                    SacI
 251 TAACACCATg ACTTTATTAg CCTgTCTATC CTggCCCCCC TggCgAggTC
 301 ATgTTTgTTT ATTTCCgAAT gCAACAAgCT CCgCATTACA CCCgAACATC
 351 ACTCCAgATg AgggCTTTCT gAgTgTgggg TCAAATAgTT TCAtgTTCCC
 401 AAATggCCCA AAACTgACAg TTTAAACgCT gTCTTggAAC CTAATATgAC
                          PmeI
 451 AAAAgCgTgA TCTCATCCAA gATgAACTAA gTTTggTTCg TTgAAATgCT
 501 AACggCCAgT TggTCAAAAA gAAACTTCCA AAAgTCgCCA TACCgTTTgT
 551 CTTgTTTggT ATTgATTgAC gAATgCTCAA AAATAATCTC ATTAATgCTTAgC
                                                        EspI
 604    gCAgTCT CTCTATCgCT TCTgAACCCg gTggCACCTg TgCCgAAACg
 651 CAAATggggA AACAACCCgC TTTTTggATg ATTATgCATT gTCCTCCACA
 701 TTgTATgCTT CCAAgATTCT ggTgggAATA CTgCTgATAg CCTAACgTTC
                XcmI
 751 ATgATCAAAA TTTAACTgTT CTAACCCCTA CTTgACAggC AATATATAAA
 801 CAgAAggAAg CTgCCCTgTC TTAAACCTTT TTTTTTATCA TCATTATTAg
 851 CTTACTTTCA TAATTgCgAC TggTTCCAAT TgACAAgCTT TTgATTTTAA
 901 CgACTTTTAA CgACAACTTg AgAAgATCAA AAAACAACTA ATTATTCgAA
!                                                   BstBI
 951 ACg
!
!      M   R   F   P   S   I   F   T   A   V   L   F   A
 954 ATg AgA TTC CCA TCT ATC TTC ACT gCT gTT TTg TTC gCT
!
!      A   S   S   A   L   A   A   P   V   N   T   T   T
 993 gCT TCC TCT gCT TTg gCT gCT CCA gTT AAC ACC ACT ACT
!
!      E   D   E   T   A   Q   I   P   A   E   A   V   I
1032 gAA gAC gAg ACT gCT CAA ATT CCT gCT gAg gCT gTC ATC
```

Table 253, continued

```
!
!        G   Y   S   D   L   E   G   D   F   D   V   A   V
 1071 ggT TAC TCT gAC TTg gAA ggT gAC TTC gAC gTC gCT gTT
                                          AatII
!
!        L   P   F   S   N   S   T   N   N   G   L   L   F
 1110 TTg CCA TTC TCT AAC TCT ACT AAC AAC ggT TTg TTg TTC
!
!        I   N   T   T   I   A   S   I   A   A   K   E   E
 1149 ATC AAC ACT ACC ATC gCT TCT ATC gCT gCT AAg gAg gAA
!
!        G   V   S   L   D   K   R   A   A   C   N   L   P
 1188 ggT gTT TCC TTg gAC AAg AgA gCT gCT TgT AAC TTg CCA
!
!        I   V   R   G   P   C   I   A   F   F   P   R   W
 1227 ATC gTC AgA ggT CCA TgC ATT gCT TTC TTC CCA AgA Tgg
!
!        A   F   D   A   V   K   G   K   C   V   L   F   P
 1266 gCT TTC gAC gCT gTT AAg ggT AAg TgC gTC TTg TTC CCA
!
!        Y   G   G   C   Q   G   N   G   N   K   F   Y   S
 1305 TAC ggT ggT TgT CAA ggT AAC ggT AAC AAg TTC TAC TCT
!
!        E   K   E   C   R   E   Y   C   G   V   P   .   .
 1344 gAg AAg gAg TgT AgA gAg TAC TgT ggT gTT CCA TAg TAA
!
 1383 gAATTC                                      gC CTTAgACATg
!     EcoRI
1401 ACTgTTCCTC AgTTCAAgTT gggCATTACg AgAAgACCgg TCTTgCTAgA
                                          AegI
1451 TTCTAATCAA gAggATgTCA gAATgCCATT TgCCTgAgAg ATgCAggCTT
1501 CATTTTTgAT ACTTTTTTAT TTgTAACCTA TATAgTATAg gATTTTTTTT
1551 gTCATTTTgT TTCTTCTCgT ACgAgCTTgC TCCTgATCAg CCTATCTCgC
1601 AgCTgATgAA TATCTTgTgg TAggggTTTg ggAAAATCAT TCgAgTTTgA
1651 TgTTTTTCTT ggTATTTCCC ACTCCTCTTC AgAgTACAgA AgATTAAgTg
1701 AgAAgTTCgT TTgTgCAAgC TTATCgATAA gCTTTAATgC ggTAgTTTAT
1751 CACAgTTAAA TTgCTAACgC AgTCAggCAC CgTgTATgAA ATCTAACAAT
1801 gCgCTCATCg TCATCCTCgg CACCgTCACC CTggATgCTg TAggCATAgg
1851 CTTggTTATg CCggTACTgC CgggCCTCTT gCgggATATC gTCCATTCCg
1901 ACAgCATCgC CAgTCACTAT ggCgTgCTgC TAgCgCTATA TgCgTTgATg
1951 CAATTTCTAT gCgCACCCgT TCTCggAgCA CTgTCCgACC gCTTTggCCg
2001 CCgCCCAgTC CTgCTCgCTT CgCTACTTgg AgCCACTATC gACTACgCgA
2051 TCATggCgAC CACACCCgTC CTgTggATCT ATCgAATCTA AATgTAAgTT
2101 AAAATCTCTA AATAATTAAA TAAgTCCCAg TTTCTCCATA CgAACCTTAA
2151 CAgCATTgCg gTgAgCATCT AgACCTTCAA CAgCAgCCAg ATCCATCACT
                       XbaI
```

Table 253, continued

```
2201 gCTTggCCAA TATgTTTCAg TCCCTCAggA gTTACgTCTT gTgAAgTgAT
                                 Bsu36I
2251 gAACTTCTgg AAggTTgCAg TgTTAACTCC gCTgTATTgA CgggCATATC
2301 CgTACgTTgg CAAAgTgTgg TTggTACCgg AggAgTAATC TCCACAACTC
2351 TCTggAgAgT AggCACCAAC AAACACAgAT CCAgCgTgTT gTACTTgATC
2401 AACATAAgAA gAAgCATTCT CgATTTgCAg gATCAAgTgT TCAggAgCgT
2451 ACTgATTggA CATTTCCAAA gCCTgCTCgT AggTTgCAAC CgATAgggTT
2501 gTAgAgTgTg CAATACACTT gCgTACAATT TCAACCCTTg gCAACTgCAC
2551 AgCTTggTTg TgAACAgCAT CTTCAATTCT ggCAAgCTCC TTgTCTgTCA
2601 TATCgACAgC CAACAgAATC ACCTgggAAT CAATACCATg TTCAgCTTgA
2651 gCAgAAggTC TgAggCAACg AAATCTggAT CAgCgTATTT ATCAgCAATA
2701 ACTAgAACTT CAgAAggCCC AgCAggCATg TCAATACTAC ACAgggCTgA
2751 TgTgTCATTT TgAACCATCA TCTTggCAgC AgTAACgAAC TggTTTCCTg
2801 gACCAAATAT TTTgTCACAC TTAggAACAg TTTCTgTTCC gTAAgCCATA
2851 gCAgCTACTg CCTgggCgCC TCCTgCTAgC ACgATACACT TAgCACCAAC
2901 CTTgTgggCA ACgTAgATgA CTTCTggggT AAgggTACCA TCCTTCTTAg
2951 gTggAgATgC AAAAACAATT TCTTTgCAAC CAgCAACTTT ggCAggAACA
3001 CCCAgCATCA gggAAgTggA AggCAgAATT gCggTTCCAC CAggAATATA
3051 gAggCCAACT TTCTCAATAg gTCTTgCAAA ACgAgAgCAg ACTACACCAg
3101 ggCAAgTCTC AACTTgCAAC gTCTCCgTTA gTTgAgCTTC ATggAATTTC
3151 CTgACgTTAT CTATAgAgAg ATCAATggCT CTCTTAACgT TATCTggCAA
3201 TTgCATAAgT TCCTCTgggA AAggAgCTTC TAACACAggT gTCTTCAAAg
3251 CgACTCCATC AAACTTggCA gTTAgTTCTA AAAgggCTTT gTCACCATTT
3301 TgACgAACAT TgTCgACAAT TggTTTgACT AATTCCATAA TCTgTTCCgT
3351 TTTCTggATA ggACgACgAA gggCATCTTC AATTTCTTgT gAggAggCCT
                                                   StuI
3401 TAgAAACgTC AATTTTgCAC AATTCAATAC gACCTTCAgA AgggACTTCT
3451 TTAggTTTgg ATTCTTCTTT AggTTgTTCC TTggTgTATC CTggCTTggC
3501 ATCTCCTTTC CTTCTAgTgA CCTTTAgggA CTTCATATCC AggTTTCTCT
3551 CCACCTCgTC CAACgTCACA CCgTACTTgg CACATCTAAC TAATgCAAAA
3601 TAAAATAAgT CAgCACATTC CCAggCTATA TCTTCCTTgg ATTTAgCTTC
3651 TgCAAgTTCA TCAgCTTCCT CCCTAATTTT AgCgTTCAAC AAAACTTCgT
3701 CgTCAAATAA CCgTTTggTA TAAgAACCTT CTggAgCATT gCTCTTACgA
3751 TCCCACAAgg TgCTTCCATg gCTCTAAgAC CCTTTgATTg gCCAAAACAg
                NcoI
3801 gAAgTgCgTT CCAAgTgACA gAAACCAACA CCTgTTTgTT CAACCACAAA
3851 TTTCAAgCAg TCTCCATCAC AATCCAATTC gATACCCAgC AACTTTTgAg
```

Table 253, continued

3901 TTCgTCCAgA TgTAgCACCT TTATACCACA AACCgTgACg ACgAgATTgg
3951 TAgACTCCAg TTTgTgTCCT TATAgCCTCC ggAATAgACT TTTTggACgA
*BspEI*
4001 gTACACCAgg CCCAACgAgT AATTAgAAgA gTCAgCCACC AAAgTAgTgA
4051 ATAgACCATC ggggCggTCA gTAgTCAAAg ACgCCAACAA AATTTCACTg
4101 ACAgggAACT TTTTgACATC TTCAgAAAgT TCgTATTCAg TAgTCAATTg
4151 CCgAgCATCA ATAATggggA TTATACCAgA AgCAACAgTg gAAgTCACAT
4201 CTACCAACTT TgCggTCTCA gAAAAAgCAT AAACAgTTCT ACTACCgCCA
4251 TTAgTgAAAC TTTTCAAATC gCCCAgTggA gAAgAAAAAg gCACAgCgAT
4301 ACTAgCATTA gCgggCAAgg ATgCAACTTT ATCAACCAgg gTCCTATAgA
4351 TAACCCTAgC gCCTgggATC ATCCTTTggA CAACTCTTTC TgCCAAATCT
4401 AggTCCAAAA TCACTTCATT gATACCATTA TACggATgAC TCAACTTgCA
4451 CATTAACTTg AAgCTCAgTC gATTgAgTgA ACTTgATCAg gTTgTgCAgC
4501 TggTCAgCAg CATAgggAAA CACggCTTTT CCTACCAAAC TCAAggAATT
4551 ATCAAACTCT gCAACACTTg CgTATgCAgg TAgCAAgggA AATgTCATAC
4601 TTgAAgTCgg ACAgTgAgTg TAgTCTTgAg AAATTCTgAA gCCgTATTTT
4651 TATTATCAgT gAgTCAgTCA TCAggAgATC CTCTACgCCg gACgCATCgT
4701 ggCCggCATC ACCggCgCCA CAggTgCggT TgCTggCgCC TATATCgCCg
4751 ACATCACCgA TggggAAgAT CgggCTCgCC ACTTCgggCT CATgAgCgCT
4801 TgTTTCggCg TgggTATggT ggCAggCCCC gTggCCgggg gACTgTTggg
4851 CgCCATCTCC TTgCATgCAC CATTCCTTgC ggCggCggTg CTCAACggCC
4901 TCAACCTACT ACTgggCTgC TTCCTAATgC AggAgTCgCA TAAgggAgAg
4951 CgTCgAgTAT CTATgATTgg AAgTATgggA ATggTgATAC CCgCATTCTT
5001 CAgTgTCTTg AggTCTCCTA TCAgATTATg CCCAACTAAA gCAACCggAg
5051 gAggAgATTT CATggTAAAT TTCTCTgACT TTTggTCATC AgTAgACTCg
5101 AACTgTgAgA CTATCTCggT TATgACAgCA gAAATgTCCT TCTTggAgAC
5151 AgTAAATgAA gTCCCACCAA TAAAgAAATC CTTgTTATCA ggAACAAACT
5201 TCTTgTTTCg CgAACTTTTT CggTgCCTTg AACTATAAAA TgTAgAgTgg
5251 ATATgTCggg TAggAATggA gCgggCAAAT gCTTACCTTC TggACCTTCA
5301 AgAggTATgT AgggTTTgTA gATACTgATg CCAACTTCAg TgACAACgTT
5351 gCTATTTCgT TCAAACCATT CCgAATCCAg AgAAATCAAA gTTgTTTgTC
5401 TACTATTgAT CCAAgCCAgT gCggTCTTgA AACTgACAAT AgTgTgCTCg
5451 TgTTTTgAgg TCATCTTTgT ATgAATAAAT CTAgTCTTTg ATCTAAATAA
5501 TCTTgACgAg CCAAggCgAT AAATACCCAA ATCTAAAACT CTTTTAAAAC
5551 gTTAAAAggA CAAgTATgTC TgCCTgTATT AAACCCCAAA TCAgCTCgTA
5601 gTCTgATCCT CATCAACTTg AggggCACTA TCTTgTTTTA gAgAAATTTg
5651 CggAgATgCg ATATCgAgAA AAAggTACgC TgATTTTAAA CgTgAAATTT

Table 253, continued

| | | | | | |
|---|---|---|---|---|---|
| 5701 | ATCTCAAgAT | CgCggCCgCg | ATCTCgAATA | ATAACTgTTA | TTTTTCAgTg |
| 5751 | TTCCCgATCT | gCgTCTATTT | CACAATACCA | ACATgAgTCA | gCTTATCgAT |
| 5801 | gATAAgCTgT | CAAACATgAg | AATTAATTCg | ATgATAAgCT | gTCAAACATg |
| 5851 | AgAAATCTTg | AAgACgAAAg | ggCCTCgTgA | TACgCCTATT | TTTATAggTT |
| 5901 | AATgTCATgA | TAATAATggT | TTCTTAgACg | TACgTCAggT | ggCACTTTTC |
| 5951 | ggggAAATgT | gCgCggAACC | CCTATTTgTT | TATTTTTCTA | AATACATTCA |
| 6001 | AATATgTATC | CgCTCATgAg | ACAATAACCC | TgATAAATgC | TTCAATAATA |
| 6051 | TTgAAAAAgg | AAgAgTATgA | gTATTCAACA | TTTCCgTgTC | gCCCTTATTC |
| 6101 | CCTTTTTTgC | ggCATTTTgC | CTTCCTgTTT | TTgCTCACCC | AgAAACgCTg |
| 6151 | gTgAAAgTAA | AAgATgCTgA | AgATCAgTTg | ggTgCACgAg | TgggTTACAT |
| 6201 | CgAACTggAT | CTCAACAgCg | gTAAgATCCT | TgAgAgTTTT | CgCCCCgAAg |
| 6251 | AACgTTTTCC | AATgATgAgC | ACTTTTAAAg | TTCTgCTATg | TggCgCggTA |
| 6301 | TTATCCCgTg | TTgACgCCgg | gCAAgAgCAA | CTCggTCgCC | gCATACACTA |
| 6351 | TTCTCAgAAT | gACTTggTTg | AgTACTCACC | AgTCACAgAA | AAgCATCTTA |
| 6401 | CggATggCAT | gACAgTAAgA | gAATTATgCA | gTgCTgCCAT | AACCATgAgT |
| 6451 | gATAACACTg | CggCCAACTT | ACTTCTgACA | ACgATCggAg | gACCgAAggA |
| 6501 | gCTAACCgCT | TTTTTgCACA | ACATgggggA | TCATgTAACT | CgCCTTgATC |
| 6551 | gTTgggAACC | ggAgCTgAAT | gAAgCCATAC | CAAACgACgA | gCgTgACACC |
| 6601 | ACgATgCCTg | CAgCAATggC | AACAACgTTg | CgCAAACTAT | TAACTggCgA |
| 6651 | ACTACTTACT | CTAgCTTCCC | ggCAACAATT | AATAgACTgg | ATggAggCgg |
| 6701 | ATAAAgTTgC | AggACCACTT | CTgCgCTCgg | CCCTTCCggC | TggCTggTTT |
| 6751 | ATTgCTgATA | AATCTggAgC | CggTgAgCgT | gggTCTCgCg | gTATCATTgC |
| 6801 | AgCACTgggg | CCAgATggTA | AgCCCTCCCg | TATCgTAgTT | ATCTACACgA |
| 6851 | CggggAgTCA | ggCAACTATg | gATgAACgAA | ATAgACAgAT | CgCTgAgATA |
| 6901 | ggTgCCTCAC | TgATTAAgCA | TTggTAACTg | TCAgACCAAg | TTTACTCATA |
| 6951 | TATACTTTAg | ATTgATTTAA | ATTgTAAACg | TTAATATTTT | gTTAAAATTC |
| 7001 | gCgTTAAATT | TTTgTTAAAT | CAgCTCATTT | TTTAACCAAT | AggCCgAAAT |
| 7051 | CggCAAAATC | CCTTATAAAT | CAAAAgAATA | gACCgAgATA | gggTTgAgTg |
| 7101 | TTgTTCCAgT | TTggAACAAg | AgTCCACTAT | TAAAgAACgT | ggACTCCAAC |
| 7151 | gTCAAAgggC | gAAAAACCgT | CTATCAgggC | gATggCCCAC | TACgTgAACC |
| 7201 | ATCACCCTAA | TCAAgTTTTT | TggggTCgAg | gTgCCgTAAA | gCACTAAATC |
| 7251 | ggAACCCTAA | AgggAgCCCC | CgATTTAgAg | CTTgACgggg | AAAgCCggCg |
| 7301 | AACgTggCgA | gAAAggAAgg | gAAgAAAgCg | AAAggAgCgg | gCgCTAgggC |
| 7351 | gCTggCAAgT | gTAgCggTCA | CgCTgCgCgT | AACCACCACA | CCCgCCgCgC |
| 7401 | TTAATgCgCC | gCTACAgggC | gCgTAAAAgg | ATCTAggTgA | AgATCCTTTT |
| 7451 | TgATAATCTC | ATgACCAAAA | TCCCTTAACg | TgAgTTTTCg | TTCCACTgAg |
| 7501 | CgTCAgACCC | CgTAgAAAAg | ATCAAAggAT | CTTCTTgAgA | TCCTTTTTTT |

Table 253, continued

```
7551 CTgCgCgTAA TCTgCTgCTT gCAAACAAAA AAACCACCgC TACCAgCggT
7601 ggTTTgTTTg CCggATCAAg AgCTACCAAC TCTTTTTCCg AAggTAACTg
7651 gCTTCAgCAg AgCgCAgATA CCAAATACTg TCCTTCTAgT gTAgCCgTAg
7701 TTAggCCACC ACTTCAAgAA CTCTgTAgCA CCgCCTACAT ACCTCgCTCT
7751 gCTAATCCTg TTACCAgTgg CTgCTgCCAg TggCgATAAg TCgTgTCTTA
7801 CCgggTTggA CTCAAgACgA TAgTTACCgg ATAAggCgCA gCggTCgggC
7851 TgAACggggg gTTCgTgCAC ACAgCCCAgC TTggAgCgAA CgACCTACAC
7901 CgAACTgAgA TACCTACAgC gTgAgCATTg AgAAAgCgCC ACgCTTCCCg
7951 AAgggAgAAA ggCggACAgg TATCCggTAA gCggCAgggT CggAACAggA
8001 gAgCgCACgA gggAgCTTCC AgggggAAAC gCCTggTATC TTTATAgTCC
8051 TgTCgggTTT CgCCACCTCT gACTTgAgCg TCgATTTTTg TgATgCTCgT
8101 CAggggggCg gAgCCTATgg AAAAACgCCA gCAACgCggC CTTTTTACgg
8151 TTCCTggCCT TTTgCTggCC TTTTgCTCAC ATgTTCTTTC CTgCgTTATC
8201 CCCTgATTCT gTggATAACC gTATTACCgC CTTTgAgTgA gCTgATACCg
8251 CTCgCCgCAg CCgAACgACC gAgCgCAgCg AgTCAgTgAg CgAggAAgCg
8301 gAAgAgCgCC TgATgCggTA TTTTCTCCTT ACgCATCTgT gCggTATTTC
8351 ACACCgCATA TggTgCACTC TCAgTACAAT CTgCTCTgAT gCCgCATAgT
8401 TAAgCCAgTA TACACTCCgC TATCgCTACg TgACTgggTC ATggCTgCgC
8451 CCCgACACCC gCCAACACCC gCTgACgCgC CCTgACgggC TTgTCTgCTC
8501 CCggCATCCg CTTACAgACA AgCTgTgACC gTCTCCgggA gCTgCATgTg
8551 TCAgAggTTT TCACCgTCAT CACCgAAACg CgCgAggCAg
```

**Table 254**: restriction map of pD2pick(MFαPrePro::EPI-HNE-3)

Non-cutters

| | | | | |
|---|---|---|---|---|
| *Afl*II | *Apa*I | *Asc*I | *Ava*I | *Avr*II |
| *Bam*HI | *Bgl*II | *Bss*HII | *Bst*EII | *Mlu*I |
| *Pac*I | *Pml*I | *Rsr*II | *Sac*II | *Sfi*I |
| *Sna*BI | *Spe*I | *Xho*I | *Xma*I | |

Cutters, 3 or fewer sites

| | | | | | | | | | |
|---|---|---|---|---|---|---|---|---|---|
| *Aat*II | 1 | 1098 | | | *Hin*dIII | 3 | 885 | 1717 | 1729 |
| *Afl*III | 1 | 8179 | | | *Hpa*I | 2 | 1017 | 2272 | |
| *Age*I | 1 | 1436 | | | *Kpn*I | 2 | 2323 | 2934 | |
| *Alw*NI | 3 | 2828 | 2852 | 7765 | *Msc*I | 2 | 2204 | 3789 | |
| *Apa*LI | 3 | 6182 | 7865 | 8363 | *Nco*I | 1 | 3766 | | |
| *Ase*I | 3 | 591 | 5822 | 6678 | *Nde*I | 1 | 8357 | | |
| *Bgl*I | 3 | 284 | 2717 | 6730 | *Ngo*MI | 2 | 4702 | 7294 | |
| *Bsa*AI | 2 | 7191 | 8427 | | *Nhe*I | 2 | 1929 | 2875 | |

(continued)

| Cutters, 3 or fewer sites | | | | | | | | | |
|---|---|---|---|---|---|---|---|---|---|
| *Bsg*I | 2 | 2545 | 4494 | | *Not*I | 3 | 6 | 5712 | 8596 |
| *Bsi*WI | 3 | 1568 | 2301 | 5929 | *Nru*I | 1 | 5208 | | |
| *Bsp*DI | 2 | 1723 | 5795 | | *Nsi*I | 2 | 684 | 1241 | |
| *Bsp*EI | 1 | 3978 | | | *Pfl*MI | 2 | 196 | 1302 | |
| *Bsp*MI | 1 | 4576 | | | *Pme*I | 1 | 420 | | |
| *Bst*1107I | 1 | 8408 | | | *Ppu*MI | 2 | 142 | 4339 | |
| *Bst*BI(*Asu*II) | 1 | 945 | | | *Pst*I | 1 | 6608 | | |
| *Bst*XI | 3 | 711 | 2765 | 2896 | *Pvu*I | 1 | 6482 | | |
| *Bsu*36I | 1 | 2223 | | | *Pvu*II | 2 | 1600 | 4497 | |
| *Dra*III | 2 | 3754 | 7188 | | *Sac*I | 1 | 216 | | |
| *Eag*I | 3 | 7 | 5713 | 8597 | *Sal*I | 1 | 3312 | | |
| *Eam*1105I | 2 | 5077 | 6849 | | *Sca*I | 2 | 1360 | 6371 | |
| *Ec1*136I | 1 | 216 | | | *Sph*I | 1 | 4863 | | |
| *Eco*47III | 2 | 1932 | 4795 | | *Ssp*I | 3 | 2806 | 6047 | 6983 |
| *Eco*NI | 3 | 3433 | 4923 | 5295 | | | | | |
| *Eco*RI | 1 | 1383 | | | | | | | |
| *Eco*RV | 2 | 1885 | 5660 | | | | | | |
| *Esp*3I(*Bsa*I) | 2 | 3120 | 8530 | | | | | | |
| *Esp*I(*Bpu*1102I) | 1 | 597 | | | | | | | |
| *Fsp*I | 2 | 1960 | 6629 | | | | | | |
| *Stu*I | 1 | 3395 | | | | | | | |
| *Tth*111I | 1 | 8432 | | | | | | | |
| *Xba*I | 1 | 2168 | | | | | | | |
| *Xcm*I | 1 | 711 | | | | | | | |

Table 400: Amino-acid Sequence of ITI light chain (SEQ ID NO. 077)

```
                 111111 111122
        12345 6789012345 678901
        avlpq eeegsgggql vtevtk

2222222233333333334444444444555555555566666666667777777
2345678901234567890123456789012345678901234567890123456
KEDSCQLGYSAGPCMGMTSRYFYNGTSMACETFQYGGCMGNGNNFVTEKECLQTC

77788
78901
rtvaa

                 11111111111111111111111111111111111111
8888888899999999990000000000111111111122222222223333333
2345678901234567890123456789012345678901234567890123456
CNLPIVRGPCRAFIQLWAFDAVKGKCVLFPYGGCQGNGNKFYSEKECREYCGVP

                        111111111111
                        333344444444
                        678901234567
                        gdgdeellrfsn
```

ITI-D1 comprises residues 22-76 and optionally one of residue 77, residues 77 and 78, or residues 77-79.
ITI-D2 comprises residues 80-135 and optionally one of residue 79 or residues 78-79.

The lines under the sequences represent disulfides.

**TABLE 602**: Physical properties of hNE inhibitors derived from Kunitz domains

| Protein | Parent | # Resid ues | Mol Wt | Pre-dicted pI | $K_D$ (pM) | $k_{on}$ ($10^6$/ M/s) | $k_{off}$ ($10^{-6}$/ s) |
|---|---|---|---|---|---|---|---|
| EPI-HNE-1 | BPTI | 58 | 6359 | 9.10 | 2.0 | 3.7 | 7.4 |
| EPI-HNE-2 | BPTI | 62 | 6759 | 4.89 | 4.9 | 4.0 | 20. |
| EPI-HNE-3 | ITI-D2 | 56 | 6179 | 10.04 | 6.2 | 8.0 | 50. |
| EPI-HNE-4 | ITI-D2 | 56 | 6237 | 9.73 | 4.6 | 10.6 | 49. |

The constants $K_D$ and $k_{on}$ above were measured with [hNE] = 8.47 x $10^{-10}$ molar;
$k_{off}$ was calculated from $k_{off} = K_D \times k_{on}$.

**TABLE 603:** SUMMARY OF PURIFICATION OF EPI-HNE-2

| STAGE | Volume (ml) | Concentration (mg/ml) | Total (mg) | Activity (mg/$A_{280}$) |
|---|---|---|---|---|
| HARVEST | 3,300 | 0.70 | 2.31 | < 0.01 |

(continued)

| STAGE | Volume (ml) | Concentration (mg/ml) | Total (mg) | Activity (mg/$A_{280}$) |
|---|---|---|---|---|
| 30K ULTRA-FILTRATION FILTRATE | 5,000 | 0.27 | 1.40 | < 0.01 |
| 5K ULTRA-FILTRATION RETENTATE | 1,000 | 1.20 | 1.20 | 0.63 |
| AMMONIUM SULFATE PRECIPITATE | 300 | 2.42 | 0.73 | 1.05 |
| IEX pH6.2 ELUATE | 98 | 6.88 | 0.67 | 1.03 |
| EPI-HNE-3, LOT 1 | 50 | 13.5 | 0.68 | 1.04 |

**TABLE 604**: SUMMARY OF PURIFICATION OF EPI-HNE-3

| STAGE | VOLUME (ml) | CONCENTRATION (mg/ml) | TOTAL (mg) | ACTIVITY (mg/$A_{280}$) |
|---|---|---|---|---|
| HARVEST | 3,100 | 0.085 | 263 | nd |
| 30K ULTRA-FILTRATION FILTRATE | 3,260 | 0.055 | 179 | 0.007 |
| FIRST IEX: pH6.2 ELUATE | 180 | 0.52 | 94 | 0.59 |
| AMMONIUM SULFATE PRECIPITATE | 100 | 0.75 | 75 | 0.59 |
| IEX pH9 ELUATE | 60 | 1.01 | 60 | 0.59 |
| EPI-HNE-3, LOT 1 | 26 | 1.54 | 40 | 0.45 |

**TABLE 605**: $K_I$ VALUES OF EPI-HNE PROTEINS FOR VARIOUS HUMAN SERUM SERINE PROTEASES

| | Inhibitor: | | | |
|---|---|---|---|---|
| Enzyme | EPI-HNE-1 | EPI-HNE-2 | EPI-HNE-3 | EPI-HNE-4 |
| Human Neutrophil Elastase | 2 pM | 5 pM | 6 pM | 5 pM |
| Human Serum Plasmin | > 6 $\mu$M | > 100 $\mu$M | > 100 $\mu$M | > 90 $\mu$M |
| Human Serum Kallikrein | > 10 $\mu$M | > 100 $\mu$M | > 100 $\mu$M | > 90 $\mu$M |
| Human Serum Thrombin | > 90 $\mu$M | > 100 $\mu$M | > 100 $\mu$M | > 90 $\mu$M |
| Human Urine Urokinase | > 90 $\mu$M | > 100 $\mu$M | > 100 $\mu$M | > 90 $\mu$M |
| Human Plasma Factor $X_a$ | > 90 $\mu$M | > 100 $\mu$M | > 100 $\mu$M | > 90 $\mu$M |
| Human Pancreatic Chymotrypsin | ~10 $\mu$M | ~10 $\mu$M | ~30 $\mu$M | ~10 $\mu$M |

**Table 607**: PEY-33 which produces EPI-HNE-2

| Elapse Fermenter Time Hours: minutes | Cell Density ($A_{600}$) | Activity in supematent (mg/l) |
|---|---|---|
| 41:09 | 89 | 28 |
| 43:08 | 89 | 57 |
| 51:54 | 95 | 92 |
| 57:05 | 120 | 140 |
| 62:43 | 140 | 245 |
| 74:45 | 160 | 360 |
| 87:56 | 170 | 473 |

(continued)

| Elapse Fermenter Time Hours: minutes | Cell Density ($A_{600}$) | Activity in supematent (mg/l) |
|---|---|---|
| 98:13 | 190 | 656 |
| 102:25 | 200 | 678 |
| 109:58 | 230 | 710 |

Fermenter culture growth and EPI-HNE protein secretion by *P. pastoris* strains PEY-33. Time course is shown for fermenter cultures following initiation of methanol-limited feed growth phase. Increase in cell mass is estimated by $A_{600}$. Concentration of inhibitor protein in the fermenter culture medium was determined from measurements of hNE inhibition by diluted aliquots of cell-free CM obtained at the times indicated and stored at -20°C until assay.

**Table 608:** PEY-43 Which produces EPI-HNE-3

| Elapse Fermenter Time Hours: minutes | Cell Density ($A_{600}$) | Activity in supernatent (mg/l) |
|---|---|---|
| 44:30 | 107 | 0.63 |
| 50:24 | 70 | 9.4 |
| 52:00 | 117 | 14. |
| 62:00 | 131 | 28. |
| 76:00 | 147 | 39. |
| 86:34 | 200 | 56. |
| 100:27 | 185 | 70. |
| 113:06 | 207 | 85. |

Fermenter culture growth and EPI-HNE protein secretion by *P. pastoris* strains PEY-43. Time course is shown for fermenter cultures following initiation of methanol-limited feed growth phase. Increase in cell mass is estimated by $A_{600}$. Concentration of inhibitor protein in the fermenter CM was determined by assays of hNE inhibition by diluted aliquots of cell-free CM obtained at the times indicated and stored at -20°C until assay.

**Table 610**: Inhibitory properties of EPI-HNE-2

| $\mu$l of EPI-HNE-2 solution added | Percent residual hNE activity |
|---|---|
| 0. | 101.1 |
| 0. | 100.0 |
| 0. | 100.0 |
| 0. | 100.0 |
| 0. | 100.0 |
| 0. | 98.9 |
| 10. | 82.9 |
| 20. | 71.8 |
| 30. | 59.5 |
| 40. | 46.2 |
| 50. | 39.2 |
| 55. | 32.2 |
| 60. | 22.5 |
| 65. | 23.5 |
| 70. | 15.0 |

(continued)

| μl of EPI-HNE-2 solution added | Percent residual hNE activity |
|---|---|
| 75. | 10.4 |
| 80. | 8.6 |
| 85. | 4.8 |
| 90. | 1.4 |
| 95. | 2.0 |
| 100. | 2.5 |
| 120. | 0.2 |
| 150. | 0.2 |
| 200. | 0.04 |

**Table 611**: hNE inhibitory properties of EPI-HNE-3

| μl of EPI-HNE-3 solution added | Percent residual hNE activity |
|---|---|
| 0. | 101.2 |
| 0. | 100.0 |
| 0. | 100.0 |
| 0. | 100.0 |
| 0. | 100.0 |
| 0. | 98.8 |
| 10. | 81.6 |
| 20. | 66.9 |
| 30. | 53.4 |
| 40. | 38.0 |
| 50. | 27.6 |
| 55. | 21.5 |
| 60. | 13.0 |
| 65. | 11.0 |
| 70. | 7.9 |
| 75. | 3.8 |
| 80. | 3.3 |
| 85. | 2.1 |
| 90. | 1.8 |
| 100. | 1.6 |
| 110. | 0.8 |
| 120. | 0.7 |
| 160. | 0.6 |
| 200. | 0.2 |

**Table 612:** pH stability of Kunitz-domain hNE inhibitors

| Incubation pH | Percent Residual hNE Inhibitory Activity | | | |
|---|---|---|---|---|
| | EPI-HNE-1 | EPI-HNE-2 | EPI-HNE-3 | EPI-HNE-4 |
| 1.0 | 102 | 98 | 97 | 98 |
| 2.0 | 100 | 97 | 97 | 100 |
| 2.6 | 101 | | | |
| 3.0 | 100 | 101 | 100 | 96 |
| 4.0 | 98 | 101 | 102 | 94 |
| 5.0 | 100 | | | |
| 5.5 | | 99 | 99 | 109 |
| 6.0 | 100 | | 103 | 99 |
| 6.5 | | | 99 | 100 |
| 7.0 | 93 | 103 | 103 | 93 |
| 7.5 | | | 87 | 109 |
| 8.0 | 96 | | 84 | 83 |
| 8.5 | | 104 | 68 | 86 |
| 9.4 | 100 | | 44 | 40 |
| 10.0 | 98 | 102 | 27 | 34 |

Proteins were incubated at 37°C for 18 hours in buffers of defined pH (see text). In all cases protein concentrations were 1 $\mu$M. At the end of the incubation period, aliquots of the reactions were diluted and residual hNE-inhibition activity determined.

**Table 620:** Stability of hNE inhibitory proteins to oxidation by Chloramine-T

| Table 620 | Percent Residual hNE-Inhibitory Activity | | | | | |
|---|---|---|---|---|---|---|
| Molar Ratio CHL-T: Inhibitor | EPI-NE-1 | EPI-HNE-2 | EPI-HNE-3 | EPI-HNE-4 | $\alpha$1 anti trypsin | SLPI |
| 0 | 100 | 100 | 100 | 100 | 100 | 100 |
| 0.25 | | 94 | | | | |
| 0.29 | | | | | | 93 |
| 0.30 | | | | | 97 | |
| .48 | 102 | | | | | |
| .50 | | 102 | 97 | 100 | 85 | |
| .59 | | | | | | 82 |
| .88 | | | | | | 73 |
| .95 | 100 | | | | | |
| 1.0 | | 102 | 97 | 100 | 41 | |
| 1.2 | | | | | | 65 |
| 1.4 | 98 | | | | | |
| 1.5 | | 95 | | | | |
| 1.9 | 102 | | | | | |
| 2.0 | | 102 | | | | |

(continued)

| Table 620 | Percent Residual hNE-Inhibitory Activity | | | | | |
|---|---|---|---|---|---|---|
| Molar Ratio CHL-T: Inhibitor | EPI-NE-1 | EPI-HNE-2 | EPI-HNE-3 | EPI-HNE-4 | α1 anti trypsin | SLPI |
| 2.1 | | | | | 7 | |
| 2.4 | | | | | | 48 |
| 3.0 | | | 97 | 100 | | |
| 3.8 | 94 | | | | | |
| 4.0 | | 95 | | | | |
| 5.0 | | | 94 | 100 | | |
| 5.2 | | | | | 7 | |
| 5.9 | | | | | | 18 |
| 9.5 | 95 | | | | | |
| 10. | | 98 | 97 | 104 | | |
| 10.4 | | | | | > 5 | |
| 12. | | | | | | 15 |
| 19. | 92 | | | | | |
| 30. | | | 100 | 100 | | |
| 50. | | | 94 | 100 | | |

Inhibitors were incubated in the presence of Chloramine-T at the molar ratios indicated for 20 minutes at RT. Oxidation reactions were quenched by adding methionine to a final concentration of 4 mM. Residual hNE-inhibition activity remaining in the quenched reactions is shown as a percentage of the activity observed with no added oxidant. Proteins and concentrations in the oxidation reactions are: EPI-HNE-1, (5 $\mu$M); EPI-HNE-2, (10 $\mu$M); EPI-HNE-3,(10 $\mu$M); EPI-HNE-4, (10 $\mu$M); API, (10 $\mu$M); and SLPI, (8.5 $\mu$M).

**Table 630:** Temperature stability of EPI-HNE proteins

| | Residual hNE Inhibitory Activity | | | |
|---|---|---|---|---|
| Temperature (°C) | EPI-HNE-1 | EPI-HNE-2 | EPI-HNE-3 | EPI-HNE-4 |
| 0 | 97 | 101 | 96 | 100 |
| 23 | 100 | 103 | 105 | 103 |
| 37 | 100 | 97 | 99 | 98 |
| 45 | 103 | | | |
| 52 | | 101 | 100 | |
| 55 | 99 | | | 98 |
| 65 | 94 | 95 | 87 | |
| 69 | | | | 82 |
| 75 | 100 | | | |
| 80 | | 101 | 79 | |
| 85 | 106 | | | 63 |
| 93 | | 88 | 57 | |
| 95 | 64 | | | 48 |

Proteins were incubated at the stated temperature for 18 hours in buffer at pH 7.0. In all cases protein concentrations

were 1 $\mu$M. At the end of the incubation period, aliquots of the reactions were diluted and residual hNE-inhibition activity determined.

**Table 711**: Mutations that are likely to improve the affinity of a Kunitz domain for hNE

**Most Preferred**

X18F;

[X15I(preferred), X15V];

**Highly Preferred**

[X16A(Preferred), X16G];

[X17F(preferred), X17M, X17L, X17I, X17L];

[{X19P, X19S}(equally preferred), X19K, X19Q];

X37G;

X12G;

**Preferred**

X13P;

X20R;

X21Y; X21W;

[X34V(preferred), X34P];

[X39Q, X39M];

[X32T, X32L];

[X31Q, X31E, X31V];

[X11T, X11A, X11R];

[X10Y, X10S, X10V];

[X40G, X40A];

X36G;

**Table 720**: M13_III_signal::Human_LACI-D2::mature_M13_III DNA has SEQ ID NO. 078, amino-acid sequence has SEQ ID NO. 079 DNA is linear and *in vivo* it is double stranded. Amino-acid sequence is of a protein that is processed *in vivo* by cleavage after $Ala_{-1}$; the entire gene encodes an amino-acid sequence that continues to give a functional M13 III protein.

```
  M   K   K   L   L   F
 -18 -17 -16 -15 -14 -13
|atg|aaG|aaG|ctt|ctc|ttc|
        |_______|
         HindIII

  A   I   P   L   V   V   P   F   Y   S   G   A
 -12 -11 -10 -9  -8  -7  -6  -5  -4  -3  -2  -1
|gcc|att|cct|ctg|gtg|gta|cct|ttc|tat|tcc|ggc|gcc|
 | BstXI         | | KpnI |              | KasI  |
 |     XcmI          |

  K   P   D   F   C   F   L   E   E   D   P   G
  1   2   3   4   5   6   7   8   9   10  11  12
|aag|cct|gac|ttc|tgc|ttc|ctc|gag|gag|gat|ccc|ggg|
                        | XhoI  |       | XmaI  |

  I   C   R   G   Y   I   T   R   Y   F
  13  14  15  16  17  18  19  20  21  22
|att|tgc|cgc|ggt|tat|att|acg|cgt|tat|ttc|
     | SacII|           | MluI  |

  Y   N   N   Q   T   K   Q   C   E   R
  23  24  25  26  27  28  29  30  31  32
|tat|aat|aac|cag|act|aag|caa|tgt|gag|cgg|
                       | BsrDI| | BsrI  |

  F   K   Y   G   G   C   L   G   N   M
  33  34  35  36  37  38  39  40  41  42
|ttc|aag|tat|ggt|ggt|tgc|cta|ggt|aat|atg|
                       | AvrII|

  N   N   F   E   T   L   E   E   C   K
  43  44  45  46  47  48  49  50  51  52
|aac|aac|ttc|gag|act|cta|gaa|gag|tgt|aag|
                 | XbaI |

  N   I   C   E   D   G   G   A   E   T   V   E   S
  53  54  55  56  57  58 100 101 102 103 104 105 106
|aac|ata|tgt|gag|gat|ggt|ggt|gct|gag|act|gtt|gag|tct|
  | NdeI |                        | DrdI            |
```

$Ala_{101}$ is the first residue of mature M13 III.

**Table 725**: Synthetic *laci-d1* with sites for cloning into display vector

DNA has SEQ ID NO. 080, amino-acid sequence has SEQ ID NO. 081

```
      A   A   E   M   H   S   F   C   A   F   K   A   D
                  1   2   3   4   5   6   7   8   9  10
 5'-gcg|gcc|gag|atg|cat|tcc|ttc|tgc|gct|ttc|aaa|gct|gat|
    |EagI   |  | NsiI  |

     D   G   P   C   K   A   I   M   K   R
    11  12  13  14  15  16  17  18  19  20
   |gaC|ggT|ccG|tgt|aaa|gct|atc|atg|aaa|cgt|
     | RsrII |             | BspHI|

     F   F   F   N   I   F   T   R   Q   C
    21  22  23  24  25  26  27  28  29  30
   |ttc|ttc|ttc|aac|att|ttc|acG|cgt|cag|tgc|
                           | MluI  |

     E   E   F   I   Y   G   G   C   E   G   N   Q
    31  32  33  34  35  36  37  38  39  40  41  42
   |gag|gaA|ttC|att|tac|ggt|ggt|tgt|gaa|ggt|aac|cag|
        | EcoRI |                       | BstEII  |

                 N   R   F   E   S   L   E   E
                43  44  45  46  47  48  49  50
               |aac|cgG|ttc|gaa|tct|ctA|gag|gaa|
               |       | BstBI |   | XbaI |
               | AgeI |

     C   K   K   M   C   T   R   D   G   A
    51  52  53  54  55  56  57  58  59  101
   |tgt|aag|aag|atg|tgc|act|cgt|gac|ggc gcc
                                   | KasI  |
```

$Ala_{101}$ is the first residue of mature M13 III.

Table 730: LACI-D1 hNE Library
DNA has SEQ ID NO. 082, amino-acid sequence has SEQ ID NO. 083

```
         A   A   E   M   H   S   F   C   A   F   K   A
                     1   2   3   4   5   6   7   8   9
    5'-gcg|gcc|gag|atg|cat|tcc|ttc|tgc|gct|ttc|aaa|gct|
        |EagI  |  |  NsiI |
                                                 S
         T|N                                     T|N
       C|R K|R                                   I|M
       S|G S|A                                   Q|H
       Y|H E|G     H|R              F|L          L|P
       D|N D   G   P|L  C   V|I A|G I|V   F      K|R  R
       10  11  12  13  14   15  16  17   18     19   20
      |NRt|RVS|ggT|cNt|tgt|Rtt|gSt|Ntc|ttc|MNS|cgt|

        C
       Y|W
       F|L  F   F   N   I   F   T   R   Q   C
       21  22  23  24  25  26  27  28  29  30
      |tDS|ttc|ttc|aac|att|ttc|acG|cgt|cag|tgc|
                                |  MluI  |

           Q       Q                    Q
          L|P     L|P                  L|P
          T|K     T|K                  T|K
       L|Q V|I    V|E                  V|M          E|G
       E|V E|A F  I|A  Y   G   G   C   E|A G|A  N   Q|R
       31  32  33  34  35  36  37  38  39  40  41  42
      |SWG|VHA|ttC|VHA|tac|ggt|ggt|tgt|VHG|gSt|aac|SRG|

               N   R   F   E   S   L   E   E
               43  44  45  46  47  48  49  50
              |aac|cgG|ttc|gaa|tct|ctA|gag|gaa|
                |      | BstBI |  | XbaI |
                | AgeI |

       C   K   K   M   C   T   R   D   G   A
       51  52  53  54  55  56  57  58  59  101
      |tgt|aag|aag|atg|tgc|act|cgt|gac|ggc gcc
                                      | KasI  |
```

Variegation at 10, 11, 13, 15, 16, 17, 19, and 20 gives rise to 253,400 amino-acid sequences and 589,824 DNA sequences. Variegation at 31, 32, 34, 39, 40, and 42 gives 23,328 amino-acid and DNA sequences. There are about 5.9 x $10^9$ protein sequences and 1.4 x $10^{10}$ DNA sequences.
$Ala_{101}$ would be the first residue of mature M13 III.

**Table 735**: LACI-D2 hNE Library

DNA has SEQ ID NO. 084; amino-acid sequence has SEQ ID NO. 085

```
                                                          P|H
                                                          T|N
                                                      C|R K|R
                                                      S|G S|A
                                                      Y|H E|G
  G   A   K   P   D   F   C   F   L   E   E   D|N D|Q   G
 -2  -1   1   2   3   4   5   6   7   8   9   10  11  12
|ggc|gcc|aag|cct|gac|ttc|tgc|ttc|ctc|gag|gag|NRt|VVS|ggg|
|  KasI |                       |  XhoI |

                             I|N
H|R            F|L           Q|M
P|L            I|V           L|H       C
N|S            Y|H           K|P     F|L
I|T  C  V|I G|A N|D  F       T|R   R Y|W   F
 13  14  15  16  17  18  19  20  21  22
|MNt|tgc|Rtt|gSt|NWt|ttt|MNS|cgt|tDS|ttc|

                                     Q|G
                                     L|P
                                     T|K
                                     V|I
                                   L|Q E|A
  Y   N   N   Q   A   K   Q   C   E|V  R
 23  24  25  26  27  28  29  30  31  32
|tat|aat|aac|cag|Gct|aag|caa|tgt|SWg|VNA|
                        | BsrDI |
                |  EspI     |

    Q|L                     Q|P
    P|T                     T|K         R|G
    V|E                     V|M         K|E
    I|A                     E|A         L|Q
  F  K    Y   G   G   C   L  G|A  N   M|V
 33  34  35  36  37  38  39  40  41  42
|ttc|VHA|tat|ggt|ggt|tgc|VHG|gSt|aat|VBg|

  N   N   F   E   T   L   E   E   C   K
 43  44  45  46  47  48  49  50  51  52
|aac|aac|ttc|gag|act|cta|gaa|gag|tgt|aag|
                    | XbaI |

  N   I   C   E   D   G   G   A   E   T   V   E   S
 53  54  55  56  57  58 100 101 102 103 104 105 106
|aac|ata|tgt|gag|gat|ggt|ggt|gct|gag|act|gtt|gag|tct|
  | NdeI |                      |   DrdI          |
```

$6.37 \times 10^{10}$ amino acid sequences; $1.238 \times 10^{11}$ DNA sequences

| **Table 790**: Amino acids preferred in hNE-inhibiting Kunitz domains | |
|---|---|
| Position | Allowed amino acids |
| 5 | C |
| 10 | YSV,(NA) |
| 11 | TAR, (QP) |
| 12 | G |
| 13 | P,(VALI) |
| 14 | C |
| 15 | IV |
| 16 | AG |
| 17 | FM,ILV(A) |
| 18 | F |
| 19 | PS, QK |
| 20 | R |
| 21 | YW,(F) |
| 30 | C |
| 31 | QEV, (AL) |
| 32 | TL,(PSA) |
| 33 | F |
| 34 | VP |
| 35 | Y |
| 36 | G |
| 37 | G |
| 38 | C |
| 39 | MQ |
| 40 | G,A |
| 41 | N highly preferred |
| 42<br>45 | G preferred, A allowed<br>F |
| 51 | C |
| 55 | C |

**CITATIONS**

ALBR83a: Albrecht *et al., Hoppe-Seyler's Z Physiol Chem* (1983), **364**:1697-1702. ALBR83b: Albrecht *et al., Hoppe-Seyler's Z Physiol Chem* (1983), **364**:1703-1708. ALTM91: Altman *et al., Protein Engineering* **4**(5)593-600 (1991).

AUER87: Auerswald *et al., Biol Chem Hoppe-Seyler* (1987), **368**:1413-1425. AUER88: Auerswald *et al., Bio Chem Hoppe-Seyler* (1988), **369**(Supplement):27-35. AUER89: Auerswald *et al.*, UK Patent Application GB 2,208,511 A.

AUER90: Auerswald *et al.,* US Patent 4,894,436 (16 Jan 1990).

AUSU87: Ausubel *et al*., Editors. **Current Protocols in Molecular Biology**, Greene Publishing Associates and Wiley-Interscience, Publishers: John Wiley & Sons, New York, 1987.
BERN93: Berndt *et al.*, *J Mol Biol* (1993) **234** (3) p735-50.
BRIN90: *Biol Chem Hoppe-Seyler* (1990) **371**(Suppl)43-52. Brinkmann and Tschesche. BRIN91: *Eur J Biochem* (1991) **202**(1)95-99. Brinkmann *et al.*
CAMP82: *J Clin Invest* **70:**845-852 (1982) Campbell *et al.*
CAMP88: *J Cell Biol* **106:**667-676 (1988) Campbell *et al.*
CANT89: Cantor JO, and GM Turino, pp. 159-168 in Elastin and Elastase, Vol. II, Editors L Robert and W Hornebeck, CRC Press, Boca Raton, Fla., 1989.
DIAR90: Diarra-Mehrpour *et al.*, *Eur J Biochem* (1990), **191:**131-139.
DIGA89: Digan *et al.,* (1989) *Bio/Technology* **7**:160*ff.*
ENGH89: Enghild *et al.*, *J Biol Chem* (1989), **264:**15975-15981.
GEBH86: Gebhard, W, and K Hochstrasser, pp.389-401 in Barret and Salvesen (eds.) **Protease Inhibitors** (1986) Elsevier Science Publishers BV (Biomedical Division).
GEBH90: Gebhard *et al., Biol Chem Hoppe-Seyler* (1990), **371**,suppl 13-22.
GOLD86 *Am Rev Respir Dis* **134**:49-56 (1986) Goldstein, W, and G Doering.
GREG93: Gregg *et al., Bio/Technology* (1993) **11**:905-910.
HF3D86 Heidtmann, H, and J Travis, pp. 441-446 in Proteinase Inhibitors, Editors Barrett and Salvesen, Elsevier Science Publishers BV, Amsterdam, 1986.
HYNE90: Hynes *et al., Biochemistry* (1990), **29**:10018-10022.
KAUM86: Kaumerer *et al.*, *Nucleic Acids Res* (1986), **14**:7839-7850.
MCEL91 *The Lancet* **337**:392-4 (1991) McElvaney *et al.*
MCWH89 *Biochem* **28**:5708-5714 (1989) McWherter *et al.*
NORR93: Norris *et al.*, WIPO Application 93/14123.
ODOM90: Odom, L, *Int J Biochem* (1990), **22**:925-930.
ROBE92: Roberts *et al.*, (1992) *Proc Natl Acad Sci USA* **89**(6)2429-33.
SALI90 *TIBS* **15**:435-9 (Nov 1990) Salier, J-P.
SAMB89: Sambrook *et al.*, **Molecular Cloning, A Laboratory Manual**, Second Edition, Cold Spring Harbor Laboratory, 1989.
SCHA87: Schagger, H. and G. von Jagow (1987) *Analytical Biochemistry* **166**:368*ff.* SCHE67: Schecter and Berger, *Biochem Biophys Res Comm* (1967) **27**:157-162. SELL87: Selloum *et al.*, *Biol Chem Hoppe-Seyler* (1987), **368**:47-55.
SKAR92: Skarzynski, T, *J Mol Biol* (1992) **224**(3)671-83.
SPRE94: Sprecher *et al.*, *Proc Natl Acad Sci USA* **91**:3353-3357 (1994).
STOL90: Stoll and Blanchard (1990) *Methods in Enzymology* **182**:24*ff.*
SWAI88: Swaim, MW, and SV Pizzo, *Biochem J* (1988), **254**:171-178.
TRAB86: Traboni, C, R Cortese, *Nucleic Acids Res* (1986), **14**(15)6340.
TRAV88 *Am J Med* **84**(6A)37-42 (1988) Travis.
VEDV91: Vedvick *et al.*, *J Ind Microbiol* (1991) **7**:197-201.
WAGN92: Wagner *et al.*, *Biochem Biophys Res Comm* (1992) **186**:1138-1145.

In the following particular embodiments of the invention are listed:

1. A non-naturally occurring protein which comprises an engineered aprotonin-like Kunitz domain that binds and inhibits hNE with a Ki of less than 50 picomolar,
wherein the domain comprises an amino acid sequence which is at least substantially homologous, over a region of homology which extends from the first cysteine to the last cysteine, with a reference sequence selected from the group consisting of sequences EPI-HNE-3, EPI-HNE-4, DPI.1.1, DPI.1.2, DPI.1.3, DPI.2.1, DPI.2.2, DPI.2.3, DPI.3.1, DPI.3.2, DPI.3.3, DPI.4.1, DPI.4.2, DPI.4.3, DPI.5.1, DPI.5.2, DPI.5.3, DPI.6.1, DPI.6.2, DPI.6.3, DPI.6.4, DPI.6.5, DPI.6.6, DPI.6.7, DPI.7.1, DPI.7.2, DPI.7.3, DPI.7.4, DPI.7.5, DPI.8.1, DPI.8.2, DPI.8.3, DPI.9.1, DPI.9.2, and DPI.9.3,
and wherein said domain is not identical to any domain selected from the group consisting of the EpiNEalpha, EpiNE1, EpiNE2, EpiNE3, EpiNE4, EpiNE5, EpiNE6, EpiNE7, EpiNE8, ITI-E7, BITI-E7, BITI-E7-1222, AMINO1, AMINO2, MUTP1, BITI-E7-141, MUTT26A, MUTQE, and MUT1619 domains.
2. The protein of embodiment 1 wherein, within said region, the domain is at least 80% identical in amino acid sequence with a reference sequence and differs from said reference protein sequence, if at all, solely by one or more conservative modifications.

3. The protein of embodiment 2 wherein the domain comprises and amino acid sequence that is identical to a sequence selected from the group consisting of sequences EPI-HNE-3, EPI-HNE-4, DPI.1.1, DPI.1.2, DPI.1.3, DPI. 2.1, DPI.2.2, DPI.2.3, DPI.3.1, DPI.3.2, DPI.3.3, DPI.4.1, DPI.4.2, DPI.4.3, DPI.5.1, DPI.5.2, DPI.5.3, DPI.6.1, DPI. 6.2, DPI.6.3, DPI.6.4, DPI.6.5, DPI.6.6, DPI.6.7, DPI.7.1, DPI.7.2, DPI.7.3, DPI.7.4, DPI.7.5, DPI.8.1, DPI.8.2, DPI. 8.3, DPI.9.1, DPI.9.2, and DPI.9.3

4. The protein of embodiment 1 wherein said domain differs from a naturally occurring aprotonin-type Kunitz domain by at least the mutations X18F and a mutation selected from the group consisting of X15V and X15I.

5. The protein of embodiment 4 wherein said domain differs from a naturally occurring aprotonin-type Kunitz domain by at least one mutation selected from the group consisting of [X16A, X16G]; [X17F, X17L,X17I, X17M]; [X19P, X19Q, X19K, X19S]; X13P; [X34V, X34P]; [X39Q, X39M]; [X32T, X32L]; [X31Q, X31E, X31V]; [X11T, X11A, X11R]; [X10Y, X10S, X10V]; [X40G, X40A]; X36G; X37G; and X12G.

6. The protein of embodiment 1 in which the number of amino acid sequence differences between the engineered domain and the most similar naturally occurring aprotonin-like Kunitz domain, within the region of homology running from the first cysteine to the last cysteine, is four or less.

7. The protein of embodiment 1 wherein the domain comprises an amino acid sequence selected from the group consisting of sequences EPI-HNE-3 and EPI-HNE-4.

8. A DNA molecule comprising a DNA sequence encoding the protein of any of embodiments 1-7.

9. An expression vector comprising a DNA coding sequence encoding the protein of any of embodiments 1-7, said coding sequence being operably linked to regulatory DNA sequences whereby said coding sequence may be expressed to produce said protein in a suitable host.

10. A transformed cell comprising the expression vector of embodiment 9, said cell producing said protein under conditions conducive to the expression of said coding sequence under the control of said regulatory sequences.

11. A method of producing a protein with hNE-inhibitory activity which comprises cultivating the transformed cell of embodiment 10 under conditions conducive to the expression of said coding sequence, whereby said protein is produced by said cell.

12. The method of embodiment 11 wherein the cell is a *Pichia pastoris* cell.

13. A method of inhibiting hNE activity in a location which comprises introducing to said location an inhibtory amount of the protein of embodiment 1.

14. The method of embodiment 13 in which the location is a site of excessive hNE activity within an animal.

15. The method of embodiment 14 in which the animal is a human.

## Claims

1. A protein which is an inhibitor of human neutrophil elastase comprising an aprotinin-like Kunitz domain and having a polypeptide sequence which, when aligned to minimize mismatches, can be aligned, with four or fewer mismatches, to the pattern:
Cys-Xaa-Xaa-Xaa-Xaa-Xaa-Xaa-Gly-Xaa-Cys-Xaa-Xaa-Xaa-Xaa-Xaa-Xaa-Xaa-Xaa-(Tyr/Phe)-Xaa-Xaa-Xaa-Xaa- Xaa- Xaa- Cys- Xaa- Xaa- Phe- Xaa-(Tyr/Trp/Phe)-Xaa- Gly- Cys- Xaa- Xaa- Xaa-Xaa-(Asn/Gly)-Xaa-(Phe/Tyr)-Xaa-Xaa-Xaa-Xaa-Xaa-Cys-Xaa-Xaa-Xaa-Cys,
wherein Xaa denotes that at that position any amino acid can be used.

2. The protein of claim 1, wherein the mismatches are conservative modifications.

3. A protein comprising a Kunitz domain that is substantially homologous to the polypeptide sequence of SEQ ID NO: 29 (DPL 1.1), SEQ ID NO:30 (DPI. 1.2), SEQ ID NO:31 (DPI.1.3), SEQ ID NO: 33 (DPI.2.1), SEQ ID NO: 34 (DPI. 2.2), SEQ ID NO: 35 (DPI.2.3), SEQ ID NO:37 (DPI.3.1), SEQ ID NO:38 (DPI.3.2), SEQ ID NO:39 (DPI.3.3), SEQ ID NO:41 (DPI.4.1), SEQ ID NO:42 (DPI.4.2), SEQ ID NO:43 (DPI.4.3), SEQ ID NO:45 (DPI.5.1), SEQ ID NO:46 (DPI.5.2), SEQ ID NO:47 (DPI.5.3), SEQ ID NO:49 (DPI.6.1), SEQ ID NO:50 (DPI.6.2), SEQ ID NO: 51 (DPI.6.3), SEQ ID NO:52 (DPI.6.4), SEQ ID NO:53 (DPI.6.5), SEQ ID NO:54 (DPI.6.6), SEQ ID NO:55 (DPI.6.7), SEQ ID NO: 57 (DPI.7.1), SEQ ID NO:58 (DPI.7.2), SEQ ID NO:59 (DPI.7.3), SEQ ID NO:60 (DPI.7.4), SEQ ID NO:61 (DPI. 7.5), SEQ ID NO:63 (DP1.8.1), SEQ ID NO:64 (DPI.8.2), SEQ ID NO:65 (DPI.8.3), SEQ ID NO:67 (DPI.9.1), SEQ ID NO:68 (DPI.9.2), SEQ ID NO:69 (DPI.9.3), SEQ ID NO:26 (EPI-HNE-3), or SEQ ID NO:27 (EPI-HNE-4).

4. The protein of claims 1 to 3 in purified form.

5. A non-naturally occurring protein which comprises an engineered aprotonin-like Kunitz domain that binds and inhibits hNE with a Ki of less than 50 picomolar,
wherein the domain comprises an amino acid sequence which is at least substantially homologous, over a region of homology which extends from the first cysteine to the last cysteine, with a reference sequence selected from the group consisting of sequences EPI-HNE-3, EPI-HNE-4, DPI.1.1, DPI.1.2, DPI.1.3, DPI.2.1, DPI.2.2, DPI.2.3, DPI. 3.1, DPI.3.2, DPI.3.3, DPI.4.1, DPI.4.2, DPI.4.3, DPI.5.1, DPI.5.2, DPI.5.3, DPI.6.1, DPI.6.2, DPI.6.3, DPI.6.4, DPI. 6.5, DPI.6.6, DPI.6.7, DPI.7.1, DPI.7.2, DPI.7.3, DPI.7.4, DPI.7.5, DPI.8.1, DPI.8.2, DPI.8.3, DPI.9.1, DPI.9.2, and DPI.9.3,

and wherein said domain is not identical to any domain selected from the group consisting of the EpiNEalpha, EpiNE1, EpiNE2, EpiNE3, EpiNE4, EpiNE5, EpiNE6, EpiNE7, EpiNE8, ITI-E7, BITI-E7, BITI-E7-1222, AMINO1, AMINO2, MUTP1, BITI-E7-141, MUTT26A, MUTQE, and MUT1619 domains,
particularly

(a) wherein, within said region, the domain is at least 80% identical in amino acid sequence with a reference sequence and differs from said reference protein sequence, if at all, solely by one or more conservative modifications, more particularly wherein the domain comprises and amino acid sequence that is identical to a sequence selected from the group consisting of sequences EPI-HNE-3, EPI-HNE-4, DPI.1.1, DPI.1.2, DPI.1.3, DPI.2.1, DPI.2.2, DPI.2.3, DPI.3.1, DPI.3.2, DPI.3.3, DPI.4.1, DPI.4.2, DPI.4.3, DPI.5.1, DPI.5.2, DPI.5.3, DPI.6.1, DPI.6.2, DPI.6.3, DPI.6.4, DPI.6.5, DPI.6.6, DPI.6.7, DPI.7.1, DPI.7.2, DPI.7.3, DPI.7.4, DPI.7.5, DPI.8.1, DPI.8.2, DPI.8.3, DPI.9.1, DPI.9.2, and DPI.9.3;
(b) wherein said domain differs from a naturally occurring aprotonin-type Kunitz domain by at least the mutations X18F and a mutation selected from the group consisting of X15V and X15I, more particularly wherein said domain differs from a naturally occurring aprotonin-type Kunitz domain by at least one mutation selected from the group consisting of [X16A, X16G]; [X17F, X17L,X17I, X17M]; [X19P, X19Q, X19K, X19S]; X13P; [X34V, X34P]; [X39Q, X39M]; [X32T, X32L]; [X31Q, X31E, X31V]; [X11T, X11A, X11R]; [X10Y, X10S, X10V]; [X40G, X40A]; X36G; X37G; and X12G;
(c) in which the number of amino acid sequence differences between the engineered domain and the most similar naturally occurring aprotonin-like Kunitz domain, within the region of homology running from the first cysteine to the last cysteine, is four or less; or
(d) wherein the domain comprises an amino acid sequence selected from the group consisting of sequences EPI-HNE-3 and EPI-HNE-4.

**6.** A DNA molecule comprising a DNA sequence encoding the protein of any of claims 1 to 5.

**7.** An expression vector comprising a DNA coding sequence encoding the protein of any of claims 1 to 5, said coding sequence being operatively linked to regulatory DNA sequences whereby said coding sequence may be expressed to produce said protein in a suitable host.

**8.** A transformed cell comprising the expression vector of claim 7, said cell producing said protein under conditions conducive to the expression of said coding sequence under the control of said regulatory sequences.

**9.** A method of producing a protein with inhibitory activity against human neutrophil elastase, which comprises cultivating the transformed cell of claim 8 under conditions conducive to the expression of said coding sequence, whereby said protein is produced by said cell.

**10.** The method of claim 9, wherein the cell is a *Pichia pastoris* cell.

**11.** A protein according to any one of claims 1 to 5 in a pharmaceutically acceptable form.

**12.** A pharmaceutical composition comprising a protein according to any one of claims 1 to 5.

**13.** The protein according to claim 11 or the pharmaceutical composition according to claim 12 for use in medicine.

**14.** A method of inhibiting hNE activity in a location which comprises introducing to said location an inhibitory amount of the protein of claim of any of claims 1 to 5, particularly in which the location is a site of excessive hNE activity within an animal, especially within a human.

**15.** Any product, method or use or any other subject-matter disclosed in the description including the examples.